# EUROPÄISCHE PATENTSCHRIFT

(11) **EP 2 714 162 B1**
(45) Veröffentlichungstag und Bekanntmachung des Hinweises auf die Patenterteilung: **01.01.2020**
(21) Anmeldenummer: 12721548.1
(22) Anmeldetag: 21.05.2012
(51) Int. Cl.: A61M 15/00, A61K 9/48, A61K 9/00, A61J 3/07, B65D 47/28

(54) **INHALATOR UND KAPSEL FÜR EINEN INHALATOR**
INHALER AND CAPSULE FOR AN INHALER
INHALATEUR ET CAPSULE POUR INHALATEUR

(30) Priorität: 27.05.2011 GB 201109087; 13.01.2012 EP 12151105
(43) Veröffentlichungstag der Anmeldung: 09.04.2014
(73) Patentinhaber: Boehringer Ingelheim International GmbH, 55216 Ingelheim am Rhein (DE)
(72) Erfinder: DUNNE, Stephen, Terence, Stowmarket Suffolk IP14 2ED (GB); BESSELER, Jens, 55216 Ingelheim am Rhein (DE); FRENTZEL-BEYME, Jessica, 55216 Ingelheim am Rhein (DE); HOLAKOVSKY, Holger, 55216 Ingelheim am Rhein (DE); KLADDERS, Heinrich, 55216 Ingelheim am Rhein (DE); ZUMBLICK, Ole, 55216 Ingelheim am Rhein (DE); WEILER, Claudius, 55216 Ingelheim am Rhein (DE)
(74) Vertreter: Simon, Elke Anna Maria
(86) Internationale Anmeldenummer: PCT/EP2012/059324
(87) Internationale Veröffentlichungsnummer: WO 2012/163704

(56) Entgegenhaltungen:
- WO-A1-01/72605
- WO-A2-2006/074982
- DE-A1-102004 040 928
- FR-A- 1 215 560
- FR-A2- 2 032 436
- US-A- 4 076 848
- US-A- 4 889 114
- US-A1- 2004 131 668
- US-A1- 2004 173 211
- US-A1- 2009 194 105

## Beschreibung

Die vorliegende Erfindung betrifft eine Kapsel zur Aufnahme von medizinischen Formulierungen zum Einsatz in einem Inhalator und einen zugehörigen Inhalator. Insbesondere bezieht sich die Erfindung auf Kapseln, die mit einer pulverförmigen pharmazeutischen Zubereitung gefüllt sind und auf Inhalatoren, mit denen eine pulverförmige pharmazeutische Zubereitung zur Inhalation bereit gestellt werden soll, wobei sich das Pulver in einer Kapsel befindet und zur Inhalation mittels mindestens eines Lochs in der Kapselwand aus der Kapsel austritt.

Aus dem Stand der Technik sind Kapseln bekannt, die in bestimmten medizinischen Vorrichtungen wie Pulverinhalatoren zum Einsatz kommen. Die äußere Form von in solchen Inhalatoren verwendeten Kapseln ist häufig (wie auch in der vorliegenden Schrift) die eines geschlossenen Zylinders mit halbkugelförmigen Enden, wobei die Länge des Zylinders größer ist als sein Durchmesser. Solche Kapseln bestehen üblicherweise aus zwei becherartigen Teilen, nämlich einem Kapselkörper und einer Kapselkappe, die teleskopartig ineinander gesteckt sind. Verschiedene Materialien solcher Kapseln sind bekannt. Viele in der Medizin verwendete Kapseln bestehen aus Gelatine oder Hartgelatine.
Die WO2000/07572 offenbart Kunststoffkapseln zur Verwendung in Pulverinhalatoren. Die Kapseln bestehen aus Kapselkörper und Kapselkappe, die so miteinander verbunden werden können, dass ein stabiler, abgeschlossener Hohlraum von definiertem Volumen gebildet wird. Die Kapsel kann Rastelemente aufweisen, welche die Kapselkappe fest mit dem Kapselkörper verbinden. Ein Beispiel für solche Rastelemente sind punktförmige Erhebungen im Innenmantel der Kapselkappe, die in etwas größere punktförmige Vertiefungen am Außenmantel des Kapselkörpers einrasten. Kapselkappe und Kapselkörper bestehen beide aus dem gleichen nicht-wasserlöslichen, hydrophoben Kunststoff, vorzugsweise Polyethylen.
Die WO2006/074982 A2 offenbart ein Verschlusskonzept für Kapselkappe und Kapselkörper, durch dass es möglich ist, die beiden Teile für den Transport der Kapsel zur Befüllanlage provisorisch über einen Vorverschluss zu verbinden, der im Gegensatz zum Hauptverschluss zerstörungsfrei geöffnet werden kann. Die Verschlüsse werden im Innenmantel der Kapselkappe durch ringförmig verlaufende oder segmentartige Erhebungen und dazu passende peripher den Außenmantel des Kapselkörpers ringförmig umlaufende Vertiefungen gebildet.

Die US 4076848 A zeigt eine Kapsel zur Aufnahme von dehydrierten, pulverisierten Früchten, Kräutern oder Gemüse. Die Kapsel besteht aus zwei gegeneinander beweglichen Hälften. Durch Auseinanderziehen oder Drehen der beweglichen Hälften wird eine Öffnung freigelegt, durch die man den pulverisierten Inhalt z.B. ins Essen herausrieseln lassen kann.

Aus dem Stand der Technik sind verschiedene Pulverinhalatoren bekannt, sowohl Pulverinhalatoren, bei denen sich das Pulver in Blistern befindet als auch solche, bei denen sich das Pulver vor der Inhalation in Kapseln befindet. In der WO 01/72605 wird beispielsweise ein Blister-basierter Pulverinhalator und ein Inhalator mit einer Blisterscheibe mit Einlegeteilen offenbart.
In den Geräten, bei denen sich das Pulver in Kapseln befindet, werden die Kapseln in der Regel in irgendeiner Weise geöffnet, um das Pulver für die Zerstäubung zur Verfügung zu stellen: In manchen Geräten werden die Kapseln beispielsweise mit Messerklingen aufgeschnitten, in anderen wird ihr Inneres über Hohlnadeln an Luftwege im Inhalator angeschlossen. Bei einer Gruppe von Inhalatoren, die insbesondere den Hintergrund zu der hier vorliegenden Erfindung bilden, werden mit Hilfe von Nadelwerkzeugen Löcher in die Kapseln gestochen.
Die WO2004/082750 A1 zeigt ein Beispiel für einen solchen Inhalator, in dem eine Kapsel an ihren beiden Enden durch zwei entgegengesetzte Nadeln angestochen. Beim Inhalationsvorgang rotiert die Kapsel um ihre Querachse, wobei sie durch tangential einströmende Luft angetrieben wird. Partikel, die durch die Rotation der Kapsel aus ihrem Innern gefördert werden, bewegen sich dann durch den Luftstrom zum Mundstück.
Die US 5896855 zeigt einen Inhalator, in denen mehrere Kapseln in einem drehbaren Magazin bevorratet sind und die durch einen wahlweise motorgesteuerten Mechanismus einer Wirbelkammer zugeführt werden, wo das Pulver ebenfalls durch ein Rotieren der Kapsel um ihre Querachse aus Löchern an den Kapselenden ausgebracht wird. Im Magazin werden die Kapseln von Nadeln oder Stöpseln an beiden Enden festgehalten. Hierbei werden die Kapseln entweder vor Einsetzen in das Magazin an ihren Pol-Enden angestochen und diese Löcher werden durch die Stöpsel im Magazin bis zur Zuführung der jeweiligen Kapsel in die Wirbelkammer verschlossen; oder die Kapseln werden durch eben diese Nadeln beim Einsetzen der Kapseln ins Magazin angestochen und die Anstechnadeln verbleiben bis zur Zuführung der jeweiligen Kapsel in die Wirbelkammer abdichtend in den Löchern.

Die WO04/052435 A1 und die US 2004/173211 A1 zeigen verschiedene Kapsel-basierte Pulverinhalatoren, in denen die Zerstäubung unter der Nutzung des so genannten Bernoulli-Effekts stattfindet. Hierbei befindet sich eine die Pulverformulierung enthaltende Kapsel in einer Kammer, die mit einer Vorrichtung zur seitlichen Öffnung der Kapsel vorgesehen ist. Die Kapsel kann sich im Wesentlichen nur in Längsrichtung in der Kammer bewegen und wird durch eine Luftströmung parallel zur Längsachse der Kammer bewegt. Ein gezeigter Inhalator weist ein Mundstück auf, das ähnlich wie eine Kappe ausgebildet ist und auf ein Unterteil aufgesetzt wird, das die Kapselkammer beinhaltet. Am Gehäuseunterteil ist eine Schneidevorrichtung zum Öffnen der Kapseln vorgesehen. Zum Austausch der verbrauchten Kapseln gegen neue wird das Mundstück hochgeklappt oder eine Steckverbindung gelöst, die sich zwischen Mundstück und Gehäuseunterteil bzw. zwischen Mundstück und einer ins Gehäuseunterteil eingesetzten und mit der Kapselkammer verbundenen Platte befindet. Ein anderer gezeigter Inhalator weist ein drehbar gelagertes, austauschbares oder nachfüllbares Revolvermagazin mit mehreren, je mit einer Kapsel bestückten Kammern auf.

Solche den Bernoulli-Effekt nutzenden Pulverinhalatoren bilden den Ausgangspunkt für die hier vorgestellte Erfindung und die im Folgenden beschriebene Funktionsweise gilt auch für die Inhalatoren, die Gegenstand der vorliegenden Erfindungen sind.
In den hier betrachteten Inhalatoren ist der auszubringende Wirkstoff in einer im Wesentlichen zylindrischen Kapsel gelagert und diese Kapsel wird in die Inhalationskammer eines Inhalators eingesetzt. Die Kapselkammer ist dabei an die Größe der Kapsel insofern angepasst, dass sie ebenfalls im Wesentlichen zylindrisch ausgebildet ist, wobei ihre Länge und ihr Durchmesser jeweils etwas größer als die entsprechende Maße der Kapsel sind. Dadurch hat eine in die Kapselkammer eingesetzte Kapsel genug Spielraum, um sowohl in axialer als auch in radialer Richtung Vibrationsbewegungen ausführen zu können, dabei aber im wesentlichen entlang der Kammerachse ausgerichtet bleibt. Die Kapselkammer weist im Bereich eines der beiden Enden einen Lufteinlass und im Bereich des anderen Endes eine Luftauslassöffnung auf. Der Luftauslass ist an einen Inhalationskanal angeschlossen, der zum Mundstück des Inhalators führt. In der Regel sind Kapselkammer, Luftauslass, Inhalationskanal und Öffnung im Mundstück entlang einer gemeinsamen Achse angeordnet.
Zum Ausbringen des Inhalts der Kapsel wird die Kapsel zunächst an üblicherweise zwei Stellen längsseits am Mantel geöffnet. In der Regel befinden sich die Öffnungen in der Nähe der beiden längsseitigen Enden der Kapsel. Wird nun in der Kapselkammer ein Luftstrom vom Lufteinlass zum Luftauslass erzeugt, führt dieser entlang der Längsachse der Kapsel und bewirkt dabei zweierlei: Zum einen vibriert die Kapsel, wobei ihre Vorzugsbewegungsrichtung bedingt durch den Luftstrom entlang der Längsachse verläuft. Zum anderen erzeugt die an den beiden Kapselöffnungen entlang strömende Luft gegenüber dem Kapselinneren einen Unterdruck, so dass das in der Kapsel befindliche Pulver vom Luftstrom herausgesaugt und dabei zerstäubt wird.

Der vorliegenden Erfindung liegt die Aufgabe zugrunde, eine gegenüber dem Stand der Technik verbesserte Kapsel für die Verwendung in Inhalatoren und einen verbesserten Inhalator anzugeben. Insbesondere soll ein System aus Kapsel und Inhalator angegeben werden, bei dem die Reproduzierbarkeit der Zerstäubung, insbesondere die Pulverausbringung aus der Kapsel verbessert wird. Vorzugsweise soll ein System angegeben werden, bei dem Unregelmäßigkeiten beim Öffnen der Kapseln reduziert oder minimiert werden.

Die genannte Aufgabe wird erfindungsgemäß gelöst durch eine Kapsel für die Verwendung als Reservoir für eine pharmazeutische Zubereitung bzw. medizinische Formulierung in einem Inhalator, wobei die Kapsel zwei einseitig offene Kapselelemente, nämlich einen Kapselkörper und eine Kapselkappe, umfasst, die zur Bildung eines Hohlraums teleskopartig über ihre Öffnungen ineinander gesteckt werden können. Kapselkörper und Kapselkappe sind dabei dadurch gekennzeichnet, dass mindestens einer von beiden, vorzugsweise beide, zusätzlich zur einseitigen Öffnung mindestens je ein vorgefertigtes Loch aufweisen.

Des Weiteren wird die erfindungsgemäße Aufgabe gelöst durch einen Inhalator, der eine Kapselkammer mit einem Lufteinlass und einem in Richtung eines Mundstücks führenden Luftauslass aufweist, wobei eine Kapsel in die Kapselkammer eingesetzt oder aus einem Vorratsmagazin oder einer Kapselaufnahme in die Kapselkammer eingeführt werden kann und der Inhalator einen Schieber aufweist, bei dessen Betätigung Teile der Kapsel so gegeneinander verschoben werden können, dass an der Kapsel vorgefertigte Löcher freigegeben werden.

Des Weiteren wird die Aufgabe erfindungsgemäß gelöst durch ein System, das aus der genannten Kapsel und dem genannten Inhalator gebildet wird.

Des Weiteren wird die Aufgabe erfindungsgemäß gelöst durch ein System, das aus der genannten Kapsel und einem Inhalator gebildet wird, der eine Kapselkammer aufweist, wobei die Kapsel vor der Anwendung des Inhalators in einer zum Inhalator gehörenden Kapselaufnahme bevorratet ist. Diese Kapselaufnahme dichtet mindestens ein Loch und/oder alle Löcher ab, das bzw. die nach dem Ineinanderstecken der Kapselelemente in den Hohlraum der Kapsel führt bzw. führen. Die Kapselaufnahme ist dabei zumindestens teilweise in der Kapselkammer angeordnet und derart aus der Kapselkammer entfernbar, dass die Kapsel bei Entfernung der Kapselaufnahme in der Kapselkammer zurückbleibt.

Des weiteren wird die Aufgabe erfindungsgemäß gelöst durch ein System, das aus der genannten Kapsel und einem Inhalator gebildet wird, wobei die zusammengestzte Kapsel mindestens ein vorgefertigtes Loch aufweist und sich in einem Transportzustand des Systems im Inhalator befindet. Die Kapsel ist dabei zumindest teilweise derart von einer vorzugsweise dehnbaren und/oder biegsamen Folie umschlossen, dass die Folie das mindestens eine vorgefertigte Loch und/oder alle Löcher der Kapsel im Transportzustand des Systems verschließt. Dabei ist die Folie mit einem Zugband verbunden und/oder ragt zum Teil an einem Ende der Kapsel über die Kapsel hinaus. Der Inhalator weist eine Öffnung auf, durch welche die Folie an ihrem überstehenden Teil und/oder an ihrem Zugband aus dem Inhalator herausgezogen werden kann, wobei die vorgefertigten Löcher an der Kapsel frei gelegt werden.

Des Weiteren wird die Aufgabe erfindungsgemäß gelöst durch ein System, das aus einer oben offenen Kapsel und einem Inhalator gebildet wird, wobei sich die Kapsel in einer Vibrationskammer des Inhalators befindet oder aus der Lagerung im Inhalator in eine Vibrationskammer verschoben werden kann.

Des Weiteren wird die Aufgabe erfindungsgemäß gelöst durch ein Verfahren zur Bereitstellung eines Systems aus Kapsel und Inhalator, wobei zunächst ein einseitig offenes Kapselelement in ein ringförmiges Bauteil und/oder eine Kapselaufnahme eingesetzt wird, mit einer abgemessenen Menge pharmazeutischer Zubereitung befüllt wird, das Kapselelement verschlossen wird und das ringförmige Bauteil und/oder die Kapselaufnahme mit allen anderen Bauteilen des Inhalators zusammengesetzt wird.

Vorteilhafte Weiterbildungen werden im Folgenden und im Detail anhand der Figuren beschrieben.

Ein Merkmal der vorliegenden Erfindung ist, dass die zusammengesetzte Kapsel in zwei verschiedenen Zuständen vorliegen kann: Im ersten Zustand ist das mindestens eine vorgefertigte Loch geschlossen, in der zweiten liegt es frei. In dem Inhalator, in dem diese Kapsel eingesetzt wird, wird die Kapsel von ihrem ersten in den zweiten Zustand überführt, in dem dann die pharmazeutische Zubereitung über das vorgefertigte Loch aus der Kapsel austreten kann.

In dem erfindungsgemäßen System aus Inhalator und Kapsel wird das mindestens eine vorgefertigte Loch in der Kapsel im Transportzustand (erster Zustand) des Systems abgedichtet und im Anwendungszustand (zweiter Zustand) liegt es offen vor. Durch die Betätigung eines Schiebe- oder Zugmechanismus wird das Loch freigegeben. Zuvor ist das Loch durch einen Teil der Kapsel selbst oder durch eine zum Inhalator gehörende Kapselaufnahme o.ä. verschlossen.

Ein weiteres Merkmal der vorliegenden Erfindung ist, dass beide Kapselelemente vorgefertigte Löcher und beim teleskopartigen Ineinanderstecken zwei Einsteckstellungen zueinander aufweisen (Diese beiden Einsteckstellungen entsprechen in dieser Ausführung dem so genannten ersten und zweiten Zustand der Kapsel): Eine erste Einsteckstellung, in welcher beide Elemente so ineinander gesteckt sind, dass die vorgefertigten Löcher verdeckt sind und der Hohlraum der Kapsel insgesamt geschlossen ist, und eine zweite Einsteckstellung, in der sich die vorgefertigten Löcher in Kapselkörper und Kapselkappe so überdecken, dass die gesamte Kapsel an der Stelle der Überdeckung beider Löcher ein Loch aufweist.
Vorzugsweise sind beide Kapselelemente becherartig geformt: Der von ihnen gebildete, einseitig offene Hohlraum wird seitlich durch einen umlaufenden Kapselmantel und gegenüber der offenen Seite durch ein geschlossenes Ende begrenzt. Vorzugsweise bildet der Kapselmantel eine zylindrische oder elliptisch umlaufende Wand, so dass in der zusammengeschobenen Kapsel innen keine Ecken gebildet werden, in denen sich insbesondere pulverförmige pharmazeutische Zubereitung ansammeln und dadurch bei einer späteren Ausbringung des Pulvers aus der Kapsel zurück bleiben könnte. Aus gleichem Grund haben die Unterseiten von Kapselkörper und Kapselkappe und somit beide Enden der beim Ineinanderstecken resultierenden Kapsel eine konvexe, insbesondere im Wesentlichen halbkugelartige oder ellipsoidale Form.
Die vorgefertigten Löcher in Kapselkappe und Kapselkörper befinden sich vorzugsweise in ihrem jeweiligen Mantelbereich, so dass beim Ineinanderstecken jeweils der Mantel des anderen Kapselelements das jeweilige Loch abdeckt, bis die Einsteckstellung erreicht ist, in der beide Löcher zur Überdeckung gebracht werden.

Darunter, dass ein Loch "vorgefertigt" ist, ist zu verstehen, dass das Loch in dem jeweiligen Kapselelement bereits im werksseitigen Herstellprozess der Kapseln entsteht. Das Loch im jeweiligen Kapselelement ist hierbei bereits vorhanden, bevor die einzelnen Kapsel fertig zusammengesetzt werden. Insbesondere ist das Loch bzw. sind die Löcher in den Kapselelementen bereits vorhanden, bevor die Kapsel als ganzes in einen Inhalator eingesetzt wird.

Bevorzugt sind Kapselkappe und Kapselkörper auf jeweils den Seiten, die im ineinandergesteckten Zustand aneinander liegen, im Kapselmantelbereich strukturiert. Diese Strukturierung ist vorzugsweise so ausgelegt, dass sie unterschiedliche Funktionen erfüllt. Zum einen weisen die Strukturen von Kapselkappe und Kapselkörper wechselseitige Rastelemente auf. Bevorzugt bewirkt diese Struktur beim Ineinanderstecken der Kapselelemente, dass die Kapselkappe und der Kapselkörper in Relation zu einander in mindestens zwei Stellungen, insbesondere den genannten Einsteckstellungen, verrasten. Durch eine Verrastung in der ersten Einsteckstellung wird sichergestellt, dass zum einen die Löcher in den Kapseln nicht zufällig z.B. durch Erschütterungen beim Transport vorzeitig freigelegt werden und ein definierter Vorgang wie das Zusammenschieben beider Elemente unter Ausübung eines definierten Drucks notwendig ist, um die Löcher freizulegen. Des Weiteren sind die miteinander wechselwirkenden Rastelemente von Kapselkappe und Kapselkörper vorzugsweise so gestaltet, dass sich die beiden Kapselelemente nach dem Ineinanderstecken bis zur ersten Einsteckstellung nicht mehr zerstörungsfrei von einander trennen lassen. Dadurch wird verhindert, dass sich die Kapsel zufällig öffnet. Vorzugsweise sind im Kontaktbereich zwischen Kapselkappe und Kapselkörper zusätzlich Strukturelemente vorhanden, die als Führung beim Ineinanderstecken der beiden Kapselelemente dienen. Diese Strukturelemente an den zueinander gerichteten Mantelbereichen von Kapselkappe und Kapselkörper bewirken, dass die beiden Kapselelemente nur in definierten Ausrichtungen ineinander gesteckt werden können. Die "definierte Ausrichtung" bezieht sich auf die Drehung der Kapselelemente um die Längsachse der Kapsel, es ist also eine azimutale Ausrichtung. Vorzugsweise haben diese Strukturelemente die Gestalt mindestens einer Nut in der äußeren / inneren Mantelfläche von Kapselkappe / Kapselkörper und respektive die Gestalt mindestens einer Führungsschiene in der inneren / äußeren Mantelfläche von Kapselkörper / Kapselkappe. Auf diese Weise wird sichergestellt, dass beim Ineinanderstecken der Kapselelemente die vorgefertigten Löcher in Kapselkappe und Kapselkörper sicher zur Überdeckung gebracht werden können. Bevorzugt werden hierbei geradlinige Führungen, insbesondere solche parallel zur Hauptachse der Kapsel. Varianten in denen die Strukturelemente die Bewegung der Kapselelemente beim Ineinanderstecken gekurvt führen sind ebenfalls möglich. Dies würde beispielsweise durch eine schraubenförmige Führungsnut oder Führungsschiene bewirkt werden.

Ein weiteres - sowohl unabhängig als auch in Kombination mit den oben genannten Aspekten umsetzbares - Merkmal der vorliegenden Erfindung ist, dass die Kapsel statt eines zylindrischen Querschnitts einen elliptischen Querschnitt aufweist. Vorzugsweise handelt es sich bei der Ellipse um so eine, die nur leicht von der Form eines Kreises abweicht (Das Verhältnis von Längs-zu-Querachse der Ellipse sollte kleiner als 75%, vorzugsweise zwischen 90% und 85% sein). Ein solcher elliptischer Querschnitt erzwingt eine definierte azimutale Ausrichtung der beiden Kapselelemente beim Ineinanderstecken und/ oder eine definierte Orientierung beim Einsetzen einer resultierenden Kapsel in eine Kapselaufnahme.

Ein weiteres Merkmal der vorliegenden Erfindung ist, dass das Loch im äußeren Kapselelement vorzugsweise als Langloch, elliptisch oder etwas größer als das zugehörige Loch im inneren Kapselement gestaltet ist. Bei Ausführungen des äußeren Lochs als Langloch oder als Ellipse ist vorzugsweise der schmale Durchmesser des Lochs mindestens so groß wie der Durchmesser des Lochs im inneren Kapselelement. Das äußere Kapselelement ist jenes, das von Kapselkörper und Kapselkappe im ineinandergesteckten Zustand im Bereich der aneinanderliegenden Kapselmäntel die Außenwand der Kapsel bildet. Durch diese Vergrößerung des äußeren Lochs gegenüber dem inneren werden die Toleranzen hinsichtlich der Genauigkeit des Ineinanderstecks der Kapselelemente aufgeweitet. Somit wird sichergestellt, dass auch bei unpräzisem Aufeineinanderschieben die volle Kapselöffnung zur Verfügung steht.

Ein weiteres Merkmal der vorliegenden Erfindung ist, dass eine mit einer pharmazeutischen Zubereitung gefüllte Kapsel, die eine Kapselkappe und/oder einen Kapselkörper mit vorgefertigten Löchern aufweist, in einem Inhalator verwendet wird, wobei die vorgefertigten Löcher zum Zeitpunkt des Einsetzens der Kapsel in eine Kapselkammer des Inhalators verdeckt sind und durch Betätigung eines Schiebers im Inhalator freigelegt werden.

Dies ermöglicht die Bereitstellung eines kapselbasierten, vorzugsweise den Bernoulli-Effekt zur Zerstäubung nutzenden, Inhalators, in dem keine Anstechvorrichtungen zur Lochung der Kapsel bereit gestellt werden müssen. Das Freilegen von vorgefertigten Löchern bietet gegenüber der Verwendung von Anstechvorrichtungen im Inhalator verschiedene Vorteile: Die vorgefertigten Löcher sind bedingt durch ihren Fertigungsprozess, z.B. im Kunststoff-Spritzguss, in Größe und Form von Kapsel zu Kapsel sehr gut reproduzierbar während durch Anstechen erzeugte Löcher in Abhängigkeit von Kapselwerkstoff, Kapselgröße und Material so wie Anstechposition und Geometrie der Nadeln individuell variieren und/oder zu unregelmäßigen Lochgeometrien führen können. Des Weiteren ist beim Anstechen von Kapseln eine gewisse Rückformung der Kapseloberfläche im Bereich der Einstechstelle möglich. Die hier vorgestellten, vorgefertigten Löcher hingegen haben nach ihrer Freilegung eine stabile Form und keine Vorsprünge im Kapselmaterial. Solche Vorsprünge im Kapselmaterial bilden sich z.B. beim Eindrücken der Kapselwand durch eine Anstechvorrichtung und können möglicherweise zur Anlagerung von insbesondere pulverförmiger pharmazeutischer Zubereitung und somit bei Zerstäubung zu einer leicht verminderten Ausbringung der Zubereitung aus der Kapsel führen. Aufgrund der nun durch die Vorfertigung der Löcher möglichen hohen Präzisierung von der Größe und Form der Kapsellöcher sind sowohl die Menge als auch insbesondere die Verteilung des Pulvers reproduzierbarer, das aus Kapseln im Luftstrom in der Kapselkammer eines Inhalators ausgebracht wird. Die Zerstäubung der pharmazeutischen Zubereitung selbst wird dadurch reproduzierbarer, insbesondere im Vergleich zu Inhalatoren mit Kapselanstechvorrichtungen.

In weiteres Merkmal der vorliegenden Erfindung ist, dass der Inhalator zur Verwendung mit Kapseln mit vorgefertigten Löchern einen Schieber aufweist und dieser Schieber zum Freilegen der vorgefertigten Kapsellöcher eine Begrenzung des Innenraums der Kapselkammer, insbesondere einen Teil der Wand der Kapselkammer selbst bildet. Für das Öffnen der Kapsel müssen somit an der Kapselkammer keine zusätzlichen Öffnungen ausgebildet werden, die das Strömungsverhalten bei der Zerstäubung nachteilig beeinflussen können. Es kommt nicht zur Bildung von unerwünschter Seitenluft bzw. Falschluft neben den für die Zerstäubung benötigten Luftkanälen. Das System ist hinsichtlich des Strömungswiderstandes stabil und reproduzierbar.
Vergleichsweise müsste bei der Verwendung einer Anstechvorrichtung das zugehörige Anstechelement durch eine oder mehrere Öffnungen in die Kapselkammer eingeführt werden, was während der Inhalation zu zusätzlichen unerwünschten Luftströmungen durch diese Öffnungen führen kann. Solche Öffnungen zusätzlich zu Lufteinlass und Luftauslass der Kapselkammer werden beim erfindungsgemäßen Inhalator nicht benötigt, sofern sie nicht ihrerseits auf eine Verbesserung der Luftströmung in der Kapselkammer ausgerichtet sind.
Die Verwendung eines Schiebers zum Freilegen von vorgefertigten Löchern hat im Vergleich zur Verwendung von Anstechelementen den weiteren Vorteil, das auch bei mehrfacher Anwendung keine Abnutzungserschienungen oder Materialablagerungen zu erwarten sind. Vergleichsweise können solche nachteiligen Effekte bei Inhalatoren mit Anstechvorrichtungen auftreten, die mehrmals zum Anstechen verwendet werden, z.B. bei Inhalatoren, in denen nach Benutzung die alte Kapsel in der Kapselkammer regelmäßig durch eine unbenutzte ersetzt wird. Insbesondere bei der Verwendung von teilweise klebrigen, pulverförmigen pharmazeutischen Zubereitungen kann es neben einer Abnutzung der Schneidkanten oder Spitzen auch zur Bildung von Materialablagerung an den Anstechspitzen kommen. Diese Ablagerungen können im schlimmsten Fall einerseits zu geringen Dosisveränderungen bei der Ausbringung aus dem Inhalator führen, sei es durch Verbleib solcher Ablagerungen an der neuen Kapsel oder durch Bildung dieser Ablagerungen aus dem Inhalt einer neuangestochenen Kapsel. Zum anderen können diese Ablagerungen an den Anstechspitzen genauso wie Verschleißerscheinungen an den Spitzen die Geometrie der Löcher in den Kapseln beeinflussen und Unregelmäßigkeiten in Größe und Form der Löcher hervorrufen, wodurch wiederum die Reproduzierbarkeit der Ausbringung der pharmazeutischen Zubereitung aus den Kapsel beeinträchtig wird. Des Weiteren kann es, bedingt durch den für den Zerstäubungsprozess notwendigen Bewegungsspielraum der Kapsel in der Kapselkammer, zu Schwankungen bei der Position der Kapsel zu den Anstechspitzen kommen, wenn die Kapsel in der Kapselkammer angestochen wird. Dies führt beim Anstich mehrerer Kapseln zu Schwankungen bezüglich der exakten Position der Löcher in der Kapsel.
Durch die Verwendung eines Schieber-Mechanismus zur Freilegung von vorgefertigten Löchern an Kapseln werden alle aus der Verwendung von Anstechvorrichtungen resultierenden Schwankungen in der Form, Größe und Position der Löcher und die sich daraus ergebenden Schwankungen in der Ausbringung der pharmazeutischen Zubereitung vermieden. Die Zerstäubung der pharmazeutischen Zubereitung ist nun unabhängig von dem Mechanismus zum Öffnen der Löcher in der Kapsel. Dies bedeutet auch, dass bei Verwendung von brüchigem Kapselmaterial das Risiko einer Splitterung, wie sie insbesondere bei Anstechvorgängen auftreten könnte, minimiert ist. Auf diese Weise erweitert sich die Gruppe möglicher Kapselmaterialien für die Verwendung in Inhalatoren.

Die Verwendung eines Schiebemechanismus zum Freilegen vorgefertigter Löcher hat des Weiteren den Vorteil, dass der gleiche Mechanismus für die Freilegung unterschiedlichster Lochanordnungen an Kapseln benutzt werden kann. Z.B. können sich bei zylindrischen Anordnungen von Kapsel, Kapselkammer und Schieber mehrere Löcher an der Kapsel an - bezogen auf ihren Umfang - unterschiedlichen Stellen befinden. Größe, Form, Position, Anzahl der Löcher in einer Kapsel sind bei diesem Konzept sehr variabel, insbesondere bei einer Fertigung der Kapselteile aus Spritzgussprozessen. An dieser Stelle können die Ergebnisse von auf die Zerstäubung von bestimmten pharmazeutischen Zubereitungen bzw. bestimmten Pulvern abgestimmten Strömungssimulationen benutzt werden, um optimale Lochstrukturen für Kapseln für diese bestimmte Zubereitung bzw. dieses bestimmte Pulver zu verwenden.

Ein weiteres Merkmal der vorliegenden Erfindung ist, dass in einer bevorzugten Ausführungsform die in der Kapsel vorgefertigten Löcher durch ein ebenfalls zur Kapsel gehörendes, zusätzliches Bauteil verschlossen sind. Bei im Wesentlichen zylindrischen Kapseln mit vorgefertigten Löchern im Kapselmantel hat dieses zusätzliche Bauteil eine ringförmige Struktur. Dieser Ring wird von außen so entlang der Längsachse über die Kapsel geschoben, dass er die Löcher dicht verschließt.

In einer alternativen Ausführungsform, wird das mindestens eine in der Kapsel vorgefertigte Loch durch ein Bauteil verschlossen, das zum Inhalator gehört. Vor der Überführung in eine Kapselkammer ist die mindestens eine im Wesentlichen zylindrische Kapsel in einer Kapselaufnahme im Inhalator bevorratet. Diese Kapselaufnahme hat im Wesentlichen die Gestalt einer zylindrischen Röhre, die so bemessen ist, dass sie den zylindrischen Teil der Kapsel passgenau umschließt. Das mindestens eine vorgefertigte Loch in der Kapsel befindet sich in dem zylindrischen Mantelbereich der Kapsel und wird somit bei der Bevorratung oder Lagerung der Kapsel im Inhalator durch die Wand der Kapselaufnahme verschlossen. Vorzugsweise weist die so bevorratete und mit Pulver gefüllte Kapsel zwei Löcher oben und unten auf, d.h. je ein Loch am Anfang und eins am Ende des zylindrischen Mantelbereichs. Vorzugsweise besteht die Kapsel aus einem Kapselkörper und einer Kapselkappe, die beide mindestens ein vorgefertigtes Loch haben, wobei nach Zusammenschieben von Kapselkörper und Kapselkappe ineinander die Löcher frei bleiben, d.h. nicht vom jeweils anderen Kapselelement abgedeckt werden. Alternativ können die Löcher auch nach Befüllung der Kapsel und nach Zusammenführung von Kapselkappe und Kapselkörper außerhalb des Inhalators vorgefertigt werden. In den Kapseln können beispielsweise Pulvermengen zwischen 0,1 Milligramm und 100 Milligramm eines reinen Wirkstoffs oder einer Wirkstoffmischung, zur späteren Ausbringung bevorratet sein.

In Abhängigkeit vom Abdichtungskonzept der vorgefertigten Löcher in der Kapsel wird der Schieber ausgelegt, mit dessen Hilfe die Löcher freigelegt werden. Bei Kapseln, deren Löcher bei Einsetzen in die Kapselkammer mit einem Ring verschlossen sind, hat der Schieber vorzugsweise die Gestalt eines Hohlzylinders oder mehrerer seitlich an der Kapsel vorbeigreifenden Stempel, die den Ring von der Kapsel schieben und somit die Löcher freilegen. Auch hierbei bildet der Schieber (14) insbesondere eine Begrenzung des Innenraums der Kapselkammer (13).
Bei Kapseln, die im Inhalator bevorratet sind und deren Löcher durch ein die Kapsel an der Stelle der Löcher umschließendes Bauteil des Inhalators verdeckt sind, hat der Schieber vorzugsweise die Gestalt eines Kolbens. Vorzugsweise hat dieser Kolben den gleichen Durchmesser wie die Kapselaufnahme und drückt die Kapsel aus ihrer Kapselaufnahme direkt in die Kapselkammer (die einen größeren Durchmesser als die Kapselaufnahme hat). Besonders bevorzugt ist der Kolben als Hohlkolben ausgebildet und weist den Lufteinlass für die Kapselkammer auf und/oder bildet nach Zuführung der Kapsel in die Kapselkammer einen Wandbereich der Kapselkammer. Kapseln mit vorgefertigten Löchern, die durch Aufnahmen im Inhalator verschlossen werden, können sowohl in Geräten für den einmaligen Gebrauch (dann mit nur einer Kapsel) als auch in Mehrdosis-Geräten (dann mit Vorratsmagazin mit mehreren Kapselaufnahmen) verwendet werden.
Ebenfalls möglich sind Abdichtungskonzepte, in denen sowohl ein Teil der Löcher über einen Ring als auch ein anderer Teil der Löcher über eine Kapselaufnahme abgedichtet werden. Vorzugsweise verschiebt ein Schieber in einem solchen System, die Kapsel sowohl aus der Kapselaufnahme als auch aus dem Ring.
Ein weiteres Merkmal der vorliegenden Erfindung ist hierbei, dass der Ring vorzugsweise so ausgebildet ist, dass er im voll eingeschobenen Zustand des Schiebers eine Dichtung zwischen Schieber und Kapselaufnahme und/oder Kapselkammer bildet. Hierzu besteht der Ring insbesondere aus einem zumindest teilweise elastischen Material wie beispielsweise PE, TPFE oder Silikon. Die dadurch gebildete Abdichtung dient vorzugsweise zur Verhinderung bzw. Reduzierung von Nebenluftströmungen in die Kapselkammer (solche Nebenluftströmungen könnten die Ausbildung des Bernoulli-Effekts bei Anwendung des Inhalators beeinträchtigen). Insbesondere bei Schiebern, die im eingeschobenen Zustand einen Teil der Kapselkammerwand bilden, werden so Spalte abgedichtet, die sonst zwischen Schieber und Kapselaufnahme oder zwischen Schieber und den weiteren Wänden der Kapselkammer vorhanden sein können.

Alternativ zum beschriebenen Schiebemechanismus weist das System aus Inhalator und Kapsel, insbesondere im Falle eines Systems zur einmaligen Anwendungen (Einwegsystem), einen Zugmechanismus zum Freilegen der vorgefertigten Kapsellöcher auf. Ein solcher Zugmechanismus bietet die gleichen Vorteile wie der Schiebemechanismus in Bezug auf das Freilegen von vorgefertigten Löchern. Der Zugmechanismus ist vorzugsweise derart gestaltet, dass sich die Kapsel in einer Kapselaufnahme befindet und sich die Kapselaufnahme in einem für Transport und Lagerung geeigneten Zustands des Systems in der Kapselkammer befindet. Um das System anwendungsbereit zu machen wird die Kapselaufnahme aus der Kapselkammer, vorzugsweise auch komplett aus dem Inhalator, herausgezogen. Dabei ist eine Vorrichtung vorgesehen, die verhindert, dass die Kapsel mit der Kapselaufnahme aus der Kapselkammer herausgezogen wird und/oder die Kapsel aus der Kapselaufnahme beim Herausziehen der Kapselaufnahme löst.
Vorzugsweise wird die Kapselaufnahme durch ein Mundrohr am Inhalator herausgezogen, das Mundrohr bildet hierbei den Luftauslass aus der Kapselkammer in Richtung mundseitigem Ende des Inhalators. Diese Vorrichtung zur Rückhaltung der Kapsel in der Kapselkammer beinhaltet vorzugsweise einen Stab oder Steg, der gleichzeitig die obere Begrenzung der Kapselkammer bildet. Dieser Stab oder Steg durchdringt vorzugsweise im Lagerzustand des Systems die Kapselaufnahme auf der einem Lufteinlass der Kapselkammer entgegengesetzten Seite. Die Kapselaufnahme weist vorzugsweise schlitzförmig ausgebildete Aussparungen auf, aufgrund derer die Kapselaufnahme beim Herausziehen aus dem Inhalator an dem Steg vorbeigleiten und sich somit von ihm trennen kann. Vorzugsweise weist das die Kapselaufnahmebildende Bauteil im Transportzustand des Systems eine aus dem Inhalator herausragenden Bereich auf, an dem eine Grifffläche ausgebildet ist, an welcher der Anwender anfassen kann, um die Kapselaufnahme aus dem Inhalator herausziehen kann. Besonders bevorzugt ist darüber hinaus das die Kapselaufnahme bildende Bauteil als Kappe gestaltet, die im Transportzustand das mundseitige Ende des Inhalators abdeckt.

Ein weiteres Merkmal der Erfindung ist, dass im Fall von Systemen für einmalige Anwendung die Bauteile, die am jeweiligen Schiebe- oder Zugmechanismus beteiligt sind, Vorrichtungen aufweisen, die bewirken, dass die jeweilige Schiebe- oder Zug-Bewegung nicht zerstörungsfrei rückgängig gemacht werden kann. Insbesondere weist hierzu der Schieber oder die Kapselaufnahme Federarme oder sonstige Rastelemente auf, die zu einer Verrastung des Schiebers nach insbesondere vollständigem Einschub führt bzw. im Fall der herausgezogenen Kapselaufnahme ein zerstörungsfreies Wiedereinsetzen der Kapselaufnahme in die Kapselkammer verhindert.

Die beschriebenen Systeme für einmalige Anwendung können alternativ auch als Systeme mit zwei Kapseln ausgelegt werden. Der Inhalator weist bei solchen Ausführungsformen für die zwei Kapseln auch zwei zugehörige zwei Kapselkammern und zwei Kapselaufnahmen aufweist. Je nach Einsatzzweck eines solchen Systems mit zwei Kapseln, sind die beiden Kapseln optional mit verschiedenen Formulierungen und/oder verschiedenen Formulierungsmengen befüllt. Ein solches System aus Inhalator und zwei Kapseln kann beispielsweise in Therapien verwendet werden, in denen zwei verschiedene medizinische Formulierungen gleichzeitig einem Patienten verabreicht werden sollen. Bei verschieden befüllten Kapseln kann es zweckdienlich sein, die Größe der Kapseln auf die jeweilige Füllmenge anzupassen. In einer solchen sehr speziellen Ausführungsform sind dann auch die jeweiligen Kapselkammern und Kapselaufnahmen hinsichtlich ihrer Größen an ihre jeweiligen Kapseln angepasst. Unabhängig davon, ob es sich um eine Ausführungsform mit zwei gleichen oder zwei verschiedenen Kapseln handelt, sind bei einem solchen System mit zwei Kapseln der Schiebe- oder Zugmechanismus am Inhalator so gestaltet, dass mit einer Bewegung gleichzeitig die Löcher an beiden Kapseln freigelegt werden. Weist das System beispielsweise Kapselaufnahmen, die sich in Mundrohren des Inhalators erstrecken, und eine Kappe mit einer Grifffläche oder einer Lasche zum Ziehen auf, so werden beim Ziehen an der Kappe beide vorzugsweise im Kappenbereich miteinander verbundene Kapselaufnahmen gleichzeitig aus dem Inhalator gezogen.

Bei Kapseln, deren Löcher durch eine relative Verschiebung von Kapselkörper und Kapselkappe ineinander freigelegt werden, wirkt der Schieber ebenfalls auf ein Ende der Kapsel ein. Die wirksame Fläche des Schiebers ist hierbei flach oder konkav gekrümmt, so dass sie flächig an dem Ende der Kapsel anliegt und die Kraft nicht nur in einem Punkt (dem Pol oder Scheitelpunkt des Kapselendes) übertragen wird. Der Schieber zum Zusammenschieben von Kapselelementen kann Teil der Kapselkammer und/oder Teil des Zuführmechanismus der Kapsel aus einem Vorratsmagazin sein, falls ein Inhalator mit der Bevorratung mehrerer Kapseln betrachtet wird. Insbesondere ist der Schieber (14) so ausgebildet, dass er eine Begrenzung des Innenraums der Kapselkammer (13) bildet. Bei solch einem Inhalator mit Vorratsmagazin kann der Kapseltransport zur Kapselkammer beispielsweise aus einem Vorratsschacht erfolgen, bei dem die Kapseln quer durch eine sich über eine Rampe verjüngende Öffnung geschoben werden und diese verjüngende Öffnung das weitere Ineinanderschieben der Kapselelementen erzwingt. In einer solchen Ausführungsform wirkt der Schieber gegebenenfalls nur indirekt auf die Kapsel ein - nämlich als Druckstempel oben am Vorratsschacht.
In einer anderen Ausführungsform mit Vorratsmagazin werden die Kapseln aus einer Vorlageposition mit dem Schieber in die Kapsel hinein gedrückt. Mit dem Hineindrücken in die Kapselkammer erfolgt das zur Öffnung der Löcher notwendige weitere Ineinanderschieben von Kapselkappe und Kapselkörper.
Aufgrund der geringen Komplexität eines Schiebersystems und der damit verbundenen geringen Fertigungskosten, eignet sich ein solches System aus Kapsel mit vorgefertigten, vorerst verdeckten Löchern und in eine Kapselkammer intergriertem Schieber auch für die Fertigung von Einzeldosis-Inhaltoren (Wegwerfartikel). Insbesondere bei Anwendungen, bei denen klebrige pulverförmige pharmazeutischen Zubereitungen zur Inhaltion bereit gestellt werden sollen, bilden sich schnell Ablagerungen in Kapselkammer, Luftauslass und Mundstück, so dass ein häufiger Austausch eines solchen Systems gewünscht ist, wie er durch ein Einweg-Produkt gegeben ist.

Alternativ zu den beschriebenen Systemen aus Inhalator und zweiteiliger Kapsel umfasst die hier vorliegende Erfindung auch ein System, das aus einer oben offenen Kapsel und einem Gerät zur Inhalation trockener Pulver gebildet wird. Die Funktion des Geräts basiert auf der Vibration einer Kapsel, d.h. auf dem Bernoulli-Effekt, und die Kapsel ist hierbei oben offen, wahlweise mit oder ohne vorgefertigte Löcher. Vorgefertigte Löcher befinden sich vorzugsweise im oder nahe des Bodens der Kapsel. Die oben offene Kapsel kann aus einem Reservoir, d.h. einem Pulvervorrat für mehrere Dosiseinheiten bzw. Dosen, oder einem Magazin in Form eines Speichers für mehrere, einzeln abgemessene Dosen innerhalb des Geräts befüllt werden, oder die Kapseln können mit einem abgemessenen Volumen an Pulver vorab befüllt sein. Somit kann das Gerät sowohl ein Mehrdosis-Gerät oder ein Einzeldosis-Gerät sein. Die oben offenen Kapseln können aus ihrem Lagerzustand in eine Vibrationskammer des Inhalators verschoben werden oder sich - insbesondere im Fall eines Eindosis-Geräts - bereits in der Vibrationskammer des Inhalators befinden.
Unter der Bezeichnung "oben offene Kapsel" ist hierbei allgemein ein becherartiges, einseitig offenes Bauteil zu verstehen. Die Gestalt dieses Bechers kann beliebig, auch z.B. rechteckig sein, bevorzugt wird aber (in Anlehnung an die geschlossenen Kapseln der vorher angeführten alternativen Systeme) eine im Wesentlichen zylindrische Form, bei der insbesondere das untere geschlossene Ende derart abgerundet, vorzugsweise halbkugelförmig gestaltet ist, dass sich keine Ecken ergeben. Besonders bevorzugt befindet sich mindestens ein vorgefertigtes Loch im unteren Bereich des zylindrischen Mantelbereichs der oben offenen Kapsel und die Kapsel befindet sich im Lagerzustand im Gerät in einer vorzugsweise zylindrischen Röhre, welche die Kapsel an der Stelle des Lochs passgenau umschließt und so das Loch abdeckt. Vorzugsweise ist im Lagerzustand bei einer vorbefüllten Kapsel die obere Öffnung der Kapsel durch ein flaches Bauteil verschlossen, das zur Anwendung des Geräts von der Kapselöffnung weggezogen werden kann.

Die einzelnen Merkmale der vorliegenden Erfindung können unabhängig voneinander genutzt oder miteinander kombiniert werden.
Weitere Vorteile, Merkmale, Eigenschaften und Aspekte der vorliegenden Erfindung ergeben sich aus den Ansprüchen und der folgenden Beschreibung bevorzugter Ausführungsformen anhand der Zeichnungen. Die Zeichnungen zeigen in:
Fig. 1 eine schematische Darstellung einer erfindungsgemäßen Kapsel in unterschiedlichen Zuständen: a) Kapselkappe und Kapselkörper vor dem Ineinanderstecken, b) Kapselkappe und Kapselkörper in einer ersten Einsteckstellung und c) Kapselkappe und Kapselkörper in einer zweiten Einsteckstellung;
Fig. 2 eine schematische Darstellung einer zweiten Ausführungsform der erfindungsgemäßen Kapsel in unterschiedlichen Zuständen: a) Kapselkappe und Kapselkörper vor dem Ineinanderstecken und b) Kapselkappe und Kapselkörper in einer zweiten Einsteckstellung;
Fig. 3 eine schematische Darstellung einer dritten Ausführungsform der erfindungsgemäßen Kapsel, wobei Kapselkappe und Kapselkörper vor dem Ineinanderstecken gezeigt werden;
Fig. 4 eine schematischen Querschnitt einer vierten Ausführungsform der erfindungsgemäßen Kapsel, wobei in der ersten Bildhälfte Kapselkappe und Kapselkörper vor dem Ineinanderstecken und in der zweiten Bildhälfte Kapselkappe und Kapselkörper nach dem Ineinanderstecken gezeigt werden.
Fig. 5 eine schematischen Querschnitt einer fünften Ausführungsform der erfindungsgemäßen Kapsel, wobei in der ersten Bildhälfte Kapselkappe und Kapselkörper vor dem Ineinanderstecken und in der zweiten Bildhälfte Kapselkappe und Kapselkörper nach dem Ineinanderstecken gezeigt werden.
Fig. 6 eine schematische Darstellung zur Funktionsweise eines Inhalators mit Vorratsmagazin zur Verwendung mit erfindungsgemäßer Kapseln.
Fig. 7 eine schematische Darstellung zur Funktionsweise einer zweiten Ausführungsform eines Inhalators mit Vorratsmagazin zur Verwendung mit erfindungsgemäßer Kapseln.
Fig. 8 eine schematische Darstellung zur Funktionsweise eines einmal verwendbaren Inhalators zur Verwendung mit einer erfindungsgemäßen Kapsel.
Fig. 9 eine schematische Darstellung zur Funktionsweise einer zweiten Ausführungsform eines einmal verwendbaren Inhalators zur Verwendung mit einer erfindungsgemäßen Kapsel.
Fig. 10 eine schematische Darstellung zur Funktionsweise einer dritten Ausführungsform eines einmal verwendbaren Inhalators zur Verwendung mit einer erfindungsgemäßen Kapsel.
Fig. 11 eine schematische Darstellung einer sechsten Ausführungsform der erfindungsgemäßen Kapsel in unterschiedlichen Zuständen: Fig. 11a zeigt Kapselkappe, Kapselkörper und Ring vor dem Ineinanderstecken, Fig. 11b zeigt Kapselkappe getrennt von den zusammengeschobenen Bauteilen Ring und Kapselkörper, Fig. 11c zeigt Kapselkappe und Kapselkörper in einer zweiten Einsteckstellung.
Fig. 12 eine schematische Darstellung zur Funktionsweise einer vierten Ausführungsform eines einmal verwendbaren Inhalators zur Verwendung mit einer erfindungsgemäßen Kapsel.
Fig. 13 eine schematische Darstellung einer siebten und einer achten Ausführungsform der erfindungsgemäßen Kapsel: Fig. 13a zeigt die geschlossene Kapsel mit Füllung, Fig. 13b zeigt die Position von Löchern der Kapsel gemäß der siebten und Fig. 13c der achten Ausführungsform.
Fig. 14 eine schematische Darstellung einer neunten Ausführungsform der erfindungsgemäßen Kapsel mit zugehöriger Funktionsweise der fünften Ausführungsform eines Inhalators: Fig. 14a zeigt die Kapsel mit vorgefertigten Löchern, Fig. 14b die Kapsel bevorratet in einem Inhalator und Fig. 14c die Kapsel nach Verschiebung aus ihrem Lagerzustand in die Kapselkammer des Inhalators.

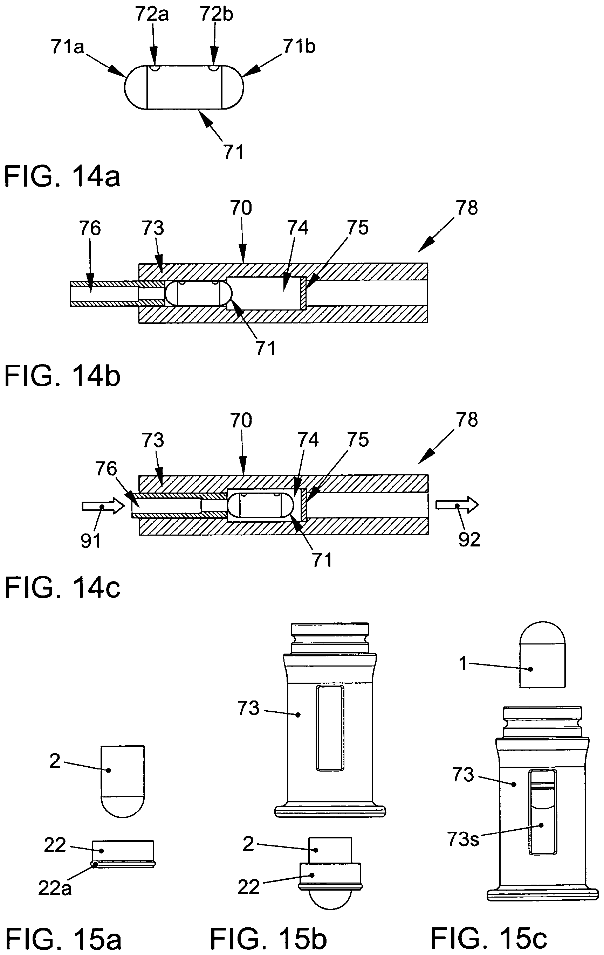

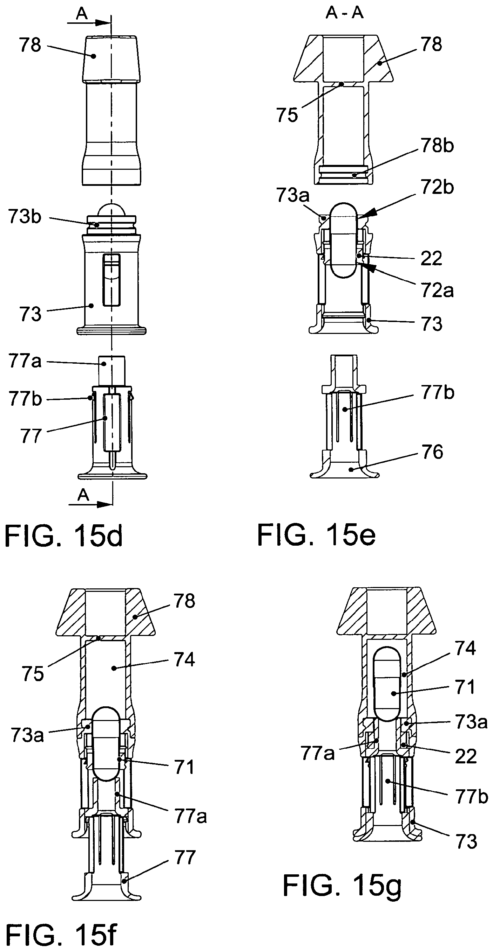

Fig. 15 eine schematische Darstellung der Funktionsweise einer sechsten Ausführungsform eines Inhalators und des Aufbaus des Systems aus Kapsel (ähnlich der sechsten oder neunten Ausführungsform) und Inhalator: Fig. 15a zeigt ein Kapselelement mit zugehöriger ringförmiger Halterung, Fig. 15b die Röhre des Inhalators, in welche Kapsel und Ring eingesetzt werden, Fig. 15c den Verschluss der Kapsel in der Röhre, Fig. 15d eine Explosionsdarstellung der Bauteile bzw. Bauteilgruppen des Inhalators, Fig. 15e die gleiche Explosionsdarstellung in schematischem Längsschnitt, Fig. 15f das fertig zusammen gesetzte System aus Kapsel und Inhalator im Transportzustand und Fig. 15g das System im anwendungsbereiten Zustand.

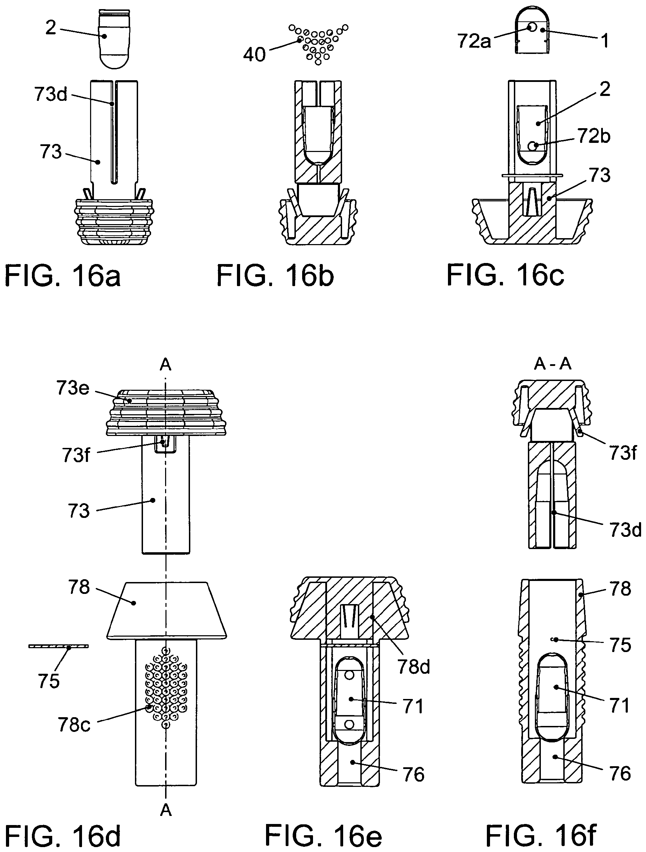

Fig. 16 eine schematische Darstellung der Funktionsweise einer siebten Ausführungsform eines Inhalators und des Aufbaus des Systems aus Kapsel (ähnlich der sechsten oder neunten Ausführungsform) und Inhalator: Fig. 16a zeigt den Einsatz eines Kapselelements in eine Röhre, Fig. 16b die Befüllung des Kapselelements mit Pulver im schematischen Längsschnitt durch die Röhre, Fig. 16c den Verschluss der Kapsel in der Röhre im schematischen, gegenüber Fig. 16b um 90° gedrehten Längsschnitt der Röhre, Fig. 16d eine Explosionsdarstellung der Bauteile bzw. Bauteilgruppen des Inhalators, Fig. 16e einen schematischem Längsschnitt des fertig zusammen gesetzten System aus Kapsel und Inhalator im Transportzustand und Fig. 16fb einen schematischen Längsschnitt des Systems aus Kapsel und Inhalator im Anwendungszustand.
Fig. 17 einen schematischen Längsschnitt eines Systems aus zwei Kapseln (ähnlich der sechsten oder neunten Ausführungsform) und einem Inhalator gemäß einer achten Ausführungsform.
Fig. 18 eine schematische Darstellung einer zehnten bis vierzehnten Ausführungsform der erfindungsgemäßen Kapsel: Fig. 18a zeigt die Kapsel mit Füllung, Fig. 18b zeigt die Kapsel gemäß der zehnten, Fig. 18c der elften, Fig. 18d der zwölften, Fig. 18e der dreizehnten und Fig. 18f der vierzehnten Ausführungsform.
Fig. 19 eine schematische Darstellung zur Funktionsweise der neunten Ausführungsform eines Inhalators: Fig. 19a zeigt die Befüllung der Kapsel in der Kapselkammer im Gerät, Fig. 19b den anschließenden Verschluss der Kapselkammer und Fig. 19c das Verhalten der Kapsel bei Benutzung des Inhalators.
Fig. 20 eine schematische Darstellung zur Befüllung der Kapsel in der neunten Ausführungsform des Inhalators: Fig. 20a zeigt die Kapsel in der Kapselkammer vor Befüllung und Fig. 20b während der Befüllung
Fig. 21 eine schematische Darstellung des Pulver-Magazins zum Einsatz in der neunten oder zehnten Ausführungsform des Inhalators und der Entleerung des Magazins: Fig. 21a zeigt eine Pulver-Dosis im Magazin im Lagerzustand, Fig. 21b zu Beginn des Entleerungsvorgangs und Fig. 21c zum Ende des Entleerungsvorgangs
Fig. 22 eine schematische Darstellung zur Befüllung der Kapsel in einer zehnten Ausführungsform des Inhalators: Fig. 22a zeigt die Kapsel in der Kapselkammer vor Befüllung und Fig. 22b während der Befüllung
Fig. 23 eine schematische Darstellung zur Funktionsweise der elften Ausführungsform eines Inhalators: Fig. 23a zeigt die offene Kapsel mit vorgefertigtem Loch, Fig. 23b die offene Kapsel bevorratet in einem Inhalator und Fig. 23c die offene Kapsel nach Verschiebung aus ihrem Lagerzustand in die Kapselkammer des Inhalators.

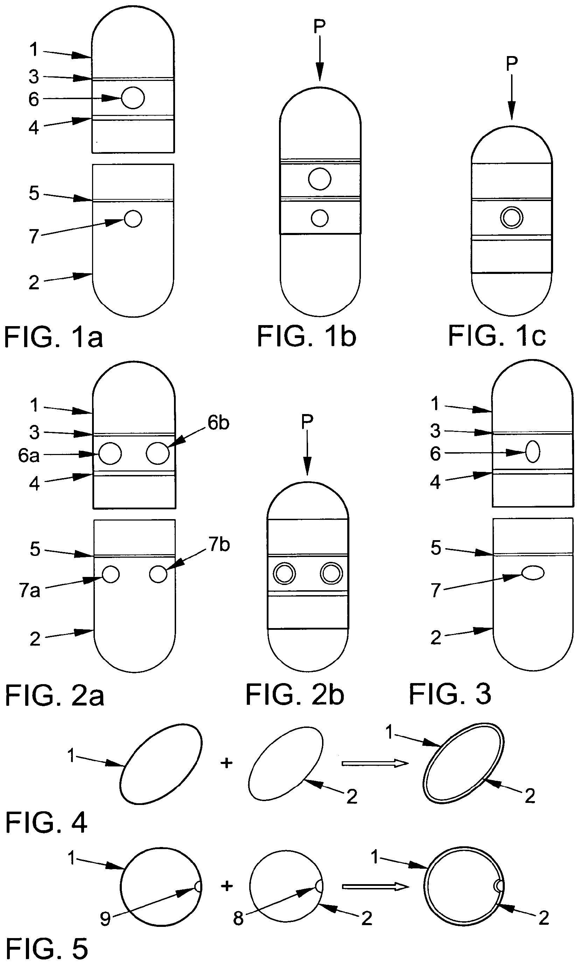

Figur 1 zeigt in schematischer Darstellung eine erfindungsgemäßen Kapsel bestehend aus Kapselkappe (1) und Kapselkörper (2), die beide eine becherartige Form haben und teleskopartig über ihre Öffnungen ineinander gesteckt werden können. Diese und alle nachfolgenden Darstellungen sind als Skizzen zu verstehen, in denen Wandstärken und ähnliche Details nicht in vollem Umfang dargestellt bzw. teilweise nicht unbedingt maßstabsgerecht zueinander abgebildet sind.
Die in dieser und den folgenden Figuren dargestellten Kapseln werden vorzugsweise mit einer pulverförmigen Arzneimittelzubereitung gefüllt. Bevorzugt haben die Kapselkappe (1) und der Kapselkörper (2) die Form eines auf einer Seite offenen Zylinders mit rundem Querschnitt und konvexer, nahezu halbkugelförmiger geschlossener anderer Seite. Kapselkappe (1) und Kapselkörper (2) bestehen beide vorzugsweise aus Polypropylen (PP) oder Polyethylen (PE), besonders bevorzugt aus high-density Polyethylen mit einer Dichte zwischen 950 und 1000 kg/m³. Alternativ sind auch Ausführungsformen möglich bei denen Kapselkappe (1) und Kapselkörper (2) aus unterschiedlichen Materialien sind, beispielsweise Kapselkörper aus PP oder PE und die Kapselkappe aus Gelatine. Die Kapselgrößen sind an die jeweiligen Inhalatoren bzw. die Dimensionen der darin enthaltenen Kapselkammern angepasst, in denen sie eingesetzt werden sollen. Typische Längen der zusammengefügten Kapseln sind beispielsweise 9 mm bis 22 mm bei äußeren Durchmessern von 4 mm bis 10 mm. Beispiele für die Kapseldimensionen sind der Offenbarung der WO2006/074982 A2 auf Seite 6 Zeile 6 bis 27 zu entnehmen. Der Inhalt jener referenzierter Zeilen sei hiermit vollumfänglich mit aufgenommen.

Bezüglich der Materialauslegung der Kapsel, für die neben dem hier bevorzugten Polyethylen auch alle pharmazeutisch akzeptablen Kunststoffe in Frage kommen, wird in dieser Hinsicht Bezug genommen auf die Offenbarung in der Anmeldung WO2006/074982 A2 auf Seite 5 Zeile 6 bis 31. Der Inhalt jener Zeilen wird hiermit vollumfänglich, auch zur Aufnahme von Merkmalen, in die vorliegende Anmeldung mit aufgenommen.

Figur 1a zeigt die beiden getrennten Kapselelemente (1) und (2) mit den vorgefertigten Löchern (6) und (7) vor dem Ineinanderstecken. In der hier dargestellten Ausführungsform wird beim teleskopartigen Ineinanderschieben die Kapselkappe (1) auf den Kapselkörper (2) aufgesetzt. (Auch der umgekehrte Fall des Einschiebens der Kapselkappe (1) in den Kapselkörper (2) hinein ist denkbar; für diesen Fall müssten alle nachfolgenden Beziehungen bzgl. "innen" und "außen" gerade umgekehrt betrachtet werden). Für die hier gewählte Ausführungsform mit Aufsetzen der Kapselkappe (1) auf den Kapselkörper (2) ist der äußere Durchmesser der Kapselkappe (1) im Bereich ihrer Becheröffnung etwas größer als der Kapselkörper (2). Der äußere Durchmesser des Kapselkörpers (2) ist an dieser Stelle von vergleichbarer Größe wie der innere Durchmesser der Kapselkappe (1), wobei die Durchmesser von ihren Toleranzen her so aufeinander abgestimmt sind, dass die Kapselelemente beim Fügen ohne nennenswerte Spaltbildung im Bereich der Kapselmantelbereiche ineinander passen. In der gewählten Darstellung bildet somit nach Ineinanderstecken der Kapselelemente der Kapselkörper (2) die innere Wand der Kapsel im Bereich der zwei aufeinander liegenden Mantelbereiche der Kapselelemente.
Die Kapsel wird mit der vorzugsweise pulverförmigen Arzneimittelzubereitung gefüllt, z.B. durch Einfüllen der Zubereitung in den Kapselkörper. Nach der Füllung wird die Kapselkappe (1) bis zu einer ersten Einsteckstellung auf den Kapselkörper (2) aufgeschoben. Der mit "p" gekennzeichnete Pfeil in den Abbildungen kennzeichnet die Richtung, in der die Kapselelemente zusammengeschoben werden bzw. zusammengeschoben worden sind. Er soll des Weiteren den Druck symbolisieren, der für dieses Zusammenschieben angewendet werden muss.
In der ersten Einsteckstellung (siehe Fig. 1b) greifen Rastelemente am äußeren Mantelbereich des Kapselkörpers (2) und Rastelemente am inneren Mantelbereich der Kapselkappe (1) ineinander. In allen hier dargestellten Figuren sind diese Rastelemente in Form von ringförmig umlaufenden Vorsprüngen oder Wülsten und Nuten oder Sicken dargestellt. So hat der Kapselkörper (2) in Figur 1 beispielsweise eine nach außen gerichtete umlaufende Wulst (5), die in der ersten Einsteckstellung in eine erste ringförmig umlaufende Sicke (4) innen an der Kapselkappe (1) einrastet. Die gleiche Wirkung würde erzielt durch eine ringförmige Nut im äußeren Mantelbereich des Kapselkörpers (1), wobei in der Einsteckstellung ein ringförmig umlaufender Vorsprung am inneren Mantelbereich der Kapselkappe (1) in diese Nut einrastet. Die Rastelemente müssen jedoch nicht zwangsläufig ringförmig ausgebildet sein, sondern können auch durch eher punktförmige Erhebungen in Kapselkörper und passende Vertiefungen in Kapselkappe bzw. umgekehrt gebildet werden. In einer bevorzugten Ausführungsform weist der Kapselkörper mehrere punktförmige, ringförmig angeordnete Erhebungen auf, die in eine entsprechende, vorzugsweise ringförmig umlaufende, Nut außen an der Kapselkappe einrasten. Bezüglich der Auslegung einer solchen eher punktuell gebildeten Verrastung sei hiermit als Beispiel auf die Offenbarung der WO2006/074982 A2 auf Seite 7 Zeile 1 bis Seite 8 Zeile 32 verwiesen. Der Inhalt jener referenzierter Zeilen sei hiermit vollumfänglich mit aufgenommen. In der ersten Einsteckstellung (siehe Figur 1b) überlappen sich die Mantelbereiche von Kapselkörper (2) und Kapselkappe (1) vorzugsweise so, dass der Mantel des Kapselkörpers (2) das Loch (6) in der Kapselkappe (1) von innen abdeckt und der Mantel der Kapselkappe (1) das Loch (7) im Kapselkörper (2) von außen abdeckt. Die Kapsel ist in dieser ersten Einsteckstellung komplett geschlossen.

In der zweiten Einsteckstellung (siehe Fig. 1c) sind Kapselkappe (1) und Kapselkörper (2) soweit ineinander geschoben, dass die jeweiligen Löcher (6) und (7) einander überdecken. Die Kapsel ist in dieser zweiten Einsteckstellung also "geöffnet" in dem Sinne, dass Pulver aus dem Inneren der Kapsel ausgebracht werden kann. Das vorgefertigte Loch (6) im hier außen liegenden Mantelbereich der Kapselkappe (1) ist größer als das Loch (7) des innen liegenden Mantelbereichs des Kapselkörpers (2). Dadurch dass eines der beiden Löcher kleiner als das andere ist, wird sichergestellt, dass auch bei Unregelmäßigkeiten beim Ineinanderstecken von Kapselkappe (1) und Kapselkörper (2) es nicht zur teilweisen Abdeckung des für die Ausbringung des Pulvers vorgesehenen Lochdurchmessers kommt. Die Größe des innenliegenden Loches (7) bestimmt in diesem bevorzugten Fall die Gesamtgröße des Lochs in der zusammengeschobenen Kapsel. Auf diese Weise kommt es auf der Innenseite der Kapsel nicht innen am Loch zu einer Stufe, an der bei Ausbringung pulverförmiges Material hängen bleiben könnte. Für die zweite Einsteckstellung sind an der Kapselkappe (1) ähnliche Rastelemente wie für die erste Einsteckstellung vorgesehen. Dementsprechend weist die Kapselkappe (1) im Darstellungsbeispiel von Figur 1 eine zweite Sicke (3) auf, in welche die Wulst (5) oder ein anderes vorspringendes Rastelement am inneren Mantelbereich des Kapselkörpers (2) einrasten kann. Dadurch werden die beiden Löcher (6) und (7) in ihrer Überdeckung gehalten und die Kapselelemente können sich auch bei Bewegung der Kapsel nicht mehr relativ zueinander verschieben. Die für die erste und zweite Einsteckstellung benötigten Rastelemente können beispielsweise durch Formgestaltung im Spritzguss von Kapselkappe (1) und Kapselkörper (2) oder durch Materialverformung an den Bauteilen realisiert werden. So kann beispielsweise eine innen im Kapselkörper umlaufende Wulst (5) mit einer außen umlaufenden Sicke einhergehen.

Zusätzlich zu der beschriebenen, als makroskopisch anzusehenden, Strukturierung der Kapselelemente weist eine weitere Ausführungsform der erfindungsgemäßen Kapsel eine Mikro- oder Nanostruktur oder Oberflächenbeschichtung innen an einem Kapselelement auf. Dies ist insbesondere jenes Kapselelement, das bei aneinanderliegenden Kapselmänteln die Außenwand der Kapsel bildet - die Kapselkappe (1) im Beispiel von Figur 1. Die Mikrostruktur befindet sich hierbei vorzugsweise innen an der dem anderen Kapselelement zugewandten Mantelfläche. Insbesondere erstreckt sich die Mikrostruktur über einen in einem ringförmigen Bereich der inneren Mantelfläche, wobei dieser ringförmige Bereich beim Vorliegen der Kapsel in der ersten Einsteckstellung (Figur 1a) eine direkte Wand des Hohlraums der Kapsel bildet und beim Vorliegen der Kapsel in der zweiten Einsteckstellung (Figur 1c) an der äußeren Mantelfläche des anderen Kapselelementes (des Kapselkörpers (2) im Beispiel von Figur 1) anliegt.
Diese Mikrostruktur bewirkt einen so genannten Lotus-Effekt, d.h. dass sie die Anhaftung von bestimmtem Material an dieser Oberfläche reduziert. Um den optimalen Effekt zu erzielen muss die Art der Mikrostruktur so gewählt sein, dass sie die geringsten Hafteigenschaften für die definierte pharmazeutische Zubereitung bietet, die in dem entsprechenden Kapseltyp bevorratet werden soll. Auf diese Weise haftet kein oder nur sehr wenig Material aus der pharmazeutischen Zubereitung, beispielsweise Pulver, an der Innenwand der Kapsel an. Dies hat insbesondere in dem beschriebenen ringförmigen Bereich den Effekt, dass es beim Zusammenschieben der Kapsel von der ersten zur zweiten Einsteckstellung nicht zu Reibung durch an der Wand anhaftendes Material kommt. Eine Ausdehnung der Mikrostruktur auf alle inneren Wandbereiche der Kapsel ist ebenfalls möglich und hat den Effekt, dass kein Material durch Wandanhaftung in der Kapsel verbleibt, wenn das Material während eines Zerstäubungsvorgangs ausgebracht wird.
Die Mikrostruktur wird durch Erhebungen und/oder Vertiefungen in der Oberfläche gebildet. Die Erhebungen und/oder Vertiefungen können die Form von Spitzen, Halbkugeln, planaren Flächen, Keilen etc. haben. Sie können eine zufällige Anordnung aufweisen oder geordnet sein, z.B. in Reihen, Kreisen, zick-zack-förmig, meanderförmig etc.
Der Abstand zwischen den Erhebungen der Oberflächenstruktur liegt im Bereich von 0,1 bis 200 Mikrometer, vorzugsweise 0,1 bis 100 Mikrometer. Bei pulverförmigen pharmazeutischen Zubereitungen sind hierbei Strukturdimensionen bevorzugt, die kleiner sind als die Korngrößen des Pulvers. Am stärksten bevorzugt sind Höhen von Erhebungen bzw. Tiefen von Vertiefungen im Bereich von 0,1 bis 50 Mikrometer und Abstände von 0,1 bis 10 Mikrometer.

Bezüglich von Verfahren zur Erzeugung einer solchen Mikrostruktur und ihrer Eigenschaften sei hiermit auf die Offenbarung der WO2004/062716 A1 auf Seite 11 Zeile 1 bis Seite 13 Zeile 13 verwiesen. Der Inhalt jener Zeilen wird hiermit vollumfänglich, auch zur Aufnahme von Merkmalen, in die vorliegende Anmeldung mit aufgenommen.
Bevorzugt werden für die Anbringung einer Mikrostruktur an Kapselinnenwänden solche Verfahren, die kein zusätzliches Material in die Kapseln hinein bringen, d.h. solche Mikrostrukturen, wie sie allein in dem das jeweilige Kapselelement bildenden Material abgeformt werden können. Im bevorzugten Fall von Kapselelementen, die in Spritzgussverfahren hergestellt werden, sind die Mikrostrukturen vorzugsweise bereits in den jeweiligen Formeinsätzen der Spritzgusswerkzeugen sozusagen spiegelbildlich abgebildet, so dass die Kapselelemente diese Mikrostrukturen wie auch die vorgefertigten Löcher bereits im ersten Fertigungsschritt erhalten. Alternativ können solche Mikrostrukturen an den Kapselinnenwänden auch durch subtraktive Oberflächenbehandlung wie Ätzen oder galvanische Abtragung oder durch nachträgliches Prägen geschaffen werden, wiebeispielsweise mittels eines aufspreizbaren Stempels, der in die Hauptöffnung des Kapselelements eingeführt wird.

Figur 2 zeigt eine zweite Ausführungsform einer aus einer Kapselkappe (1) und einem Kapselkörper (2) zusammengesetzten Kapsel. Diese Ausführungsform unterscheidet sich von der ersten nur dadurch, dass Kapselkappe (1) und Kapselkörper (2) jeweils mehrere vorgefertigte Löcher (6a) und (6b) bzw. (7a) und (7b) aufweisen. In der nicht gezeigten ersten Einsteckstellung sind alle diese Löcher durch das jeweils andere Kapselelement analog zum ersten Ausführungsbeispiel verschlossen. In der zweiten Einsteckstellung (Figur 2b) überdecken sich die jeweiligen Löcher (6a) und (6b) der Kapselkappe (1) mit den Löchern (7a) und (7b) des Kapselkörpers (2), so dass die Kapsel an mehreren Stellen geöffnet ist. Im Darstellungsbeispiel sind jeweils zwei Löcher (6a, 6b) bzw (7a, 7b) in Kapselkappe (1) und Kapselkörper (2) abgebildet, die in zwei Öffnungen an der Kapsel in der zweiten Einsteckstellung resultieren. Auf diese Weise können jedoch beliebig viele Löcher vorgesehen werden. Die Löcher können je nach Gestaltung der Mantelbereiche von Kapselkappe (1) und Kapselkörper (2) über Umfang und Länge der Kapsel verteilt werden. Besonders bevorzugt weist die resultierende geöffnete Kapsel zwei Löcher auf, die sich auf dem Mantelbereich der Kapsel jeweils nahe der entgegengesetzten Enden der Kapsel befinden, also bezogen auf die Länge der Kapsel deutlich voneinander beabstandet sind.

Figur 3 zeigt eine dritte Ausführungsform einer aus einer Kapselkappe (1) und einem Kapselkörper (2) zusammensetzbaren Kapsel. Diese Ausführungsform unterscheidet sich von der ersten nur dadurch, dass die Löcher (6) und (7) in Kapselkappe (1) und Kapselkörper (2) statt einer kreisförmigen eine elliptische Gestalt haben. Die Längsachsen dieser Ellipsen sind relativ zueinander um 90° verdreht. Auf diese Weise wird bei Unregelmäßigkeiten beim Zusammenschieben sichergestellt, dass die bei der Überdeckung zustande kommenden Öffnungen immer eine gleichbleibende Gesamtfläche einnehmen. Statt einer elliptischen Lochform können an dieser Stelle auch entsprechend ausgerichtete Langlöcher verwendet werden.

Figur 4 und 5 zeigen Kapselkörper (2) und Kapselkappe (1) schematisch im Querschnitt vor und nach dem Ineinanderstecken für verschiedene Ausführungsformen. Damit beim Ineinanderstecken von Kapselkappe (1) und Kapselkörper (2) die jeweiligen Löcher (6) und (7) sauber zur Überdeckung gebracht werden können, müssen die Kapselelemente bezogen auf ihren Umfang zueinander ausgerichtet ineinandergesteckt werden. Dies erfordert eine Struktur und Gegenstruktur ähnlich eines Schlüssel-Schloss-Prinzips an den Kanten bzw. jeweiligen Mantelflächen von Kapselkappe (1) und Kapselkörper (2). Des Weiteren ist eine Längsführung von Vorteil, die bewirkt, dass es nach gesteuertem erstem Ineinanderstecken der Kapselelemente zu keiner unerwünschten Verdrehung der Kapselelemente zueinander mehr kommen kann. In dem Ausführungsbeispiel gemäß Figur 4 sind Kapselkappe (1) und Kapselkörper (2) gleichermaßen mit leicht elliptischem Querschnitt gestaltet. Die Ellipsenform erzwingt hierbei das orientierte Ineinanderstecken der beiden Kapselelemente. Bei der Zusammenführung der elliptischen Kapselelemente ergeben sich folglich zwei mögliche, um 180° zueinander verdrehte Orientierungen. Vorzugsweise sollte daher eines der beiden Kapselelemente, besonders bevorzugt das äußere (in diesem Beispiel also die Kapselkappe (1)), nicht nur das mindestens eine vorgefertigte Loch (6) aufweisen sondern auch eine Duplizierung der Lochstruktur um 180° entlang des Umfangs des Kapselelements versetzt aufweisen. Somit wird unabhängig von der Orientierung beim Zusammenführen zweier elliptischer Kapselelemente immer ein Loch (6) beim Ineinanderschieben in die zweite Einsteckstellung freigegeben.
In dem Ausführungsbeispiel gemäß Figur 5 weist der Außenbereich des Kapselkörpers (2) zusätzlich zu den nicht gezeigten Rastelementen einen parallel zur Längsachse verlaufenden Steg (8) auf, dessen Kontur zu einer ebenfalls längs, innen an der Kapselkappe (1) angebrachten Nut (9) passt. Die Kapselelemente lassen sich nur in der azimutalen Ausrichtung, in welcher der Steg (8) und die Nut (9) ineinander greifen, zusammen schieben. Steg (8) und Nut (9) bilden eine Führung über die gesamte Einschublänge. Wahlweise können auch mehrere unterschiedlich zueinander beabstandete und/oder unterschiedliche breite Steg-und-Nut-Paare an den Mantelflächen der Kapselelemente angeordnet sein.
In einer nicht dargestellten Ausführungsform kann das gezeigte Strukturpaar aus Steg (8) und Nut (9) auch eine Kurvenbahn aufweisen, durch welche ein Zusammenschieben der Kapselelemente eine Drehung derselben relativ zueinander erzwingt. Dies kann insbesondere bei dem gezielten Verdecken und Freilegen von mehreren Löchern in der Kapsel von Vorteil sein.

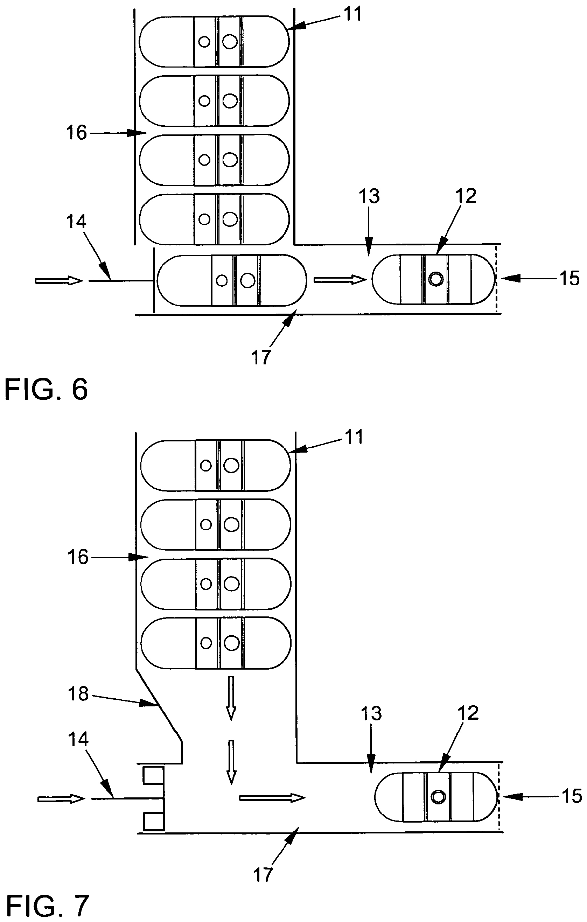

Figur 6 zeigt schematisch die Funktionsweise eines Inhalators, in dem mehrere erfindungsgemäße Kapseln mit vorgefertigten Löchern bevorratet sind. In dieser Ausführungsform sind mehrere Kapseln (11) in erster Einsteckstellung in einem Magazin bevorratet (Die Darstellung der Kapsel (11) in erster Einsteckstellung ist in Fig. 6,7 und 8 bezüglich der Lochpositionen nicht funktionsgetreu und daher hier nur rein schematisch zu verstehen). Das Magazin ist in Form eines vertikalen Schachts (16) dargestellt, dessen Querschnitt an die Längsausdehnung der Kapsel (11) angepasst ist, d.h. beispielsweise rechteckig oder langlochförmig mit Breiten nur wenig größer als Länge und Durchmesser der Kapsel (11). Die Kapseln, die sich in ihrem ersten Verrastungszustand befinden, sind zunächst im Magazin gestapelt. Die jeweils zur Anwendung kommende Kapsel (11) wird in einen Kanal (17) gefördert, entlang dessen vorzugsweise auch die im Inhalator wirkende Luftströmung verläuft. Diese Förderung kann entweder allein auf der Wirkung der Schwerkraft beruhen oder durch eine oben am Magazin angebrachte Federkraft unterstützt werden. In dem Kanal (17) wird die Kapsel (11) durch einen Schieber (14) unmittelbar vor der Anwendung des Inhalators in die zweite Einsteckstellung zusammengeschoben, so dass sich die jeweiligen Löcher von Kapselkappe und Kapselkörper übereinander befinden. Die exakte Zusammenführung wird durch entsprechende Dimensionierung gesichert. Der Schieber (14) kann eine Lufteintrittsöffnung aufweisen und somit die Wand der Kapselkammer (13) bilden, in der das Pulver im Luftstrom aus der Kapsel (12) ausgebracht werden soll. Auf diese Weise kann unmittelbar nach der Kapselöffnung die Inhalation des Kapselinhaltes erfolgen. Dabei muss der Schieber (14) durch entsprechende Konstruktion so arretiert werden, dass er zusammen mit der Begrenzung der Kapselkammer (13) in Schubrichtung eine Kapselkammer (13) bildet, deren Länge derart auf die Länge der Kapsel (12) in ihrer zweiten Einsteckstellung angepasst ist, dass sich die Kapsel (12) gemäß dem Bernoulli-Effekt im Luftstrom bewegen und vibrieren kann. Der Schieber (14) kann somit mindestens drei Positionen im Kanal (17) einnehmen: Eine erste Position, in der eine Kapsel (11) aus dem Magazin in den Kanal (17) gefördert werden kann, eine zweite, ganz in den Kanal (17) eingeschobene Position, in der die Kapsel in ihre zweite Einsteckstellung zusammen geschoben wird (gleichzeitig stößt sie an die Begrenzung der Kapselkammer (13) in Richtung Luftauslass), und eine dritte, gegenüber der zweiten leicht zurückgezogene Position, in der der Schieber die Lufteinlass-seitige Begrenzung der Kapselkammer (13) während der Zerstäubung bildet. Die Begrenzung der Kapselkammer (13) in Richtung des Luftauslasses wird vorzugsweise durch ein Sieb (15) oder durch ein aerodynamisch vorteilhaft geformtes Bauteil gebildet, das nur einen geringen Strömungswiderstand darstellt. An diese den Luftauslass beinhaltende Begrenzung der Kapselkammer (13) schließt sich ein nicht dargestelltes Mundstück an, an dem der ebenfalls nicht dargestellte Anwender inhaliert und dadurch den zur Zerstäubung des Kapselinhalts notwendigen Luftstrom erzeugt.
Um nach Ausbringung der pharmazeutischen Zubereitung aus der Kapsel (12) die Kapselkammer (13) wieder mit einer neuen Kapsel (11) bestücken zu können, ist vorzugsweise eine Möglichkeit zum Öffnen der Kapselkammer (13) zur Entnahme der gebrauchten Kapsel (12) am Inhalator angebracht. Diese Öffnungsmöglichkeit kann beispielsweise durch eine verschließbaren Klappe in der Wand des Kanals (17) auf der dem Schacht (16) abgewandten Seite der Kapselkammer (13) gebildet werden.

Figur 7 zeigt schematisch die Funktionsweise einer weiteren Ausführungsform eines Inhalators, in dem mehrere erfindungsgemäße Kapseln mit vorgefertigten Löchern bevorratet sind. In dieser Ausführungsform sind mehrere Kapseln (11) in erster Einsteckstellung in einem schachtförmigen Magazin bevorratet. In dieser Ausführungsform weist der Schacht (16) an seinem Ausgang zum Kanal (17) bzw. zur Kapselkammer (13) eine Verjüngung auf, wobei eine Wand in diesem Verjüngungsbereich als schiefe Ebene oder Rampe oder vorzugsweise als konische Führungsschiene (18) ausgebildet ist. Der Schachtdurchmesser am Ende der Verjüngung bzw. am Schachtausgang entspricht der Größe der Kapsel (12) in zweiten Einsteckstellung, d.h. im vollständig ineinander geschobenen Zustand. Durch oben am Schacht (16) angelegten Druck auf die Kapseln (11)-der Druck wird hierbei von Kapsel (11) zu Kapsel (11) von oben nach unten übertragen - erzwingt die gleichmäßige Verkleinerung des Schachtdurchmessers eine Verkleinerung der Kapsellänge, d.h. die schiefe Ebene oder Rampe oder konische Führungsschiene (18) bewirkt ein weiteres Ineinanderschieben von Kapselkörper (2) und Kapselkappe (1) bis am Schachtausgang die Kapsel (2) in der zweiten Einsteckstellung vorliegt.
Im Darstellungsbeispiel in Figur 7 ist unabhängig von dem Zusammenschieben der Kapsel (11) am Ende des Schachts (16) ein Schieber (14) im Kanal (17) angeordnet. Dieser Schieber (14) hat hier vor allem die Aufgabe, die Kapsel (12) in die Kapselkammer (13) zu schieben. Andere Transportmöglichkeiten von Magazin zu Kapselkammer (13) sind ebenfalls möglich, z.B. über Klappen in der Wand der Kapselkammer (13). Der Schieber (14) in diesem Ausführungsbeispiel nimmt vor allem zwei Positionen ein: Eine erste Position, in der die bereits zusammengeschobene Kapsel (12) aus dem Magazin in den Kanal (17) gefördert werden kann, und eine weitere Position, in der der Schieber die Lufteinlass-seitige Begrenzung der Kapselkammer (13) während der Zerstäubung bildet und die Kapsel (12) in der Kapselkammer (13) Spielraum für Vibration und/oder Bewegung hat.

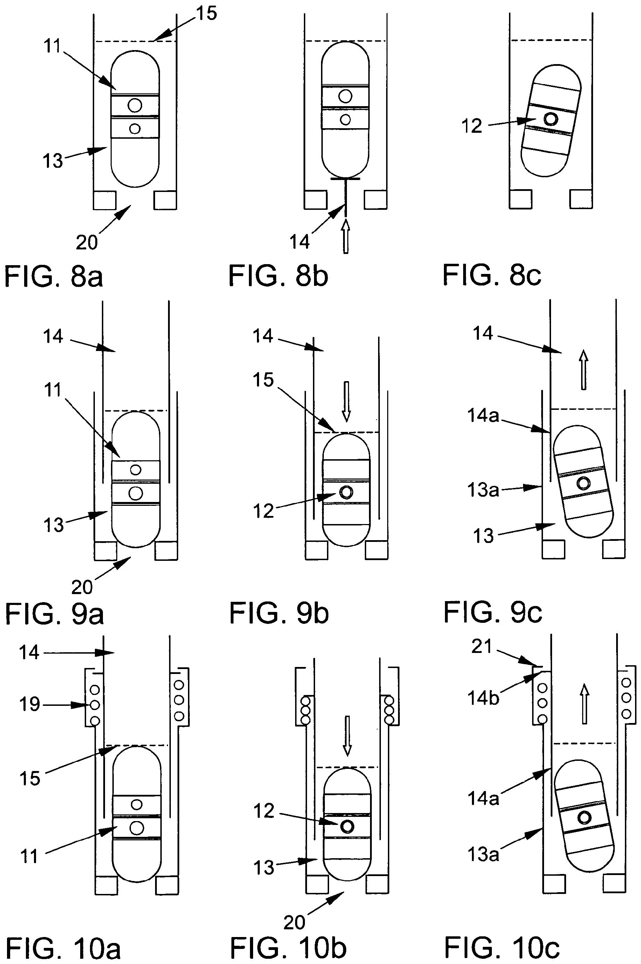

Die Figuren 8, 9 und 10 zeigen anhand verschiedener Ausführungsformen schematisch die Funktionsweise eines Inhalators, in dem sich eine erfindungsgemäße Kapsel mit vorgefertigten Löchern bereits in einer Kapselkammer (13) befindet. Die hier gezeigten Funktionsweisen sind zwar jeweils auch auf die befüllte Kapselkammer eines Inhalators mit mehreren bevorrateten Kapseln anwendbar, in erster Linie beziehen sich die hier gezeigten Ausführungsformen jedoch auf Inhalatoren zur einmaligen Anwendung. Die hier vorgestellten Inhalatoren bestehen vom Prinzip her lediglich aus einer Kapselkammer (13) mit voreingesetzter Kapsel (11), einem gegebenenfalls mittels einer Feder (19) rückstellbaren Schieber (14) und einem Mundstück mit Inhalationskanal, an den ein Luftauslass aus der Kapselkammer (13) angeschlossen ist. Die Kapselkammer (13) weist neben besagtem Luftauslass einen Lufteinlass auf. Die Begrenzung der Kapselkammer (13) wird vorzugsweise durch ein Sieb (15) oder ein aerodynamisch vorteilhaft geformtes Bauteil oder Element gebildet, das nur einen geringen Strömungswiderstand darstellt.
In Figur 8a ist eine solche Kapselkammer (13) mit voreingesetzter Kapsel (11) in der ersten Einsteckstellung zu sehen. Die Kapselkammer (13) weist eine Lufteinlassöffnung (20) auf, durch die in Figur 8b ein Stößel oder Schieber (14) eingeführt wird, mit dem die Kapsel (11) gegen das Sieb (15), d.h. gegen die Begrenzung der Kapselkammer (13) auf der Seite des Luftauslasses geschoben wird. Durch weiter in Richtung des Luftauslasses der Kapselkammer (13) gerichteten Druck werden Kapselkappe (1) und Kapselkörper (2) weiter ineinander geschoben, bis die Kapsel (12) bis zu ihrer zweiten Einsteckstellung zusammengeschoben ist. Anschließend (Figur 8c) wird der Stößel oder Schieber (14) wieder aus der Kapselkammer (13) herausgezogen und die zusammengeschobene Kapsel (12) steht zur Inhalation bereit: Ihre Löcher sind zur Ausbringung des vorzugsweise pulverförmigen, innenliegenden Materials geöffnet, und sie hat in der Kapselkammer (13) Bewegungsspielraum für Vibration und/oder Bewegung. In der in Figur 8a gezeigten Situation der voreingesetzten Kapsel (11) in erster Einsteckstellung ist kein Bewegungsspielraum für die Kapsel notwendig, bzw. sollte so klein wie möglich gehalten werden, um ein zufälliges Verkippen der Kapsel beim Einschieben des Stempels oder Schieber (14) zu vermeiden.

Figur 9 zeigt einen Inhalator, in dem der Schieber (14) einen Teil der Kapselkammer (13) bildet, in den der Luftauslass integriert ist. Der Schieber (14) umfasst dabei die Begrenzung der Kapselkammer (13) auf der Seite des Luftauslasses. Zusätzlich kann er auch teleskopartig einen Teil der Seitenwand der Kapselkammer (13) bilden (siehe Fig. 9); Ausgestaltungen ohne dieses Merkmal sind aber ebenfalls möglich.
Figur 9a zeigt eine vorzugsweise im Wesentlichen zylindrische Kapselkammer (13) mit voreingesetzter Kapsel (11) in erster Einsteckstellung. Die Lufteinlassöffnung (20) ist hier mittig in der dem Luftauslass entgegengesetzten Seite der Kapselkammer (13), d.h. bezogen auf die Abbildung unten in der Kapselkammer (13). Vorzugsweise ist die Position des Schiebers (14) in dieser Situation der voreingesetzten Kapsel (11) so, dass die Kapsel (11) oben in der Kapselkammer (13) an deren Begrenzung, d.h. bevorzugt an einem Sieb (15) oder einem aerodynamischen Element, anliegt und unten mit ihrem abgerundeten Endbereich so in die Lufteinlassöffnung (20) eintaucht, dass sie diese verschließt. Auf diese Weise kann z.B. beim Transport des Inhalators nach Entnahme aus einer Umverpackung vermieden werden, dass Verunreinigungen in die Kapselkammer gelangen. In Figur 9b wird die Kapsel (11) mittels des Schiebers (14), genauer gesagt mit der zu ihm gehörenden Begrenzung der Kapselkammer (13), so gegen ihre Auflage im Bereich des Lufteinlasses gedrückt, dass Kapselkappe und Kapselkörper weiter ineinander geschoben werden bis die zweite Einsteckstellung der Kapsel (12) erreicht ist. In diesem Beispiel hat der Schieber (14) nicht nur eine Schubfläche in Richtung der Kapsel (11) sondern auch in die Kapselkammer (13) hineinragende Wandbereiche. Die Kapselkammer (13) wird hier gewissermaßen aus zwei mit den Öffnungen ineinander gesteckten Hohlzylindern gebildet, wodurch sie seitlich eine doppelte Wandung erhält, die aus der eigentlichen Längswand (13a) der Kapselkammer (13) und der inneren eingeschobenen Längswand (14a) des Schiebers (14) gebildet wird. Hierbei muss die Länge der eingeschobenen Längswand (14a) derart auf die Länge der Kapsel (12) in der zweiten Einsteckstellung abgestimmt sein, dass der Schieber (14) entsprechend weit genug in die Kapselkammer (13) eingeschoben werden kann. Nach Zusammenschieben der Kapsel (12) muss der Schieber (14) wieder beispielsweise in seine Ausgangsposition zurück gezogen und so arretiert werden, dass die Länge der Kapselkammer (13) für die Bewegung der Kapsel (12) bei der Inhalation fest fixiert und somit auch definiert ist. Vorzugsweise ist für die Vermeidung von Nebenluft während der Inhalation eine gleitende Dichtung zwischen der eingeschobenen Längswand (14a) des Schiebers (14) und der sie aufnehmenden Längswand (13a) der Kapselkammer (13) ausgebildet bzw. eingesetzt.

Vorzugsweise besteht der Inhalator gemäß der in Figur 9 gezeigten Funktionsweise neben der Kapsel (11) aus zwei Bauteilen, die zum Freigeben der Löcher der Kapsel (11) teleskopartig ineinander und zur anschließenden Anwendung des Inhalators wieder auseinander gezogen werden: Einem Bauteil, das den Teil der Kapselkammer (13) mit dem Lufteinlass beinhaltet, und einem weiteren Bauteil, das den Teil der Kapselkammer (13) mit dem Luftauslass, den Inhalationskanal und vorzugsweise auch das daran angeschlossene Mundstück beinhaltet. Bevorzugt sind beide Bauteile einstückig z.B. aus Kunststoff-spritzguss gefertigt. Je nach Ausgestaltung der Griffflächen außen am Inhalator kann jedes der beiden Bauteile in diesem Ausführungsbeispiel als "Schieber" bezeichnet werden: Entweder der Inhalator ist so gestaltet, dass er unten gehalten wird und das Mundstück wie in Figur 9b skizziert hinein gedrückt wird, oder er ist so gestaltet, dass man ihn an einem das Mundstück umfassenden Hauptkörper hält und das andere Bauteil hineindrückt.

Figur 10a, 10b und 10c zeigen einen Inhalator entsprechend dem in den Figuren 9a, 9b und 9c, wobei sich der Inhalator in Figur 10 lediglich dadurch vom vorherigen unterscheidet, dass er eine Feder (19) umfasst, die eine Rückstellung der beiden Bauteile nach ihrem Zusammenschieben ineinander bewirkt. In diesem Ausführungsbeispiel weist der Schieber (14) einen äußeren Vorsprung (14b) auf, mit dem die Feder (19) beim Schieben des Schiebers (14) in die Kapselkammer (13) gegen die Federkraft zusammen gedrückt wird. Wird der äußere Druck auf den Schieber (14) beendet, drückt die Feder (19) den Schieber (14) wieder über seinen Vorsprung (14b) nach außen und zwar gerade so weit wie es eine Anschlagkante (21) zulässt, welche zur Halterung der Feder (19) im Inhalator gehört und die maximale Ausdehnung der Feder (19) begrenzt.

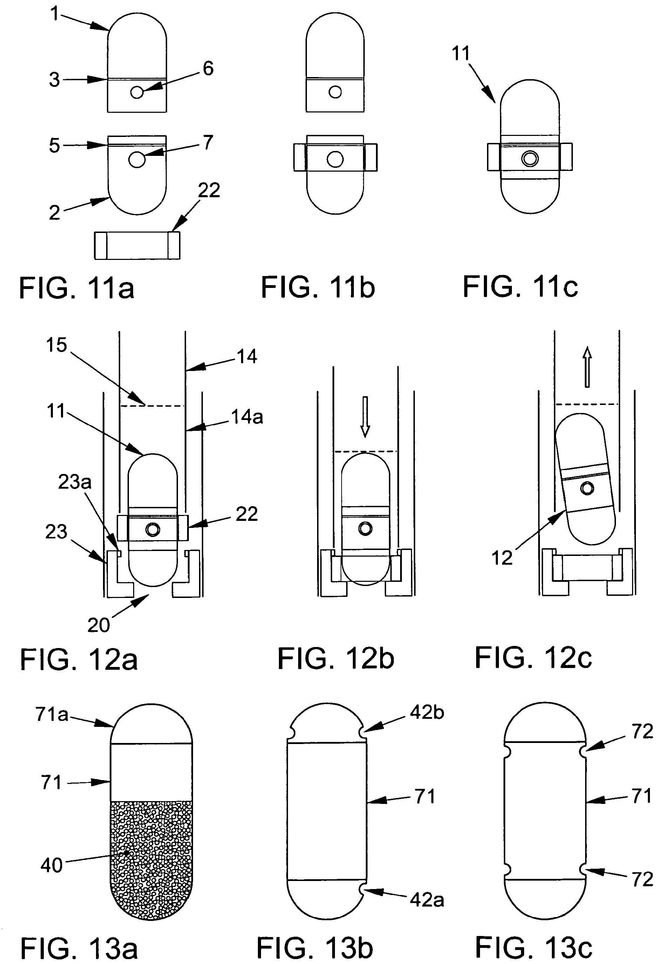

Figur 11 zeigt in schematischer Darstellung eine weitere Ausführungsform einer erfindungsgemäßen Kapsel bestehend aus Kapselkappe (1), Kapselkörper (2) und einem Ring (22). Kapselkappe (1) und Kapselkörper (2) haben analog zu dem Ausführungsbeispiel von Figur 1 beide ebenfalls eine becherartige Form und können teleskopartig über ihre Öffnungen ineinander gesteckt werden, wobei diesbezüglich anhand von Figur 1 beschriebene Aspekte hier ebenfalls ihre Gültigkeit haben. Aus Gründen der einfacheren Befüllung ist im Darstellungsbeispiel von Figur 11 abweichend zu den anderen Darstellungsbeispielen eine Ausführungsform gewählt worden, bei der die Kapselkappe (1) in den Kapselkörper (2) eingeschoben wird (Ausführungsformen mit außen aufgeschobener Kappe sind aber ebenfalls möglich). Der Kapselkörper (2) mit gegenüber der Kapselkappe (1) vergrößertem Loch (7) bildet hier also die äußere Wand der Kapsel im Bereich der zwei aufeinander liegenden Mantelbereiche der Kapselelemente, die Kapselmantelbereiche der beiden Kapselelemente weisen analog zu den vorherigen Beispielen miteinander wechselwirkenden Strukturierungen auf.
Figur 11a zeigt die beiden getrennten Kapselelemente (1) und (2) mit den vorgefertigten Löchern (6) und (7) vor dem Ineinanderstecken und vor Zusammenfügen mit dem Ring (22).
Figur 11b zeigt den Kapselkörper (2) nach Zusammenfügen mit dem Ring (22). Der Ring (22) verdeckt hierbei das Loch (7) des Kapselkörpers (2). Dies ist vorzugsweise die Situation, in der die pharmazeutische vorzugsweise pulverförmige Zubereitung in den Kapselkörper (2) eingefüllt wird, der dann wie in Figur 11c gezeigt mit der Kapselkappe (1) verschlossen wird. Die Kapselkappe (1) kann bei diesem Konzept direkt bis zum Erreichen der finalen Einsteckstellung in den Kapselkörper (2) geschoben werden.
Figur 11c zeigt die zusammengesetzte Kapsel (11), in der analog zum Ausführungsbeispiel von Figur 1 Kapselkappe (1) und Kapselkörper (2) mittels Rastelemente (3) und (5) miteinander verrastet sind. Die jeweiligen vorgefertigten Löcher (6) und (7) überdecken sich bei dieser zusammengesetzten Kapsel, das resultierende Loch wird in diesem Zustand jedoch vom Ring (22) verdeckt. Die Kapsel (11) kann so gelagert oder in einen geeigneten Inhalator ein gesetzt werden. Alternativ zu der hier gezeigten Kapsel (11) können bei diesem Konzept mit Abdichten der vorgefertigten Löcher (7) über ein weiteres z.B. ringförmiges Bauteil die Kapselelemente auch so gestaltet werden, dass sich nach Zusammensetzen nicht zwangsläufig jeweils ein Loch (7) in Kapselkörper (2) mit einem Loch (6) in Kapselkappe überdecken muss, sondern dass jeweils der Kapselmantel des anderen Kapselelements im zusammengeschobenen Zustand der Kapsel (11) so kurz ist, dass er das jeweilige Loch frei lässt. Bei diesem alternativen (nicht gezeigten Ausführungsbeispiel) weist der Kapselkörper (2) ein vorgefertigtes Loch (7), die Kapselkappe (1) hat zu diesem Loch (7) passendes Loch (6) und der Mantel der Kapselkappe (1) endet im zusammengeschobenen Zustand der Kapsel oberhalb des Loches (7) und lässt dieses frei.

Die Figur 12 zeigt schematisch die Funktionsweise eines Inhalators, dessen Kapselkammer (13) bereits mit einer erfindungsgemäßen Kapsel (11) befüllt ist, deren vorgefertigten Löcher gemäß des anhand des Ausführungsbeispiels gemäß Figur 11 geschilderten Prinzips über einen Ring von außen verschlossen sind. Ähnlich wie bei den in den Figuren 8, 9 und 10 gezeigten Funktionsweisen bezieht sich die hier gezeigte Ausführungsform bevorzugt auf einen Inhalator zur einmaligen Anwendung.
Figur 12 zeigt ähnlich zu Figur 9 einen Inhalator, in dem der Schieber (14) den Teil der Kapselkammer (13) bildet, in den der Luftauslass integriert ist. Der Schieber (14) umfasst dabei die Begrenzung der Kapselkammer (13) auf der Seite des Luftauslasses. Zusätzlich bildet der Schieber (14) teleskopartig einen Teil der Seitenwand der Kapselkammer (13). Der innere Durchmesser der sich so ergebenden eingeschobenen Längswand (14a) ist hierbei kleiner als der äußere Durchmesser des Rings (22), der die vorgefertigten Löcher (6) und (7) an der Kapsel (11) verschließt. Gleichzeitig ist der Durchmesser der eingeschobenen Längswand (14a) größer als der Durchmesser der im Wesentlichen zylinderförmigen Kapsel (12) ohne Ring (22).
In Figur 12b wird der Schieber (14) zum Lufteinlass hin bewegt, der sich in der Abbildung unten an der Kapselkammer (13) befindet. Hierbei stößt der untere Rand der eingeschobenen Längswand (14a) an den Ring (22). Dadurch wird vorzugsweise zunächst die ganze Kapsel (11) so nach unten gedrückt, dass sich das abgerundete untere Ende der Kapsel (11) so in die Lufteinlassöffnung (20) am Boden der Kapselkammer einfügt, dass die Kapsel sich insgesamt nach oben ausgerichtet und gleichzeitig sozusagen am unteren Anschlagspunkt anliegt (zusätzliches aber kein notwendiges Merkmal). Bei weiterem Druck auf den Schieber (14) von oben wird nun der Ring (22) auf dem äußeren Mantel der Kapsel (12) nach unten hin verschoben. Unten in der Kapselkammer (13) ist eine Aufnahme (23) für den Ring (22) ausgebildet oder eingesetzt. Die Unterkante der eingeschobenen Längswand (14a) des Schiebers (14) schiebt den Ring (22) in diese Aufnahme (23) über kleine Rastelemente (23a) hinweg, die den Ring (22) in seiner neuen Position in der Aufnahme (23) fixieren. In dieser Position des Rings (22) ist der Ring (22) von der Kapsel (12) getrennt worden und die übereinander liegenden vorgefertigten Löcher (6) und (7) liegen frei.
In einer anderen Ausführungsform (nicht in der Figur dargestellt) weist die Aufnahme (23) eine Nut auf, die den Ring (22) aufnimmt und einen Vorsprung an der Innenseite des Rings (22) bildet. Durch diesen Vorsprung wird die Kapsel (12) beim Eindrücken des Rings (22) in die Nut in Richtung Kapselkammer (13) vom Ring (22) gelöst. Der Vorsprung ist hierbei dann vorzugsweise so gestaltet, dass er analog zur Lufteinlassöffnung in der vorherigen Ausführungsform den unteren Anschlagspunkt für die Kapsel (12) in der Kapselkammer (13) bildet.
In Figur 12c wird der Schieber (14) wieder ein Stück in Richtung Luftauslass zurückgezogen, wodurch die Kapsel (12) Bewegungsspielraum für Vibration und/oder Bewegung erhält. Die Kapsel ist somit für die Ausbringung der innenliegenden pharmazeutischen Zubereitung vorbereitet.
In einer weiteren Ausführungsform ist am Schieber (14) analog zur Ausführungsform aus Figur 10 einer Feder (19) angeordnet, die eine Rückstellung des Schiebers nach Betätigung bewirkt.

Fig. 13 zeigt in schematischer Darstellung weitere Ausführungsform einer erfindungsgemäßen Kapsel. In Fig. 13 a wird die Kapsel als Ganzes mit Befüllung gezeigt. Die Kapsel ist im Wesentlichen zylindrisch mit halbkugelförmigen oberen und unteren Enden. Die geschlossene Kapsel (71) weist in ihrem Inneren eine abgemessene Dosis des Pulvers (40) auf.
In Fig. 13b sind Löcher 42a und 42b im halbkugelförmigen oberen Ende bzw. Deckel und im unteren Ende bzw. Boden der Kapsel platziert worden. Für die Ausbringung des Pulvers insbesondere unter Ausnutzung des Bernoulli-Effekts in einem Inhalator wird zumindest ein Loch wird im Boden und zumindest eines im Deckel der Kapsel benötigt. Es können auch jeweils mehr als ein Loch in Boden und Deckel verwendet werden. Die Lochgrößen haben vorzugsweise Durchmesser zwischen 0,01 und 5 Millimetern, bevorzugt zwischen 0,5 und 1,5 Millimeter. Die Löcher sind vorzugsweise kreisförmig, können aber auch oval, quadratisch oder von sonstiger Gestalt sein. Die Löcher können im Spritzgießprozess abgeformt, mit konventionellen Bohrern oder mit Lasern gebohrt, gestempelt oder auf sonstige Weise gebildet werden, bevor die Kapsel mit Pulver befüllt wird.
In Fig. 13c sind die Löcher (72) in den parallelen Wänden bzw. im zylindrischen Mantelbereich der geschlossenen Kapsel (71) platziert worden. Auf diese Weise kann das Pulver einfacher in der Kapsel mit vorgefertigten Löchern gelagert werden, da die Kapsel passgenau in einer zylindrischen Röhre gelagert werden kann, welche die Kapsel dann im Mantelbereich dicht umschließt.
Fig. 13b und Fig. 13c zeigen lediglich Varianten, in denen die Löcher (42a, 42b bzw. 72) entweder in den halbkugelförmigen oder in den zylindrischen Bereichen der Kapsel (71) ausgebildet sind. Darüber hinaus sind jedoch auch Varianten einer Kapsel (71) mit insgesamt mindestens zwei Löchern (42a, 42b bzw. 72) möglich, bei denen sich das Loch oder die Löcher (42a, 42b bzw. 72) auf einer Seite der Kapsel (71) im halbkugelförmigen Bereich und das Loch oder die Löcher (42a, 42b bzw. 72) auf der anderen Seite der Kapsel (71) im zylindrischen Mantelbereich befindet oder befinden. Die mindestens zwei Löcher (42a, 42b bzw. 72) können des Weiteren bezogen auf den kreisförmigen Umfang der Kapsel (71) auch um 180° oder andere Winkeleinheiten versetzt angeordnet sein. Ebenfalls sind zusätzlich viele Variationen hinsichtlich der Verteilung von unterschiedlich vielen Löchern (42a, 42b bzw. 72) an der Kapsel (71) möglich, beispielsweise kann sich auf einer Seite (unten bezogen auf Fig. 13b) nur ein Loch (42a, 42b bzw. 72) und auf der anderen Seite (oben) mindestens zwei Löcher (42a, 42b bzw. 72) befinden.

Fig. 14 zeigt schematisch die Funktionsweise eines Geräts (70) bzw. Inhalators, in dem eine Kapsel (71) (Fig. 14a) mit zwei vorgefertigten Löchern (72a, 72b) in dem Gerät so bevorratet ist, dass die Löcher (72, 72b) im Lagerzustand (Fig. 14b) verschlossen sind, und die Kapsel (71) für die Anwendung des Geräts aus dem Lagerzustand in eine Kapselkammer (74)(entsprechend der Kapselkammer (13) aus vorhergegangenen Ausführungsformen) verschoben wird.
Fig. 14a zeigt die ansonsten geschlossene Kapsel (71) mit vorgefertigten Löchern (72a, 72b). Die Kapsel (71) ist im Wesentlichen zylindrisch mit halbkugelförmigen Enden. (71a, 71b). Die Löcher (72a, 72b) befinden sich in den parallelen Wänden der Kapsel bzw. im Mantelbereich der Kapsel jeweils nahe der halbkugelförmigen Enden. Analog können in diesem Zusammenhang auch Kapseln gemäß anderer Ausführungsformen eingesetzt werden, insbesondere entsprechend der in Fig. 11c gezeigte Kapsel (11) ohne Ring (22) oder entsprechend der in Fig. 13c gezeigte Kapsel.

Fig. 14b zeigt die Kapsel in ihrem Lagerzustand in einem Gerät (70). In diesem Lagerzustand wird die Kapsel (71) fest in einer Röhre (73) gehalten. Dies verhindert den Austritt von Pulver aus der Kapsel (71), da die innere Wand der Röhre die Löcher (72a, 72b) abdeckt. Innerhalb des Geräts (70) ist eine Kapselkammer (74) ausgebildet. Die Kapselkammer (74) schließt direkt an die Röhre (73) an und hat einen etwas größeren vorzugsweise kreisförmigen Durchmesser als die Röhre (73). Die Kapselkammer (74) wird durch einen Stab (75) oder durch ein anderes vorzugsweise aerodynamisch geformtes Bauteil im Luftausgangsbereich begrenzt.
In Fig. 14c ist das Gerät (70) anwendungsbereit, d.h. es befindet sich im Anwendungszustand. Die Kapsel (71) ist durch den Einlass (76) in die Kapselkammer (74) geschoben worden. Hierzu ist auf der der Kapselkammer (74) entgegengesetzten Seite der Kapsel (71) ein kolbenförmiger Schieber in die Röhre (73) eingeführt. Die Schiebefläche des Schiebers begrenzt im Anwendungszustand die Kapselkammer (74) auf der dem Luftausgang entgegengesetzten Seite. Zur Anwendung atmet der Anwender durch ein Mundstück (78) in Richtung des Pfeils (92) in der Abbildung ein. Luft gelang in das Gerät (70) entlang der Richtung des Pfeils (91) in der Abbildung durch den Einlass (76), bzw. durch eine Luftführung im Schieber, der vorzugsweise die Gestalt eines Hohlkolbens hat. Im Luftstrom vibriert die Kapsel (71) in der Kapselkammer (74), wobei das Pulver über die Löcher (72a, 72b) aus der Kapsel (71) ausgetrieben wird.

In einer anderen nicht dargestellten Ausführungsform lagert die Kapsel schon in der Vibrationskammer bzw. Kapselkammer (74) und die Kapsel (71) ist mit einer vorzugsweise schlauchförmig ausgebildeten Folie umzogen. Die Folie liegt dicht am zylindrischen Mantelbereich der Kapsel (71) an und verschließt die Löcher (72). Vorzugsweise können dabei die Materialien von Folie und Kapselwand so gewählt sein, dass sich die vorzugsweise elastische und/oder leicht biegbare Folie durch elektrostatische Anziehung an die Kapselwand anschmiegt. An einem Endpunkt ist die Kapsel (71) vorzugsweise nicht fest von der Folie umschlossen und an der anderen Seite ragt sie deutlich über die Kapsel (71) hinaus und/oder ist mit einem Zugband verbunden. Dieser am Kapselende hinaus ragende Folienteil und/oder das Zugband liegt im Transportzustand des Inhalators derart im Lufteinlass, dass an dieser Stelle ein Teil der Folie und/oder eines Zugbandes aus dem Inhalator herausragt. Vor der Anwendung des Inhalators wird die Folie an diesem herausragenden Teil und/oder an diesem Zugband durch den Lufteinlass von der Kapsel, die nicht durch den Lufteinlass passt, abgezogen. Dadurch werden die Löcher (72) freigegeben, die Kapsel (71) erhält ihren vollen Bewegungsspielraum in der Kapselkammer (74), und der Inhalator ist anwendungsbereit.
Alternativ kann der überstehende Teil der Folie bzw. das Zugband auch im Mundrohr des Inhalators liegen und durch das Mundrohr aus dem System herausgezogen werden. Generell kann die Öffnung, durch welche die Folie aus dem System gezogen wird im Transportzustand auch verschlossen sein. In der Ausführungsform, in der der überstehende Folienteil bzw. das Zugband im Mundrohr liegt, kann beispielsweise das Mundrohr durch eine am Mundstück (78) angebrachte Kappe verschlossen sein, die erst abgenommen werden muss, bevor man die Folie heraus ziehen kann.

Fig. 15 zeigt schematisch den Aufbau einer weiteren Ausführungsform eines Systems aus Inhalator und Kapsel, wobei die Funktionsweise des Systems ähnlich ist wie bei dem in Fig. 14 gezeigten System. Die Teilbilder 15a bis 15f stellen dabei gleichzeitig auch schematisch den Ablauf einer Montage des Systems dar. Zunächst (Fig. 15a) wird ein Kapselkörper (2) mit mindestens einem vorgefertigten Loch in einen Ring (22) eingesetzt, der den Kapselkörper (2) im Bereich seines mindestens einen vorgefertigten Loches passgenau umschließt und somit das Loch abdichtet. Optional weist der Ring (22) innen einen oder vorzugsweise mehrere kleine Vorsprünge auf, die beim Zusammenfügen von Kapselkörper (2) und Ring (22) eine untere Position des Kapselkörpers (2) im Ring (22) vorgegeben, so dass der Kapselkörper (2) sich innerhalb des Rings nicht nach unten verschieben kann und/oder zwischen Kapselkörper (2) und Ring (22) eine Passung ausgebildet wird, durch die der Kapselkörper (2) im Ring (22) zurückgehalten wird. Den Ring (22) betreffende Merkmale aus diesem Ausführungsbeispiel sind analog auch auf den Ring (22) aus dem Ausführungsbeispiel gemäß Fig. 11 und Fig. 12 übertragbar. Fig. 15b zeigt wie der Kapselkörper (2) im Ring (22) von unten in eine Röhre (73) eingesetzt wird. Die Röhre (73) bildet in diesem Ausführungsbeispiel die Kapselaufnahme. Bevorzugt verrastet der Ring (22) dabei von innen mit der Röhre (73) in einer ersten Raststellung, z.B. indem eine umlaufenden Wulst (22a) oder ein anderweitig geformtes Rastelement von innen in der Röhre (73) in eine entsprechende Aussparung eingreift. Die Röhre (73) ist an ihrem oberen Ende geöffnet, so dass der Kapselkörper (2), dessen Öffnung bei Einsetzen in die Röhre (73) nach oben zeigt, von oben durch die obere Öffnung der Röhre (73) mit Pulver (40) befüllt werden kann. Fig. 15c zeigt dann, wie - nach der nicht dargestellten Befüllung mit Pulver (40) - die Kapselkappe (1), die vorzugsweise ebenfalls mindestens ein vorgefertigtes Loch aufweist, von oben in die Röhre (73) eingesetzt wird und so im Inneren der Röhre (73) den Kapselkörper (2) verschließt, bzw. die Kapsel (71) in der Röhre (73) zusammengesetzt wird. Die Röhre (73) umschließt dabei die Kapselkappe (1) vorzugsweise mit einem oberen Kragen (73a) passgenau, so dass das mindestens eine Loch (72b) in der Kapselkappe (1) durch die Röhre (73) verdeckt bzw. abgedichtet ist.
Fig. 15d und Fig. 15e zeigen, wie die weiteren Bauteile des Inhalators - Mundstück (78) und Schieber (77) - mit der Baugruppe bestehend aus Röhre (73), pulvergefüllter Kapsel (71) und Ring (22) zusammengesetzt werden: Das Mundstück (78), das vorzugsweise einstückig ausgebildet ist, enthält die Kapselkammer (74) und einen Stab (75) als Begrenzung oben im Innern der Kapselkammer (74). Das Mundstück wird von oben auf die Röhre (73) aufgesetzt, wobei es mit der Röhre (73) verrastet. Hierzu sind miteinander wechselwirkende Rastelemente an Mundstück (78) und Röhre (73) ausgebildet, z.B. in Form einer unten im Inneren des Mundstücks angebrachten, ringförmig umlaufenden Wulst (78b), die in eine ebenfalls ringförmig umlaufende Sicke (73b) außen, oben an der Röhre (73) einrastet. Auf der dem Mundstück entgegengesetzten Seite, d.h. in der Darstellung von unten, wird ein Schieber (77) in die Röhre (73) eingesetzt. Der Schieber (77) beinhaltet einen Einlass (76) durch den später bei Anwendung des Inhalators Luft in die Kapselkammer (74) einströmen kann. Vorzugsweise ist der Schieber (77) dabei als Hohlkolben ausgebildet und/oder der Einlass (76) wird durch eine radialsymmetrische Durchführung entlang der Hauptachse des Schiebers (77) gebildet. Der Schieber (77) weist an seinem oberen Ende eine Verjüngung (77a) auf, die derart bemessen ist, dass sie in den Ring (22) eintauchen kann.
Fig. 15f zeigt im schematischem Längsschnitt das fertig zusammen gesetzte System aus Kapsel (71) und Inhalator im Transportzustand: Die Kapsel (71) ist derart im Inhalator vorinstalliert, dass ihre vorgefertigten Löcher (72a, 72b) vom Ring (22) und/oder vom Inneren der Röhre (73) verdeckt bzw. abgedichtet werden (im dargestellten Ausführungsbeispiel mit insgesamt zwei vorgefertigten Löchern (72a, 72b) wird ein Loch (72a) vom Ring (22) und ein Loch (72b) vom Inneren der Röhre abgedichtet). Der Schieber (77) ragt mit seinem unteren Ende aus dem Inhalator heraus. Zur Aktivierung des Inhalators, d.h. zur Überführung des Geräts aus dem Transportzustand in den Anwendungszustand wird der Schieber an seinem unten am Gerät vorstehenden Ende in das Gerät hinein gedrückt. Vorzugsweise ist hierzu das untere Ende des Schiebers (77) derart verbreitert, dass es beim Drücken mit der Hand angenehm am Handballen des Anwenders bzw. Patienten anliegt. Beim Hineinschieben stößt der Schieber (77) vorzugsweise mit einer ringförmig gestalteten Kontaktfläche von unten gegen die Kapsel (71) und schiebt diese - bedingt durch ihre Rückhaltung im Ring (22) - zunächst zusammen mit dem Ring (22) weiter in Richtung Kapselkammer (74), bis der Ring (22) in einer zweiten Raststellung verrastet. Beim weiteren Hineinschieben des Schiebers (77) in die Röhre (73) taucht der Schieber (77) in den Ring (22) ein und drückt mit der Verjüngung (77a) die Kapsel (71) aus ihrer passgenauen Halterung im Ring (22) in die Kapselkammer (74). Die Verjüngung (77a) ist dabei vorzugsweise so lang ausgelegt, dass der verjüngte Bereich des Schiebers (77) durch den Ring (22) und durch den Kragen (73a) der Röhre (73) hindurch geschoben werden kann, bis die Oberkante des Schiebers (77) die untere Begrenzung der Kapselkammer (74) bildet. Vorzugsweise schließt die Oberkante des Schiebers (77) im eingeschobenen Zustand (Anwendungszustand des Inhalators, siehe Fig. 15g) bündig mit der Oberkante der Röhre (73) ab. Der Spalt, der sich je nach Auslegung des Systems - z.B. bedingt durch einen im Vergleich zu Kapselkörper (2) und Verjüngung (77a) größeren Durchmesser der Kapselkappe (1) - im Boden der Kapselkammer (74) zwischen dem oberen Rand des Schiebers (77) und dem Kragen (73a) oben an der Röhre (73) bildet, wird hierbei vorzugsweise durch den Ring (22) gegen das Eindringen von Nebenluft abgedichtet. Der Ring (22) besteht hierfür aus einem zumindest teilweise elastischen Material und dichtet in diesem eingeschobenen Zustand (siehe Fig. 15g) des Schiebers (77) den Schieber (77) gegen die Röhre (73) unterhalb des Kragens (73a) ab.
Der Inhalator ist dann, wie in Fig. 15g gezeigt, funktionsbereit: die Löcher (72a, 72b) sind freigelegt und die Kapsel (71) hat in der Kapselkammer (74) den Bewegungsspielraum, der für die Vibrationsbewegung gemäß dem Bernoulli-Effekt benötigt wird. Vorzugsweise sind seitlich am Schieber (77) mindestens ein, vorzugsweise zwei Federarme (77b) ausgebildet, die im eingeschobenen Zustand in entsprechende Ausnehmungen (73s) innen an der Röhre (73) eingreifen. Diese Federarme (77b) verhindern durch ihren Eingriff innen in der Röhre ein Herausziehen des Schiebers (77) aus dem Gerät heraus. Dem Anwender wird somit verdeutlicht, dass es sich hierbei um ein Gerät für eine einmalige Anwendung handelt.

Fig. 16 zeigt schematisch den Aufbau einer weiteren Ausführungsform eines Systems bestehend aus Inhalator und einer pulvergefüllten Kapsel (71), wobei insbesondere eine Kapsel gemäß der in Fig. 11 oder Fig. 14 gezeigten Ausführungsformen eingesetzt werden kann. Die Teilbilder 16a bis 16e stellen dabei gleichzeitig schematisch den Ablauf einer Montage des Systems dar.

Zunächst (Fig. 16a) wird ein Kapselkörper (2) mit mindestens einem vorgefertigten Loch von oben in eine in diesem Ausführungsbeispiel im Wesentlichen nur einseitig offene Röhre (73) eingesetzt, welche die Kapselaufnahme bildet. Die Röhre (73) umschließt den Kapselkörper (2) im Bereich seines mindestens einen vorgefertigten Loches passgenau und dichtet somit das Loch im Kapselkörper (2) ab. Die einseitige Öffnung des Kapselkörpers (2) zeigt dabei nach oben, d.h. in jene Richtung, aus der der Kapselkörper (2) in die Röhre (73) eingesetzt wurde.
Je nach Gestaltung der Kapselaufnahme ist es vorteilhaft, die Kapsel (71) orientiert in diese einzusetzen. In der hier dargestellten Ausführungsform weist die Kapselaufnahme beispielsweise zwei längs verlaufende Schlitze (73d) auf, deren Funktion anhand der Beschreibung von Fig. 16f näher erläutert wird. Um eine Abdichtung der Löcher (72a, 72b) durch das Einsetzen der Kapsel (71) in die Kapselaufnahme zu gewährleisten, wird die Kapsel (71) bevorzugt derart orientiert in die Röhre (73) eingesetzt, dass sich die Löcher (72a, 72b) nicht im Bereich der Schlitze (73d) befinden. Um eine Orientierung der Kapsel (71) beim Einsetzen in die Kapselaufnahme vorzugeben, können die anhand von Fig. 4 und Fig. 5 beschriebenen Techniken angewandt werden. So können Kapsel (71) und das Innere der Kapselaufnahme beispielsweise leicht elliptisch hinsichtlich ihres kurzen Durchmessers ausgebildet sein. Alternativ können Kapsel (71) oder das Innere der Kapselaufnahme entsprechende Paarungen von längsverlaufenden Stege und Rillen aufweisen: beispielsweise eine Längsnut oder längsverlaufende Rille an der äußeren Wand der Kapsel (71) in Kombination mit einem längs ausgebildeten Steg im Inneren der Kapselaufnahme.
Fig. 16b zeigt die Befüllung des Kapselkörpers (2) innerhalb der Röhre (73) mit Pulver (40), das von oben in die einseitige Öffnung des Kapselkörpers (2) eingefüllt wird. Anschließend (Fig. 16c) wird aus gleicher Richtung, d.h. in der Darstellung von oben, die Kapselkappe (1) in die Röhre (73) eingeführt, so dass die Kapsel (71) innerhalb der Röhre (73) verschlossen wird. Fig. 16d zeigt, wie die weiteren Bauteile des Inhalators - Mundstück (78) und Stab (75) - mit der Baugruppe bestehend aus Röhre (73) und pulvergefüllter Kapsel (71) zusammengesetzt werden: Die Röhre (73) wird (nun in Fig. 16d im Vergleich zu Fig. 16c kopfüber dargestellt) von oben durch eine Öffnung am Mundstück (78) vorzugsweise bis zu einem von der Kapselkammer (74) gebildeten unteren Anschlag eingeschoben. Die Seite, von der die Röhre (73) eingeführt wird, ist hierbei auch die Mundseite des Mundstücks, d.h. die Seite an der der Patient zum Gebrauch des Inhalators die Lippen ansetzt. Nach Einsetzen der Röhre (73) in das Mundstück (78) wird ein Stab (75) durch eine seitlich am Mundstück befindliche Durchführung eingeschoben. Der Stab (75) bildet später die obere Begrenzung der im Mundstück (78) befindlichen Kapselkammer (74). Die Röhre (73) ist vor dem Einsetzen des Stabs (75) derart orientiert in das Mundstück eingesetzt worden, dass zwei an der Röhre vorgesehene Schlitze (73d) an der Durchführung im Inneren am Mundstück (78) anliegen und somit Platz für den Einschub des Stabs (73) lassen. Auf diese Weise kann der Stab (75) so in das Mundstück (78) eingeführt werden, dass er ohne Behinderung durch die Röhre (73) das Mundstück (78) vorzugsweise quer durch die Hauptachse des Geräts hindurch das Mundstück (78) von einer Außenwand zur anderen durchdringt. Das orientierte Einsetzen der Röhre (73) in das Mundstück (78) wird vorzugsweise durch die äußere Form der beiden Bauteile vorgegeben. So zeigt das Darstellungsbeispiel beispielsweise ein Mundstück (78) mit einem im Wesentlichen ovalen oder trapezförmigen Querschnitt aus der Mundseite des Mundstücks (78). Die Röhre (73) ist in ihrem oberen - von der Kapselaufnahme entfernten - Bereich vorzugsweise in Form einer Kappe ausgebildet, die das Mundstück an seinem mundseitigen Ende komplett abdeckt. Diese Kappenstruktur der Röhre (73) bildet das Gegenstück zu der im Wesentlichen ovalen oder trapezförmigen Gestalt der Mundseite des Mundstücks (78), so dass eine Orientierung beim Zusammenführen durch die Ovalität bzw. die Vorzugsachse einer nicht kreisförmigen Symmetrie vorgegeben ist. Die in Fig. 16 gezeigte Kappenstruktur der Röhre (73) bietet auch zusätzlich den Vorteil, dass an der Mundseite die Außenflächen des Mundstücks (78) in den Bereichen, in denen der Patient seine Lippen ansetzt, im Transportzustand von der Röhre (73) verdeckt werden. Somit wird selbst bei sehr frühzeitiger Entnahme des Geräts aus einer Umverpackung gewährleistet, dass die Bereiche für den Lippenkontakt bis zur Anwendung des Geräts frei von Verschmutzungen bleiben.
Der Stab (75) bildet die obere Begrenzung der Kapselkammer (74) im Innern des Mundstücks (78). Außer dem Stab (75) werden alle anderen Bestandteile der Kapselkammer (74) einstückig vom Mundstück (78) gebildet. Am unteren Ende der Kapselkammer (74), d.h. an ihrem dem Stab (75) und der Mundstücköffnung gegenüber liegenden Ende, weist das Mundstück einen Einlass (76) auf, der im Darstellungsbeispiel als mittige Durchführung entlang der Hauptachse des Systems ausgebildet ist.
Der hier dargestellte Inhalator besteht vorzugsweise nur aus drei Teilen - Mundstück (78), Röhre (73) und Stab (75), die alle kostengünstig in Kunststoff-Spritzguss-Verfahren hergestellt werden können, so dass ein derart aufgebauter Inhalator sich sehr gut zur Einmal-Anwendung eignet, d.h. als Wegwerf-Artikel nach einmaliger Anwendung vorgesehen werden kann.
Fig. 16e zeigt das System aus Inhalator und Kapsel (71) im zusammengesetzten Zustand, der hier auch dem Transportzustand entspricht. Die (nicht gezeigten) vorgefertigten Löcher (72a, 72b) der Kapsel (71) werden durch die Innenwand der Röhre (73) verschlossen. Für Kapseln (71), von denen ein Kapselelement z.B. die Kapselkappe (1) einen größeren Außendurchmesser als der zugängliche Teil des anderen Kapselelementes z.B. des Kapselkörpers (2) hat, weist die Röhre (73) eine entsprechende Veränderung des Innendurchmessers auf: das Kapselelement mit geringerem Durchmesser befindet sich weiter im Innern der Röhre (73) als das Kapselelement mit größerem Durchmesser, und der innere Durchmesser der Röhre (73) ist entsprechend an die äußere Gestalt der Kapsel (71) angepasst, so dass auch er von innen nach außen schrittweise breiter wird. Im Transportzustand ist die Kapsel (71) somit ohne nennenswerten Bewegungsspielraum im System eingeschlossen. Für einen festen Sitz der als Kappe gestalteten Röhre (73) auf dem Mundstück (78) weist die Röhre in ihrem oberen Bereich vorzugsweise Federarme (73f) auf, die sich im Transportzustand von innen gegen die Innenwand des Mundrohrs (78d) drücken, das auf der Mundseite des Mundstücks (78) den Luftauslass aus der Kapselkammer (74) bildet. Vorzugsweise haben Mundrohr (78d) und Kapselkammer (74) den gleichen Durchmesser.
Zur Anwendung des Inhalators wird dann (wie in Fig. 16f) gezeigt die Röhre (73) aus dem Mundstück (78) herausgezogen. Die Schlitze (73d) in der Röhre (73) ermöglichen hierbei das Herausziehen der Röhre (73) am Stab (75) vorbei. Der in das Mundstück (78) eingesetzte und sich quer durch die Mundstücköffnung erstreckende Stab (75) bewirkt, dass die Kapsel (71) in der Kapselkammer (74) im Mundstück (78) verbleibt und nicht mit der Röhre (73) wieder herausgezogen werden kann. Wird die Röhre (73) aus dem Mundstück (78) herausgezogen, so spreitzen sich die zuvor im Mundrohr (78d) zusammengedrückten Federarme (73f) vorzugsweise so nach außen auf, dass die Röhre (73) nicht wieder (ohne Hilfsmittel) zerstörungsfrei in das Mundstück (78) eingesetzt werden kann. Auf diese Weise wird dem Anwender verdeutlicht, dass es sich bei diesem Gerät um einen Wegwerf-Artikel bzw. ein Ein-Weg-Produkt handelt. Um dem Anwender das Herausziehen der Röhre (73) aus dem Mundstück (78) zu erleichtern, weist die Röhre (73) vorzugsweise eine Griffhilfe (73e) auf. Diese Griffhilfe kann beispielsweise wie in Fig. 16d gezeigt durch eine Riffelung der äußeren seitlichen Oberfläche im Kappenbereich der Röhre (73) oder (nicht gezeigt) durch eine Lasche gebildet werden. Diese Lasche befindet sich vorzugsweise mittig oben im Kappenbereich der Röhre (73), also an der Seite, die der Einführöffnung für die Kapsel (71) entgegengesetzt ist, und beinhaltet eine Öffnung, die derart bemessen ist, dass der Anwender einen Finger (vorzugsweise seinen Zeigefinger) in die Öffnung einhaken kann, um anschließend die Röhre (73) aus dem Mundstück (78) zu ziehen. Damit gleichzeitig das Mundstück bequem mit Fingern der anderen Hand gehalten werden kann, ist es vorzugsweise mit einer Grifffläche (78c) ausgestattet, die in dem Moment des Rausziehens der Röhre (73) ein Wegrutschen des Mundstücks (78) in der Hand bzw. zwischen den Fingern verhindert. Diese Grifffläche (78c) wird vorzugsweise durch eine Mehrzahl von abgerundeten Erhebungen oder Noppen gebildet, die sich insbesondere mittig außen auf zwei Seiten am Mundstück (78) unterhalb des vorzugsweise verbreiterten Bereichs zum Ansetzen der Lippen befindet.

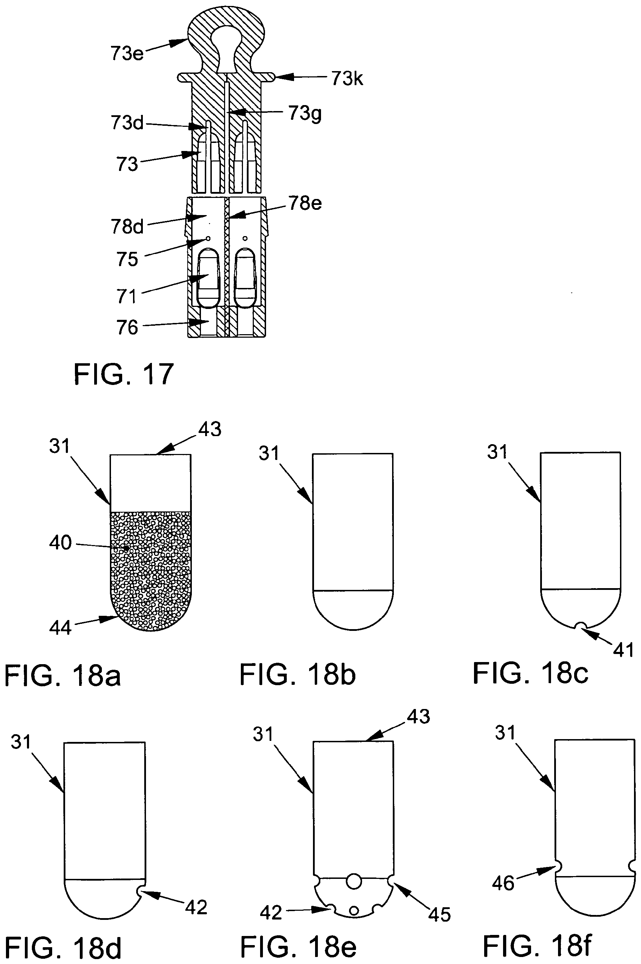

Fig. 17 zeigt ein System aus einem Inhalator und zwei Kapsel (71). Die Funktionen in diesem Ausführungsbeispiel entsprechen denen des Ausführungsbeispiels aus Fig. 16 mit dem Unterschied, dass zwei Kapseln (71) in individuellen Kapselkammern (74) vorgesehen sind. Alle mit den Kapseln (71) wechselwirkende Merkmale sind somit doppelt ausgelegt: Der Inhalator weist zwei Kapselkammer (74) mit zwei Einlässen (76), zwei Stäben (75) und zwei Röhren (73) mit Schlitzen (73d) auf. Im Transportzustand umschließen die Wände der Röhren (73) die Kapseln (71) derart, dass die vorgefertigten Löcher (72) in den Kapseln verschlossen sind. Die beiden Röhren (73) sind in der abgebildeten Ausführungsform vorzugsweise in einem Abschlussbereich (73k) so verbunden, dass sie Teil einer Kappe sind, die analog zum Ausführungsbeispiel aus Fig. 16 die Öffnung des Mundstücks (78) im Transportzustand verschließt. An dieser Kappe ist eine Griffhilfe (73e) ausgebildet, die in Fig. 17 in der Ausführungsform als Lasche mit Öffnung dargestellt ist. Vor der Inhalation zieht der Anwender die Kappe an der Lasche vom Mundstück (78) weg, erhält so den in Fig. 17 dargestellten Zustand. Die Löcher (72) an den Kapseln (71) sind dann nach der Entnahme der Röhren (73) aus den Kapselkammern (74) geöffnet und die Kapseln (71) haben den für die gewünschte Vibration benötigten Bewegungsspielraum. Die Strömungswege durch die beiden Kapselkammern sind vorzugsweise nicht miteinander verbunden. Der Inhalator weist also zwei Mundrohre (78d) auf, die durch eine Mittelwand (78e) von einander getrennt sind. Beide Mundrohre (78d) verlaufen parallel und öffnen sich nebeneinander in einem vorzugsweise außen oval gestalteten Mundstück (78). Die beiden Röhren (73) sind im Transportzustand des Geräts passgenau in die beiden Mundrohre (78d) eingesteckt, wobei ein Abstand (73g) zwischen den Röhren vorgesehen ist, um Raum für die Mittelwand (78e) zu geben.
Die beiden Kapseln (71) können je nach Anwendungszweck des Inhalators identisch oder hinsichtlich ihrer Befüllung und/oder äußeren Gestalt verschieden sein. Die Verwendung eines Geräts mit zwei identischen Kapseln (71) hat den Vorteil, dass man mit einem einzigen Wegwerf-Gerät die doppelte Formulierungsdosis ausgeben kann und somit Wegwerfmaterialkosten spart. Die Verwendung eines Geräts, in dem zwei verschieden befüllte Kapseln (71) bevorratet sind, eignet sich vor allem für den Einsatz in Therapien, bei denen zwei Wirkstoffe gleichzeitig verabreicht werden, die unter Umständen nicht stabil in einer gemeinsamen Formulierung gelagert werden können. Mit einem solchen Gerät wird sichergestellt, dass beide Wirkstoffe in richtigem Mengenverhältnis zueinander eingenommen werden. Es wird somit beispielsweise ausgeschlossen, dass ein Anwender zweimal zum gleichen Präparat statt zu zwei verschiedenen Präparaten bei einem Einnahmezyklus greift.
Die Kapseln (71) und ihre zugehörigen Kapselkammern (74), Mundrohre (78d) und Röhren (73) können für diesen Zweck an Wirkstoff bzw. Formulierungsdosis angepasst sein, beispielsweise unterschiedlich groß sein bzw. unterschiedliche Durchmesser und/oder Längen haben.

Fig. 18 zeigt in schematischer Darstellung weitere Ausführungsform einer erfindungsgemäßen Kapsel. In Fig. 18 a wird eine Kapsel (31) mit einem offenem Ende (43) (oberes Ende) und mit einem geschlossenen, unteren Ende (44) gezeigt, die Pulver (40) im Inneren hat. Fig. 18b zeigt die äußere Gestalt dieser Kapsel (31) ohne vorgefertigte Löcher. Die Kapsel ist im Wesentlichen zylindrisch mit halbkugelförmigem unterem Ende (44). Das obere Ende ist vorzugsweise offen, es ist aber insbesondere bei vorab befüllten Kapseln möglich, diese für einen Lagerzustand durch eine Folie oder ähnliches zu verschließen. Im Vergleich mit zuvor beschriebenen Ausführungsformen besteht die Kapsel (31) sozusagen nur aus einem Kapselelement, dem Kapselkörper.
Es hat sich gezeigt, dass sich, wenn ein Loch (41) in der Kapsel (31) wie in Fig. 18c gezeigt mittig in der Kuppel des unteren Endes (44) ausgebildet ist, die ausgebrachte Pulverdosis erhöht, aber der inhalierbare Anteil des ausgebrachten Pulvers reduziert wird. Um die ausgebrachte Dosis und in den in die Lungen des Anwenders eingebrachten inhalierbaren Anteil zu erhöhen, kann ein Loch (42) wie in Fig. 18d im seitlichen Bereich des halbkugelförmigen unteren Endes (44) angebracht werden.

Vorzugsweise werden hierbei mehrere Löcher (42), teilweise auch unterschiedlicher Größe angebracht. Als besonders vorteilhaft hat sich eine Kapsel (31) mit einer Lochstruktur gemäß Fig. 18e herausgestellt. Bei dieser Kapsel (31) sind zwei Gruppen von Löchern (42, 45) auf dem unteren halbkugelförmigen Ende (44) der Kapsel (31) verteilt: eine erste Gruppe von Löchern (42) befindet sich ringförmig angeordnet um den Scheitelpunkt des unteren halbkugelförmigen Endes (44) und eine zweite Gruppe von Löchern (45) befindet sich ringförmig weiter entfernt vom Scheitelpunkt angeordnet, vorzugsweise in der Nähe von oder auf der Übergangslinie von dem Kapselbereich mit halbkugelförmiger Wand und dem mit zylinderförmiger, d.h. paralleler Wand. Vorzugsweise sind die Löcher (42, 45) in ihren Gruppen auf den von ihnen gebildeten Ringen äquidistant angeordnet. Vorzugsweise weist jede Gruppe von Löchern (42, 45) jeweils 1 bis 8 einzelne Löcher (42, 45) mit Lochgrößen mit Durchmessern im Bereich von 0,01 bis 5 Millimetern, besonders bevorzugt im Bereich von 0,1 Millimeter bis 1,5 Millimeter auf. Besonders bevorzugt sind die Löcher (45) der zweiten Gruppe dabei größer als die Löcher (42) der ersten Gruppe. Ganz besonders bevorzugt wird eine Kapsel (31) mit 4 Löchern (42) eines Durchmessers von 0,4 ± 0,1 Millimeter für die erste Gruppe und 4 Löchern (45) eines Durchmessers von 0,9 ± 0,1 Millimeter für die zweite Gruppe. Mit einer solchen oben offenen Kapsel (31) mit 8 Löchern (42, 45) gemäß Fig. 18e konnte bei Laboruntersuchungen der Zerstäubung von Pulver aus Kapseln in Vibrationskammern ein deutlich höherer inhalierbarer Anteil als bei konventionellen geschlossenen Kapseln mit insgesamt nur zwei Löchern erzielt werden. Zusätzlich zeigten diese Untersuchungen den Effekt, dass der inhalierbare Anteil des Pulvers aus der Kapsel (31) mit den 8 Löchern weniger stark von der Flussrate des angelegten Luftstroms abhing als bei der konventionellen Kapsel. [Zum Vergleich wirddie Verwendung einer offenen, mehrfach gelochten Kapsel in einem entsprechenden Inhalator betrachtet.] Bei der Verwendung der auf diese Weise mehrfach gelochten, oben offenen Kapsel (31) ist also die Größe des vom Anwender (bzw. Patient im hier betrachteten Fall einer medizinischen Anwendung) eingeatmeten Anteils des Pulvers aus der Kapsel (31) vergleichsweise unabhängiger vom Einatmungsverhalten als bei Verwendung einer konventionellen Kapsel.

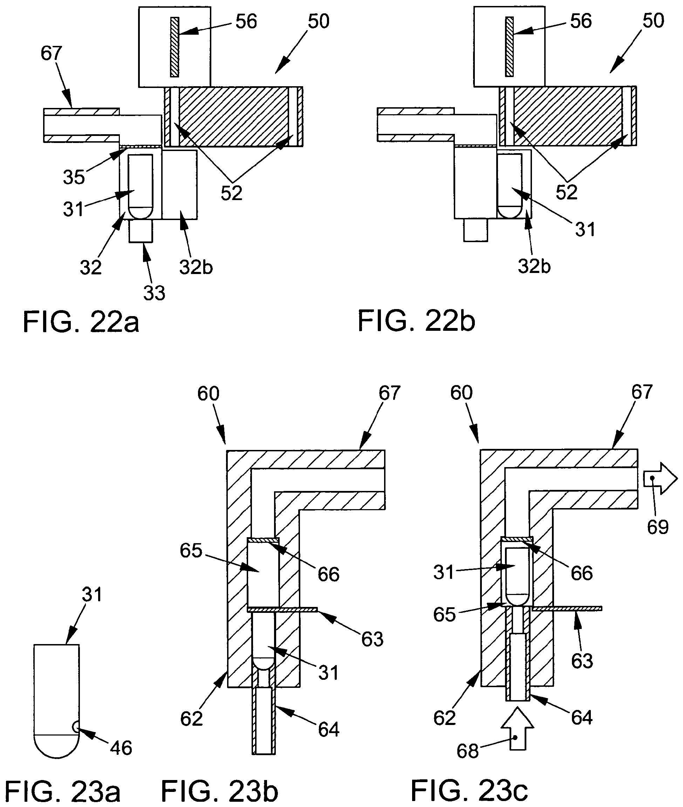

Fig. 18f zeigt eine andere bevorzugte Ausführungsform der Kapsel (31), in der das Loch (46) im unteren zylindrischen Mantelbereich nahe dem halbkugelförmigen Bereich des unteren Endes (44) der Kapsel (31) angebracht. In dieser Abbildung ist das Loch (46) also in den parallelen Wänden der offenen Kapsel (31) ausgebildet. Auf diese Weise kann das Pulver (40) einfacher in der Kapsel (31) mit vorgefertigten Löchern (46) gelagert werden, da die Kapsel passgenau in einer zylindrischen Röhre gelagert werden kann, welche die Kapsel (31) dann im Mantelbereich dicht umschließt (mehr hierzu bei der Diskussion von Fig. 23). Das vorgefertigte Loch kann somit nach Befüllung der Kapsel (31)verschlossen werden, Die Ausbildung von mehr als einem Loch (46) ist wie in der Fig. 18f gezeigt ebenfalls z.B. in Form von zwei gegenüber liegenden Löchern (46) möglich.
Die Löcher (41, 42, 46) können vorab im Spritzgussverfahren mit abgeformt werden, mit konventionellen Bohrern oder mit Lasern gebohrt, gestempelt oder in anderer geeigneter Weise geformt werden, bevor die Kapsel (31) mit Pulver (40) befüllt wird,

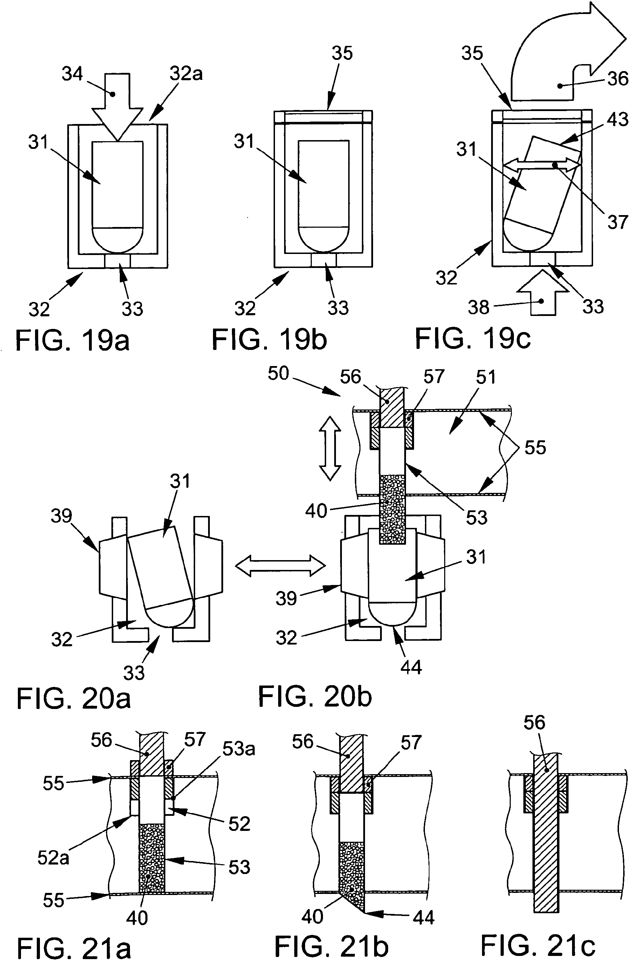

Fig. 19 zeigt schematisch die Funktionsweise eines Geräts bzw. Inhalators, in dem eine offene Kapsel wie beispielsweise gemäß einer der Ausführungsformen aus Fig. 18 innerhalb des Geräts befüllt wird und beispielsweise durch Einatmung des Patienten ein Luftstrom erzeugt wird, der die Kapsel in Bewegung bzw. Vibration versetzt, wodurch das Pulver aus der Kapsel ausgebracht wird.
In Figur 16a wird die Kapsel (31) innerhalb einer Kammer (32)(entsprechend der Kapselkammer (13) oder der Kapselkammer (74) bei vorangegangenen Ausführungsformen) gehalten, wobei die Kammer ein Einlassloch (33) (entsprechend der Lufteinlassöffnung (20) bei vorangegangenen Ausführungsformen) in ihrem Boden aufweist und an ihrer Oberseite (32a) offen ist. Die Kapsel (31) wird innerhalb des Geräts (nicht gezeigt) mit Pulver (40) in Richtung des Pfeils (34) befüllt. Das Pulver (40) kann in einem Pulver-Reservoir gelagert werden und mittels einer Abmesskammer in die Kapsel (31) hinein abgemessen werden, oder es kann in Form von vorab abgemessenen Dosen in einem entsprechenden Magazin für einzelne Dosiseinheiten gelagert werden und bei Bedarf in die offene Kapsel (31) transferiert werden.
In Fig. 19b wird ein Sieb (35) oder ein Gitter oder ein anderes vorzugsweise aerodynamisch geformter Bauteil auf das obere Ende der Kammer (32) gesetzt. Dieses Sieb (35) oder andere Bauteil ist dazu da die befüllte Kapsel (31) in Position zu halten. Gleichzeitig bestimmt es nach oben hin den Bewegungsspielraum der Kapsel (31) in der Kammer (32).
Fig. 19c zeigt die Kapsel bei Verwendung des Geräts. In Fig. 19c tritt Luft in Pfeilrichtung (38) durch das Loch (33) ein und durch das Sieb (35) aus und fließt in Richtung des Pfeils (36) in den Mund des Patienten. Wenn die Luft an der Kapsel (31) entlang strömt, vibriert diese wie vom Pfeil (37) gezeigt und gibt Pulver aus ihrem Innern über das offene Ende (43) und, im Fall von Ausführungsformen mit vorgefertigten Löchern (41, 42, 46), auch über die Löcher (41, 42, 46) ab.

Fig. 20 zeigt schematisch, wie die Befüllung der oben offenen Kapsel (31) in einem Inhalator gemäß der anhand von Fig. 19 erläuterten Funktionsweise ausgeführt werden kann. Betrachtet wird hierbei die Ausführungsform, dass die Kapsel (31) aus einem Magazin (50) befüllt wird, in dem einzelne Einheiten des Pulvers (40), bzw. einzelne Pulverdosen vorab abgemessen einzelnd bevorratet sind. (Eine Ausführungsform, in der die oben offene Kapsel (31) über eine Abmessvorrichtung direkt aus einem Pulver-Reservoir befüllt wird, wird hier nicht weiter erläutert. Hierbei können beliebige im Stand der Technik vorhandene Dosierverfahren von Pulver aus Mehrdosis-Pulver-Reservoiren angewandt werden.).
Fig. 20a zeigt die oben offene Kapsel (31) in der Kammer (32) in einem Zustand, in dem sie den für eine Vibration notwendigen Bewegungsspielraum hat. In dieser Ausführungsform sind in die Wand der Kammer (32) zwei verschiebbare Backen (39) eingelassen, die in diesem Zustand einen Teil der Innenwand der Kammer (32) bilden. Ohne Luftströmung in der Kammer (32) liegt die Kapsel (31) leicht gekippt in der Kammer (32). In Fig. 20b wird die Kammer (32) bei der Befüllung der Kapsel (31) gezeigt. Hierzu sind die Backen (39) soweit ins Innere der Kammer (32) geschoben worden, dass sie von zwei Seiten die Kapsel (31) in einer aufrechten Lage halten (Die Richtung der horizontalen Pfeile steht für die Bewegung der Backen (39)). Hierbei konnte sich die Kapsel (31) mit ihrem unteren halbkugelförmigen Ende (44) am Einlassloss (33) mittig im Boden der Kammer (32) ausrichten, so dass ihre obere Öffnung gerade nach oben zeigt. Andere Ausrichtungsmittel wie z.B. eine Backe (39), welche die Kapsel (31) gegen eine Wand der Kammer (32) drückt, sind alternativ möglich. Fig. 20b zeigt wie die Kapsel (31) von oben mit Pulver (40) aus einem - nur ausschnittsweise in der Abbildung dargestellten - Magazin (50) befüllt wird. Das Magazin (50) ist so gestaltet, dass es mehrere Aufnahmen (52) für einzeln abgemessene Pulverdosen aufweist. Vorzugsweise hat das Magazin (50) die Gestalt eines insbesondere ringförmigen Trägers (51) mit darauf äquidistant angeordneten Aufnahmen (52), die als Durchführungen parallel zur Achse des ringförmigen Trägers (51) ausgebildet sind. In die Aufnahmen sind vorzugsweise Einsätze (53) eingelassen, in denen sich jeweils das Pulver in Form von abgemessenen Dosiseinheiten befindet. Die einzelnen Dosiseinheiten sind im Lagerzustand versiegelt, vorzugsweise durch an beiden flachen Seiten des Trägers (51) angebrachte insbesondere folienartige Siegel (55), welche die oberen und unteren Öffnungen der Aufnahmen (52) verschließen. Zum Transfer einer Einheit des Pulvers (40) aus dem Magazin (50) in die oben offene Kapsel (31) wird hier eine Technik ähnlich der Dosierung mit Hilfe eines Stechhebers angewendet: In dieser Ausführungsform wird ein Stempel (56) mit einem zugehörigen Stempelring (57) auf die jeweilige Aufnahme (52), aus der das Pulver in die Kapsel (31) übertragen werden soll, aufgesetzt und in Richtung Kapsel (32) gedrückt. Details zu diesem Vorgang sind in der Fig. 21 a bis c abgebildet. Fig. 21a zeigt den auf die Aufnahme (52) aufgesetzten Stempel (56) mit Stempelring (57) vor Beginn der Bewegung zum Ausbringen des Pulvers aus der Aufnahme (52). Der Einsatz (53), in dem sich das Pulver (40) befindet, hat innen vorzugsweise die Gestalt einer zylinderförmigen Röhre. Außen weist diese Röhre im oberen Bereich einen Vorsprung (53a) auf, im vergleichsweise längeren unteren Bereich hat der Einsatz (53) eine glatte zylindrische Außenwand mit einem gegenüber dem Vorsprung (53a) verkleinerten Durchmesser.
Im ersten Teil dieser Bewegung (dargestellt in Fig. 21b) bewegen sich Stempel (56) und Stempelring (57) durch Druck von oben gemeinsam. Dabei drückt der Stempelring (57) den Einsatz (53) an dem Vorsprung (53a) in die Aufnahme (52) hinein bzw. innerhalb der Aufnahme (52) nach unten, bis die Unterkante des Vorsprungs (53a) auf einem Anschlag (52a) in der Aufnahme (52) auftrifft. Unterhalb dieses Vorsprungs (52a) umschließt die Aufnahme (52) den längeren unteren Bereich des Einsatzes (53) vorzugsweise in einer gleitenden Passung. Der Stempelring (57), der Vorsprung (53a) und somit auch der gesamte Einsatz (53) sind nun am Ende ihrer nach unten gerichteten Bewegung angekommen. Der Einsatz (53) weist vorzugsweise an seinem unteren Ende eine Spitze auf, insbesondere hat er die Gestalt einer Hohlnadel oder Kanüle mit angeschrägter Spitze, mit der er in der Bewegung nach unten die untere Folie (55) durchsticht. Die Folie wird dabei so weit durchstochen oder so zur Seite geklappt, dass sich kein Folienmaterial mehr vor der unteren Öffnung des Einsatzes (53) befindet.
Im weiteren Verlauf bewegt sich nun der Stempel (56) unabhängig vom Stempelring (57) weiter und gleitet innerhalb des Einsatzes (53) nach unten und stößt das Pulver (40) wie in Fig. 21c gezeigt) komplett aus dem Einsatz (53) heraus und somit in die darunter befindliche oben offene Kapsel (31). Das Innere des Einsatzes (53) und das Äußere des Stempels (56) sind so bemessenen, das eine gleitende Passung vorliegt.

Fig. 22 zeigt schematisch eine Ausführungsform eines Inhalators, bei dem eine nach oben offene Kapsel (31) zur Befüllung mit Pulver (40) aus der Kammer (32) heraus in eine Füllkammer (32b) verschoben wird. Dieser Mechanismus kann beispielsweise so gestaltet sein, dass der zum Verschieben der Kapsel (31) benutzte - nicht gezeigte - Schieber die Kapsel (31) so gegen die Wand der Füllkammer (32b) drückt, dass die Kapsel (31) gerade nach oben ausgerichtet ist und axial unter der jeweiligen Aufnahme (52) für die Pulvereinheiten im Magazin (50) steht. Die Kapsel (31) wird dann wie anhand von Fig. 22 geschildert befüllt und anschließend wieder in die Kammer (32) überführt. Nach der Befüllung wird das Pulver (40) dann durch einen Luftstrom, der beim Einatmen des Patienten am Mundstück (67) entsteht, durch das Sieb (35) ausgebracht. Die Ausbringung erfolgt analog wie in den vorangegangenen Ausführungsbeispielen (siehe insbesondere Fig. 19). Inhalatoren mit solchen Magazinen (50) sind vorzugsweise mit einer Mechanik zur schrittweisen Weiterschaltung des Magazins (50) ausgestattet, die bewirkt, dass nach Entleerung einer Aufnahme (52) die nächste Aufnahme (52) über der neu zu befüllende Kapsel (31) positioniert wird. Eine solche schrittweise Weiterschaltung kann beispielsweise durch einen Betätigungshebel aktiviert werden, der auch andere Vorgänge wie z.B. ein Verschieben der Kapsel (31) in die Füllkammer (32b) bewirken kann, oder an die Bewegung einer am Mundstück (67) angebrachten Kappe gekoppelt sein. In beiden Fällen ist der Mechanismus so ausgelegt, dass der Stempel (56) mit dem Stempelring (57) vor Weiterschaltung des Magazins (50) aus diesem herausgezogen wird.
Bei einer schrittweisen Weiterschaltung des Magazins (50) mittels eines Betätigungshebels können die Aufnahmen (52) - alternativ zur dargestellten axialen Ausrichtung - abgestimmt auf den jeweiligen verwendeten Mechanismus auch radial in dem ringförmigen Träger (51) angeordnet sein. Bezüglich der Konstruktion der Weiterschaltung eines Magazins (50) über einen Betätigungshebel sei hiermit als Beispiel auf die Offenbarung der WO2008138628 mit Bezug auf die darin verwendeten Figuren 9 und 16 auf Seite 20 Zeile 7 bis Seite 23 Zeile 17 und Seite 25 Zeile 1 bis Seite 26 Zeile 11 verwiesen. Der Inhalt jener referenzierter Zeilen sei hiermit vollumfänglich auch zur späteren Aufnahme von Merkmalen mit aufgenommen. Neben der zitierten Mechanik zur schrittweisen Weiterschaltung kann der erfindungsgemäße Inhalator ein Zählwerk bzw. einer Anzeige über die verbleibenden Dosen, einer Vorrichtung zur Sperrung des Geräts bei Erreichen des letzten verfügbaren Pulverdosis und/oder eine Vorrichtung zum Wiedereinschieben der rausgedrückten Einsätze (53) beim Weiterschalten des Magazins (50) aufweisen. Bezüglich der Sperrung des Geräts bei Erreichen der letzten Dosis sei hiermit als Beispiel auf die Offenbarung der WO2008138628 auf Seite 29 Zeile 33 bis Seite 30 Zeile 3 und bezüglich des Wiedereinschiebens der Einsätze auf die Offenbarung der WO2008138628 mit Bezug auf die darin verwendete Figur 17 auf Seite 31 Zeile 19 bis 32 verwiesen. Der Inhalt jener referenzierter Zeilen sei hiermit ebenfalls vollumfänglich mit aufgenommen.
Eine andere hier anwendbare Technik zur schrittweisen Weiterschaltung des Magazins (50) ist die Kopplung mit der Bewegung einer Kappe am Mundstück (67). Mit Hilfe einer solchen Kopplung kann der Inhalator derart bedienerfreundlich gestaltet werden, dass der Anwender bzw. Patient zur Benutzung des Inhalators lediglich die Kappe am Mundstück (67) öffnen muss, das Gerät direkt zur Inhalation an die Lippen setzen kann und nach erfolgter Inhalation lediglich die Kappe wieder schließen muss. Bezüglich der Konstruktion der Weiterschaltung eines Magazins (50) über die Bewegung einer Kappe, die im geschlossenen Zustand das Mundstück (67) abdeckt, sei hiermit als Beispiel auf die Offenbarung der WO2007134793 mit Bezug auf die darin verwendeten Figuren 1 und 4 auf Seite 8 Zeile 28 bis Seite 9 Zeile 31, Seite 11 Zeile 22 bis Seite 12 Zeile 36 und Seite 14 Zeile 4 bis Seite 16 Zeile 31 verwiesen. Der Inhalt jener referenzierter Zeilen sei hiermit vollumfänglich auch zur späteren Aufnahme von Merkmalen mit aufgenommen.

Fig. 23 zeigt schematisch die Funktionsweise eines Geräts (60) bzw. Inhalators, in dem eine Kapsel (31) (Fig. 23a) mit einem vorgefertigtem Loch (46) in dem Gerät so bevorratet ist, dass das Loch (46) im Lagerzustand (Fig. 23b) verschlossen ist, und die Kapsel (31) für die Anwendung des Geräts aus dem Lagerzustand in eine Vibrationskammer (65)(entsprechend der Kapselkammer (13) aus vorhergegangenen Ausführungsformen) verschoben wird.
Fig. 23 a zeigt die oben offene Kapsel (31) mit vorgefertigtem Loch (46) in der parallelen Wand nahe am halbkugelförmigen Boden der Kapsel. Die Kapsel ist mit einer abgemessenen Pulverdosis vorbefüllt (nicht gezeigt).
In Fig. 23b ist eine Kapsel (31) fest in einer zylindrischen Röhre (62) im Gerät (60) gelagert. Ein Schieber (64) mit Einlass hält die Kapsel (31) in Position. Ein flaches Bauteil (63) hindert das Pulver in der Kapsel (31) daran, vor Gebrauch heraus zu fallen. Es kann so aus dem Gerät heraus ragen, dass der Anwender direkt oder an einer Lasche oder ähnlichem daran ziehen kann, bis die Kapsel (31) im Innern freigegeben ist. Vorzugsweise sollte das flache Bauteil (63) dabei nicht ganz aus dem Gerät (60) entfernt werden, so dass keine zusätzliche Öffnung entsteht, durch die Nebenluft ins Innere des Geräts (60) gelangen kann. Vorzugsweise sollte das Material des flachen Bauteil (63) so gewählt sein, dass sich bei Auflage an den oberen Rand der Kapsel (31) eine hart-weich-Dichtung ausbilden kann, d.h. die der Kapsel (31) zugewandte Unterseite des flachen Bauteils (63) sollte beispielsweise bei festem Material der Kapsel (31) eine gewisse Elastizität aufweisen. Das Gerät (60) weist eine Vibrationskammer (65) mit einem Sieb oder Stab (66) an der Oberseite auf. Ein Mundstück (67) ist rechtwinklig zur Vibrationskammer (65) angeordnet, andere Orientierungen des Mundstücks (67) wie z.B. auf einer Achse mit der Vibrationskammer sind ebenfalls möglich.
In Fig. 23c ist die Kapsel (31) in die Vibrationskammer (65) geschoben worden, nachdem zuerst das flache Bauteil (63) aus dem Weg gezogen wurde und der Schieber (64) mit Einlass wie gezeigt in Gerät (60) hinein geschoben wurde. Zur Benutzung inhaliert der Anwender durch das Mundstück (67), wobei er Luft und Pulver in Pfeilrichtung (69) ansaugt. Die Luft tritt in das Gerät (60) über den Einlass am Schieber (64) und in der Richtung des Pfeils 68 ein. Die Kapsel (31) vibriert in der Kammer (65), wobei die Vibration das Pulver durch das Loch (46) aus der Kapsel (31) austreibt.

Einige bevorzugte Bestandteile, Verbindungen und/oder Formulierungen der pharmazeutischen Zubereitung für den Einsatz in den erfindungsgemäßen Kapseln und/oder Inhalatoren werden im Folgenden aufgeführt. Die unten aufgelisteten Verbindungen können für sich genommen oder in Kombination untereinander in Zubereitungen eingesetzt werden.

In den unten aufgeführten Verbindungen ist **W** ein pharmakologisch, aktiver Wirkstoff und (beispielsweise) ausgewählt aus einer Gruppe bestehend aus Betamimetika, Anticholinergika, Corticosteroiden, PDE4-Inhibitoren, LTD4-Rezeptor (CysLT1, CysLT2, CysLT3) Antagonisten, EGFR-Hemmern, Dopamin-Agonisten, H1-Antihistaminika, PAF-Antagonisten, SYK-Inhibitoren, PDE3 Inhibitoren, Lipoxin A4 Derivate, FPRL1 Modulatoren, LTB4-Rezeptor (BLT1, BLT2) Antagonisten, Histamin H1 Rezeptor Antagonisten, Histamin H4 Rezeptor Antagonisten, PI3 Kinase Inhibitoren, Inhibitoren von Nicht-Rezeptor Tyrosine Kinasen wie zum Beispiel LYN, LCK, SYK, ZAP-70, FYN, BTK oder ITK, Inhibitoren von MAP Kinasen wie zum Beispiel p38, ERK1, ERK2, JNK1, JNK2, JNK3 oder SAP, Inhibitoren des NF-κB Signalwegs wie zum Beispiel IKK Kinase Inhibitoren, iNOS Inhibitoren, MRP4 Inhibitoren, Leukotriene Biosynthese Inhibitoren wie zum Beispiel 5-Lipoxygenase (5-LO) Inhibitoren, cPLA2 Inhibitoren, Leukotriene A4 Hydrolase Inhibitoren oder FLAP Inhibitoren, Nicht-steroidale antientzündliche Agenzien (NSAIDs), CRTH2 Antagonisten, DP1-Rezeptor Modulatoren, Thromboxane Rezeptor Antagonisten, Chemokine Rezeptor Antagonisten von CCR1, CCR2, CCR2A, CCR2B, CCR4, CCR5, CCR6, CCR7, CCR8, CCR9, CCR10, CCR11, CXCR1, CXCR2, CXCR3, CXCR4, CXCR5, CXCR6, CX3CR1, Neurokinin (NK1, NK2) Antagonisten, Sphingosine 1-Phosphat Rezeptor Modulatoren, Modulatoren von Adenosine Rezeptoren, Modulatoren von purinergen Rezeptoren wie zum Beispiel P2X7, Histone Deacetylase (HDAC) Aktivatoren, Bradykinin (BK1, BK2) Antagonisten, TACE Inhibitoren, Mucoregulatoren, PPAR gamma Agonisten, Rho Kinase Inhibitoren, interleukin 1-beta converting enzyme (ICE) Inhibitoren, Toll-Like Rezeptor (TLR) Modulatoren, HMG-CoA Reductase Inhibitoren, VLA-4 Antagonisten, ICAM-1 Inhibitoren, SHIP Agonisten, TNFα Antagonisten, GABAa Rezeptor Antagonisten, Immunotherapeutika, Substanzen gegen Schwellungen der Atemwege und Substanzen gegen Husten.

Weiterhin können zwei- oder dreifach Kombinationen von **W** gebildet werden. Beispielhaft genannte Kombinationen von **W** wären:
- **W** stellt ein Betamimetikum dar, kombiniert mit einem Anticholinergikum, Corticosteroid, PDE4-Inhibitor, EGFR-Hemmer oder LTD4-Rezeptor Antagonisten,
- **W** stellt ein Anticholinergikum dar, kombiniert mit einem Betamimetikum, Corticosteroid, PDE4-Inhibitor, EGFR-Hemmen oder LTD4-Rezeptor Antagonisten,
- **W** stellt ein Corticosteroid dar, kombiniert mit einem PDE4-Inhibitor, EGFR-Hemmer oder LTD4-Rezeptor Antagonist
- **W** stellt ein PDE4-Inhibitor dar, kombiniert mit einem EGFR-Hemmer oder LTD4- Rezeptor Antagonist
- **W** stellt ein EGFR-Hemmer dar, kombiniert mit einem Anticholinergikum.

Als Betamimetika gelangen hierbei vorzugsweise Verbindungen zur Anwendung, die ausgewählt sind aus der Gruppe bestehend aus Arformoterol, Carmoterol, Formoterol, Indacaterol, Salmeterol, Albuterole, Bambuterol, Bitolterol, Broxaterol, Carbuterol, Clenbuterol, Fenoterol, Hexoprenalin, Ibuterol, Isoetharin, Isoprenalin, Levosalbutamol, Mabuterol, Meluadrin, Metaproterenol, Milveterol, Orciprenalin, Pirbuterol, Procaterol, Reproterol, Rimiterol, Ritodrin, Salmefamol, Soterenol, Sulphonterol, Terbutalin, Tiaramid, Tolubuterol, Zinterol und
- 6-Hydroxy-8-{1-hydroxy-2-[2-(4-methoxy-phenyl)-1,1-dimethyl-ethylamino]-ethyl}-4H-benzo[1,4]oxazin-3-on
- 8-{2-[2-(2,4-Difluor-phenyl)-1,1-dimethyl-ethylamino]-1-hydroxy-ethyl}-6-hydroxy-4H-benzo[1,4]oxazin-3-on
- 8-{2-[2-(3,5-Difluor-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 8-{2-[2-(4-Fluor-phenyl)-1,1-dimethyl-ethylamino]-1-hydroxy-ethyl}-6-hydroxy-4H-benzo[1,4]oxazin-3-on
- N-(5-{2-[3-(4,4-Diethyl-2-oxo-4H-benzo[d][1,3]oxazin-1-yl)-1,1-dimethyl-propylamino]-1-hydroxy-ethyl}-2-hydroxy-phenyl)-methansulfonamid
- N-(5-{2-[3-(4,4-Diethyl-6-fluoro-2-oxo-4H-benzo[d][1,3]oxazin-1-yl)-1,1-dimethyl-propylamino]-1-hydroxy-ethyl}-2-hydroxy-phenyl)-methansulfonamid
- N-(5-{2-[3-(4,4-Diethyl-6-methoxy-2-oxo-4H-benzo[d][1,3]oxazin-1-yl)-1,1-dimethyl-propylamino]-1-hydroxy-ethyl}-2-hydroxy-phenyl)-methansulfonamid
- N-(5-{2-[1,1-Dimethyl-3-(2-oxo-4,4-dipropyl-4H-benzo[d][1,3]oxazin-1-yl)-propylamino]-1-hydroxy-ethyl}-2-hydroxy-phenyl)-methansulfonamid
- 8-{2-[1,1-Dimethyl-3-(2-oxo-2,3-dihydro-benzoimidazol-1-yl)-propylamino]-1-hydroxyethyl}-6-hydroxy-4H-benzo[1,4]oxazin-3-on
- 8-{2-[1,1-Dimethyl-3-(6-methyl-2-oxo-2,3-dihydro-benzoimidazol-1-yl)-propylamino]-1-hydroxy-ethyl}-6-hydroxy-4H-benzo[1,4]oxazin-3-on
- 8-{2-[1,1-Dimethyl-3-(2-oxo-5-trifluormethyl-2,3-dihydro-benzoimidazol-1-yl)-propylamino]-1-hydroxy-ethyl}-6-hydroxy-4H-benzo[1,4]oxazin-3-on
- 8-{2-[1,1-Dimethyl-3-(3-methyl-2-oxo-2,3-dihydro-benzoimidazol-1-yl)-propylamino]-1-hydroxy-ethyl}-6-hydroxy-4H-benzo[1,4]oxazin-3-on
- N-[2-Hydroxy-5-((1R)-1-hydroxy-2-{2-[4-(2-hydroxy-2-phenyl-ethylamino)-phenyl]-ethylamino}-ethyl)-phenyl]-formamid
- 8-Hydroxy-5-((1R)-1-hydroxy-2-{2-[4-(6-methoxy-biphenyl-3-ylamino)-phenyl]-ethylamino}-ethyl)-1H-chinolin-2-on
- 8-Hydroxy-5-[(1R)-1-hydroxy-2-(6-phenethylamino-hexylamino)-ethyl]-1H-chinolin-2-on
- 5-[(1R)-2-(2-{4-[4-(2-Amino-2-methyl-propoxy)-phenylamino]-phenyl}-ethylamino)-1-hydroxy-ethyl]-8-hydroxy-1H-chinolin-2-on
- [3-(4-{6-[(2R)-2-Hydroxy-2-(4-hydroxy-3-hydroxymethyl-phenyl)-ethylamino]-hexyloxy}-butyl)-5-methyl-phenyl]-harnstoff
- 4-((1R)-2-{6-[2-(2,6-Dichlor-benzyloxy)-ethoxy]-hexylamino}-1-hydroxy-ethyl)-2-hydroxymethyl-phenol
- 3-(4-{6-[(2R)-2-Hydroxy-2-(4-hydroxy-3-hydroxymethyl-phenyl)-ethylamino]-hexyloxy}-butyl)-benzenesulfonamid
- 3-(3-{7-[(2R)-2-Hydroxy-2-(4-hydroxy-3-hydroxymethyl-phenyl)-ethylamino]-heptyloxy}-propyl)-benzenesulfonamid
- 4-((1R)-2-{6-[4-(3-Cyclopentanesulfonyl-phenyl)-butoxy]-hexylamino}-1-hydroxy-ethyl)-2-hydroxymethyl-phenol
- *N*-1-Adamantanyl-2-{3-[(2R)-2-({(2R)-2-hydroxy-2-[4-hydroxy-3-(hydroxymethyl)phenyl]ethyl}amino)propyl]phenyl}acetamid
- (1R)-5-{2-[6-(2,2-Difluor-2-phenyl-ethoxy)-hexylamino]-1-hydroxy-ethyl}-8-hydroxy-1 H-chinolin-2-on
- (R,S)-4-(2-{[6-(2,2-Difluor-4-phenylbutoxy)hexyl]amino}-1-hydroxy-ethyl)-2-(hydroxymethyl)phenol
- (R,S)-4-(2-{[6-(2,2-Difluor-2-phenylethoxy)hexyl]amino}-1-hydroxy-ethyl)-2-(hydroxymethyl)phenol
- (R,S)-4-(2-{[4,4-Difluor-6-(4-phenylbutoxy)hexyl]amino}-1-hydroxy-ethyl)-2- (hydroxymethyl)phenol
- (R,S)-4-(2-{[6-(4,4-Difluor-4-phenylbutoxy)hexyl]amino}-1-hydroxy-ethyl)-2-(hydroxymethyl)phenol
- (R,S)-5-(2-{[6-(2,2-Difluor-2-phenylethoxy)hexyl]amino}-1-hydroxy-ethyl)-8- hydroxychinolin-2(1 H)-on
- (R,S)-[2-({6-[2,2-Difluor-2-(3-methylphenyl)ethoxy]hexyl}amino)-1- hydroxyethyl]-2-(hydroxymethyl)phenol
- 4-(1R)-2-{[6-(2,2-Difluor-2-phenylethoxy)hexyl]amino}-1-hydroxyethyl)-2-(hydroxymethyl)phenol
- (R,S)-2-(Hydroxymethyl)-4-(1-hydroxy-2-{[4,4,5I5-tetrafluor-6-(3-phenylpropoxy)-hexyl]amino}ethyl)phenol
- (R,S)-[5-(2-{[6-(2,2-Difluor-2-phenylethoxy)hexyl]amino}-1-hydroxy-ethyl)-2- hydroxyphenyl]formamid
- (R,S)-4-[2-({6-[2-(3-Bromophenyl)-2,2-difluoroethoxy]hexyl}amino)-1-hydroxyethyl]- 2-(hydroxymethyl)phenol
- (R, S)-N-[3-(1,1 -Difluor-2-{[6-({2-hydroxy-2-[4-hydroxy-3-(hydroxymethyl)phenyl]-ethyl}amino)hexyl]oxy}ethyl)phenyl]-harnstoff
- 3-[3-(1,1-Difluor-2-{[6-({2-hydroxy-2-[4-hydroxy-3-(hydroxymethyl) phenyl]ethyl}-amino)hexyl]oxy}ethyl)phenyl]imidazolidin-2,4-dion
- (R,S)-4-[2-({6-[2,2-Difluor-2-(3-methoxyphenyl)ethoxy]hexyl}amino)-1-hydroxyethyl]-2-(hydroxymethyl)phenol
- 5-((1R)-2-{[6-(2,2-Difluor-2-phenylethoxy)hexyl]amino}-1-hydroxyethyl)-8- hydroxychinolin-2(1H)-on
- 4-((1R)-2-{[4,4-Difluor-6-(4-phenylbutoxy)hexyl]amino}-1-hydroxy-ethyl)-2-(hydroxymethyl)phenol
- (R,S)-4-(2-{[6-(3,3-Difluor-3-phenylpropoxy)hexyl]amino}-1-hydroxy-ethyl)-2-(hydroxymethyl)phenol
- (R,S)-(2-{[6-(2,2-Difluor-2-phenylethoxy)-4,4-difluorohexyl]amino}-1-hydroxyethyl)-2-(hydroxymethyl)phenol
- (R,S)-4-(2-{[6-(2,2-Difluor-3-phenylpropoxy)hexyl]amino}-1-hydroxy ethyl)-2- (hydroxymethyl)phenol
- 3-[2-(3-Chlor-phenyl)-ethoxy]-N-(2-diethylamino-ethyl)-N-{2-[2-(4-hydroxy-2-oxo-2,3-dihydro-benzothiazol-7-yl)-ethylamino]-ethyl}-propionamid
- N-(2-Diethylamino-ethyl)-N-{2-[2-(4-hydroxy-2-oxo-2,3-dihydro-benzothiazol-7-yl)-ethylamino]-ethyl}-3-(2-naphthalen-1-yl-ethoxy)-propionamid
- 7-[2-(2-{3-[2-(2-Chlor-phenyl)-ethylamino]-propylsulfanyl}-ethylamino)-1-hydroxyethyl]-4-hydroxy-3H-benzothiazol-2-on
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus: Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Aclidiniumsalze, bevorzugt das Bromidsalz, Glycopyrroniumsalzen, bevorzugt das Bromidsalz, Trospiumsalzen, bevorzugt das Chloridsalz, Tolterodin, (3R)-1-Phenethyl-3-(9H-xanthen-9-carbonyloxy)-1-azoniabicyclo[2.2.2]octan-Salze. In den vorstehend genannten Salzen stellen die Kationen die pharmakologisch aktiven Bestandteile dar. Als Anionen X'können die vorstehend genannten Salze bevorzugt enthalten Chlorid, Bromid, lodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, lodid, Sulfat, Methansulfonat oder p-Toluolsulfonat als Gegenionen bevorzugt sind. Von allen Salzen sind die Chloride, Bromide, lodide und Methansulfonate besonders bevorzugt. Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid
Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus: Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, Tipredane und Pregna-1,4-diene-3,20-dione, 6-fluoro-11-hydroxy-16,17-[(1-methylethylidene) bis(oxy)]-21-[[4-[(nitrooxy)methyl]benzoyl]oxy]-, (6-alpha,11-beta,16-alpha)-(9Cl) (NCX-1024)
- 16,17-butylidenedioxy-6,9-difluoro-11-hydroxy-17-(methylthio)androst-4-en-3-one (RPR-106541),
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6-alpha,9-alpha-difluoro-11-beta-hydroxy-16alpha-methyl-3-oxo-17alpha-(2,2,3,3-tetramethylcyclopropylcarbonyl)oxy-androsta-1,4-diene-17beta-carbonsäure cyanomethyl ester,
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), TofimilastPumafentrin, Lirimilast, Apremilast, Arofyllin, Atizoram, Oglemilast, Tetomilast, und
- 5-[(N-(2,5-dichloro-3-pyridinyl)-carboxamid]-8-methoxy-Chinolin (D-4418),
- N-(3,5-dichloro-1-oxido-4-pyridinyl)-carboxamid]-8-methoxy-2-(trifluoromethyl)-Chinolin (D-4396 (Sch-351591)),N-(3,5-dichloropyrid-4-yl)-[1-(4-fluorobenzyl)-5-hydroxy-indol-3-yl]glyoxylsäureamid (AWD-12-281 (GW-842470)), 9-[(2-fluorophenyl)methyl]-N-methyl-2-(trifluoromethyl)-9H-Purin-6-amine (NCS-613),
- 4-[(2R)-2-[3-(cyclopentyloxy)-4-methoxyphenyl]-2-phenylethyl]-Pyridine (CDP-840),
- N-[(3R)-3,4,6,7-tetrahydro-9-methyl-4-oxo-1-phenylpyrrolo[3,2,1-jk][1,4]benzodiazepin-3-yl]-4-Pyridinecarboxamide (PD-168787),
- 4-[6,7-diethoxy-2,3-bis(hydroxymethyl)-1-naphthalenyl]-1-(2-methoxyethyl)-2(1H)-Pyridinone (T-440),
- 2-[4-[6,7-diethoxy-2,3-bis(hydroxymethyl)-1-naphthalenyl]-2-pyridinyl]-4-(3-pyridinyl)-1(2H)-Phthalazinone (T-2585),
- (3-(3-cyclopenyloxy-4-methoxybenzyl)-6-ethylamino-8-isopropyl-3H-purine (V-11294A),
- beta-[3-(cyclopentyloxy)-4-methoxyphenyl]-1,3-dihydro-1,3-dioxo-2H-Isoindole-2-propanamid (CDC-801),
- Imidazo[1,5-a]pyrido[3,2-e]pyrazin-6(5H)-one, 9-ethyl-2-methoxy-7-methyl-5-propyl-(D-22888)
- 5-[3-(cyclopentyloxy)-4-methoxyphenyl]-3-[(3-methylphenyl)methyl]-,(3S,5S)-2-Piperidinon (HT-0712),
- 4-[1-[3,4-bis(difluoromethoxy)phenyl]-2-(3-methyl-1-oxido-4-pyridinyl)ethyl]-alpha,alpha-bis(trifluoromethyl)-Benzenemethanol(L-826141)
- N-(3,5-Dichloro-1-oxo-pyridin-4-yl)-4-difluormethoxy-3-cyclopropylmethoxybenzamid
- (-)p-[(4*a*R*, 10*b*S*)-9-Ethoxy-1,2,3,4,4a,10b-hexahydro-8-methoxy-2-methylbenzo[s][1,6]naphthyridin-6-yl]-N, N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxyphenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9H-pyrazolo[3,4-c]-1,2,4-triazolo[4,3-a]pyridin
- 9-Cyclopentyl-5,6-dihydro-7-ethyl-3-(tert-butyl)-9H-pyrazolo[3,4-c]-1,2,4-triazolo[4,3-a]pyridin,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als EGFR-Hemmer gelangen hierbei vorzugsweise Verbindungen zur Anwendung, die ausgewählt sind aus der Gruppe bestehend ausCetuximab, Trastuzumab, Panitumumab ( = ABX-EGF), Mab ICR-62, Gefitinib, Canertinib, Erlotinib, und
- 4-[(3-Chlor-4-fluorphenyl)amino]-6-{[4-(morpholin-4-yl)-1-oxo-2-buten-1-yl]amino}-7-cyclopropylmethoxy-chinazolin,
- 4-[(3-Chlor-4-fluorphenyl)amino]-6-{[4-(N,N-diethylamino)-1-oxo-2-buten-1-yl]amino}-7-cyclopropylmethoxy-chinazolin,
- 4-[(3-Chlor-4-fluorphenyl)amino]-6-{[4-(N,N-dimethylamino)-1-oxo-2-buten-1-yl]amino}-7-cyclopropylmethoxy-chinazolin,
- 4-[(R)-(1-Phenyl-ethyl)amino]-6-{[4-(morpholin-4-yl)-1-oxo-2-buten-1-yl]amino}-7-cyclopentyloxy-chinazolin,
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin,
- 4-[(3-Chlor-4-fluorphenyl)amino]-6-{[4-(N,N-dimethylamino)-1-oxo-2-buten-1-yl]amino}-7-cyclopentyloxy-chinazolin,
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin,
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin,
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin,
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin,
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin,
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin,
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin,
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin,
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin,
- 4-[(3-Ethinyl-phenyl)amino]-6,7-bis-(2-methoxy-ethoxy)-chinazolin,
- 4-[(3-Chlor-4-fluorphenyl)amino]-7-[3-(morpholin-4-yl)-propyloxy]-6-[(vinylcarbonyl)amino]-chinazolin,
- 4-[(R)-(1-Phenyl-ethyl)amino]-6-(4-hydroxy-phenyl)-7H-pyrrolo[2,3-d]pyrimidin,
- 3-Cyano-4-[(3-chlor-4-fluorphenyl)amino]-6-{[4-(N,N-dimethylamino)-1-oxo-2-buten-1-yl]amino}-7-ethoxy-chinolin,
- 4-{[3-Chlor-4-(3-fluor-benzyloxy)-phenyl]amino}-6-(5-{[(2-methansulfonyl-ethyl)amino]methyl}-furan-2-yl)chinazolin,
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin,
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin,
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin,
- 4-[(3-Ethinyl-phenyl)amino]-6-{[4-(5,5-dimethyl-2-oxo-morpholin-4-yl)-1-oxo-2-buten-1-yl]amino}-chinazolin,
- 4-[(3-Chlor-4-fluor-phenyl)amino]-6-[2-(2,2-dimethyl-6-oxo-morpholin-4-yl)-ethoxy]-7-methoxy-chinazolin,
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin,
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin,
- 4-[(3-Chlor-4-fluor-phenyl)amino]-6-{2-[4-(2-oxo-morpholin-4-yl)-piperidin-1-yl]-ethoxy}-7-methoxy-chinazolin,
- 4-[(3-Chlor-4-fluor-phenyl)amino]-6-[1-(tert.-butyloxycarbonyl)-piperidin-4-yloxy]-7-methoxy-chinazolin,
- 4-[(3-Chlor-4-fluor-phenyl)amino]-6-(trans-4-amino-cyclohexan-1-yloxy)-7-methoxy-chinazolin,
- 4-[(3-Chlor-4-fluor-phenyl)amino]-6-(trans-4-methansulfonylamino-cyclohexan-1-yloxy)-7-methoxy-chinazolin,
- 4-[(3-Chlor-4-fluor-phenyl)amino]-6-(tetrahydropyran-3-yloxy)-7-methoxy-chinazolin,
- 4-[(3-Chlor-4-fluor-phenyl)amino]-6-(1-methyl-piperidin-4-yloxy)-7-methoxy-chinazolin,
- 4-[(3-Chlor-4-fluor-phenyl)amino]-6-{1-[(morpholin-4-yl)carbonyl]-piperidin-4-yloxy}-7-methoxy-chinazolin,
- 4-[(3-Chlor-4-fluor-phenyl)amino]-6-{1-[(methoxymethyl)carbonyl]-piperidin-4-yloxy}-7-methoxy-chinazolin,
- 4-[(3-Chlor-4-fluor-phenyl)amino]-6-(piperidin-3-yloxy)-7-methoxy-chinazolin,
- 4-[(3-Chlor-4-fluor-phenyl)amino]-6-[1-(2-acetylamino-ethyl)-piperidin-4-yloxy]-7-methoxy-chinazolin,
- 4-[(3-Chlor-4-fluor-phenyl)amino]-6-(tetrahydropyran-4-yloxy)-7-ethoxy-chinazolin,
- 4-[(3-Chlor-4-fluor-phenyl)amino]-6-((S)-tetrahydrofuran-3-yloxy)-7-hydroxy-chinazolin,
- 4-[(3-Chlor-4-fluor-phenyl)amino]-6-(tetrahydropyran-4-yloxy)-7-(2-methoxy-ethoxy)-chinazolin,
- 4-[(3-Chlor-4-fluor-phenyl)amino]-6-{trans-4-[(dimethylamino)sulfonylamino]-cyclohexan-1-yloxy}-7-methoxy-chinazolin,
- 4-[(3-Chlor-4-fluor-phenyl)amino]-6-{trans-4-[(morpholin-4-yl)carbonylamino]-cyclohexan-1-yloxy}-7-methoxy-chinazolin,
- 4-[(3-Chlor-4-fluor-phenyl)amino]-6-{trans-4-[(morpholin-4-yl)sulfonylamino]-cyclohexan-1-yloxy}-7-methoxy-chinazolin,
- 4-[(3-Chlor-4-fluor-phenyl)amino]-6-(tetrahydropyran-4-yloxy)-7-(2-acetylamino-ethoxy)-chinazolin,
- 4-[(3-Chlor-4-fluor-phenyl)amino]-6-(tetrahydropyran-4-yloxy)-7-(2-methansulfonylamino-ethoxy)-chinazolin,
- 4-[(3-Chlor-4-fluor-phenyl)amino]-6-{1-[(piperidin-1-yl)carbonyl]-piperidin-4-yloxy}-7-methoxy-chinazolin,
- 4-[(3-Chlor-4-fluor-phenyl)amino]-6-(1-aminocarbonylmethyl-piperidin-4-yloxy)-7-methoxy-chinazolin,
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin,
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin,
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin,
- 4-[(3-Chlor-4-fluor-phenyl)amino]-6-(trans-4-ethansulfonylamino-cyclohexan-1-yloxy)-7-methoxy-chinazolin,
- 4-[(3-Chlor-4-fluor-phenyl)amino]-6-(1-methansulfonyl-piperidin-4-yloxy)-7-ethoxy-chinazolin,
- 4-[(3-Chlor-4-fluor-phenyl)amino]-6-(1-methansulfonyl-piperidin-4-yloxy)-7-(2-methoxy-ethoxy)-chinazolin,
- 4-[(3-Chlor-4-fluor-phenyl)amino]-6-[1-(2-methoxy-acetyl)-piperidin-4-yloxy]-7-(2-methoxy-ethoxy)-chinazolin,
- 4-[(3-Chlor-4-fluor-phenyl)amino]-6-(cis-4-acetylamino-cyclohexan-1-yloxy)-7-methoxy-chinazolin,
- 4-[(3-Ethinyl-phenyl)amino]-6-[1-(tert.-butyloxycarbonyl)-piperidin-4-yloxy]-7-methoxy-chinazolin,
- 4-[(3-Ethinyl-phenyl)amino]-6-(tetrahydropyran-4-yloxy]-7-methoxy-chinazolin,
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin,
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin,
- 4-[(3-Chlor-4-fluor-phenyl)amino]-6-{cis-4-[(morpholin-4-yl)carbonylamino]-cyclohexan-1-yloxy}-7-methoxy-chinazolin,
- 4-[(3-Chlor-4-fluor-phenyl)amino]-6-{1-[2-(2-oxopyrrolidin-1-yl)ethyl]-piperidin-4-yloxy}-7-methoxy-chinazolin,
- 4-[(3-Chlor-4-fluor-phenyl)amino]-6-{1-[(morpholin-4-yl)carbonyl]-piperidin-4-yloxy}-7-(2-methoxy-ethoxy)-chinazolin,
- 4-[(3-Ethinyl-phenyl)amino]-6-(1-acetyl-piperidin-4-yloxy)-7-methoxy-chinazolin,
- 4-[(3-Ethinyl-phenyl)amino]-6-(1-methyl-piperidin-4-yloxy)-7-methoxy-chinazolin,
- 4-[(3-Ethinyl-phenyl)amino]-6-(1-methansulfonyl-piperidin-4-yloxy)-7-methoxy-chinazolin,
- 4-[(3-Chlor-4-fluor-phenyl)amino]-6-(1-methyl-piperidin-4-yloxy)-7(2-methoxy-ethoxy)-chinazolin,
- 4-[(3-Chlor-4-fluor-phenyl)amino]-6-(1-isopropyloxycarbonyl-piperidin-4-yloxy)-7-methoxy-chinazolin,
- 4-[(3-Chlor-4-fluor-phenyl)amino]-6-(cis-4-methylamino-cyclohexan-1-yloxy)-7-methoxy-chinazolin,
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin,
- 4-[(3-Ethinyl-phenyl)amino]-6-(piperidin-4-yloxy)-7-methoxy-chinazolin,
- 4-[(3-Ethinyl-phenyl)amino]-6-[1-(2-methoxy-acetyl)-piperidin-4-yloxy]-7-methoxy-chinazolin,
- 4-[(3-Ethinyl-phenyl)amino]-6-{1-[(morpholin-4-yl)carbonyl]-piperidin-4-yloxy}-7-methoxy-chinazolin,
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin,
- 4-[(3-Chlor-4-fluor-phenyl)amino]-6-{1-[(2-methyl-morpholin-4-yl)carbonyl]-piperidin-4-yloxy}-7-methoxy-chinazolin,
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin,
- 4-[(3-Chlor-4-fluor-phenyl)amino]-6-{1-[(N-methyl-N-2-methoxyethyl-amino)carbonyl]-piperidin-4-yloxy}-7-methoxy-chinazolin,
- 4-[(3-Chlor-4-fluor-phenyl)amino]-6-(1-ethyl-piperidin-4-yloxy)-7-methoxy-chinazolin,
- 4-[(3-Chlor-4-fluor-phenyl)amino]-6-{1-[(2-methoxyethyl)carbonyl]-piperidin-4-yloxy}-7-methoxy-chinazolin,
- 4-[(3-Chlor-4-fluor-phenyl)amino]-6-{1-[(3-methoxypropyl-amino)-carbonyl]-piperidin-4-yloxy}-7-methoxy-chinazolin,
- 4-[(3-Chlor-4-fluor-phenyl)amino]-6-[cis-4-(N-methansulfonyl-N-methyl-amino)-cyclohexan-1-yloxy]-7-methoxy-chinazolin,
- 4-[(3-Chlor-4-fluor-phenyl)amino]-6-[cis-4-(N-acetyl-N-methyl-amino)-cyclohexan-1-yloxy]-7-methoxy-chinazolin,
- 4-[(3-Chlor-4-fluor-phenyl)amino]-6-(trans-4-methylamino-cyclohexan-1-yloxy)-7-methoxy-chinazolin,
- 4-[(3-Chlor-4-fluor-phenyl)amino]-6-[trans-4-(N-methansulfonyl-N-methyl-amino)-cyclohexan-1-yloxy]-7-methoxy-chinazolin,
- 4-[(3-Chlor-4-fluor-phenyl)amino]-6-(trans-4-dimethylamino-cyclohexan-1-yloxy)-7-methoxy-chinazolin,
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin,
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin,
- 4-[(3-Chlor-4-fluor-phenyl)amino]-6-(1-methansulfonyl-piperidin-4-yloxy)-7-methoxy-chinazolin,
- 4-[(3-Chlor-4-fluor-phenyl)amino]-6-(1-cyano-piperidin-4-yloxy)-7-methoxy-chinazolin,
- 3-Cyano-4-[(3-chlor-4-fluorphenyl)amino]-6-{[4-(N,N-dimethylamino)-1-oxo-2-buten-1-yl]amino}-7-ethoxy-chinolin ;
- [4-[(3-chloro-4-fluoro-phenyl)amino]-6-{[4-(homomorpholin-4-yl)-1-oxo-2-buten-1-yl]amino}-7-[(S)-(tetrahydrofuran-3-yl)oxy]-quinazoline ,
- 4-[(3-Chlor-4-fluor-phenyl)amino]-7-(2-{4-[(S)-(2-oxo-tetrahydrofuran-5-yl)carbonyl]-piperazin-1-yl}-ethoxy)-6-[(vinylcarbonyl)amino]-chinazolin,
- 4-[(3-Chlor-4-fluor-phenyl)amino]-7-[2-((S)-6-methyl-2-oxo-morpholin-4-yl)-ethoxy]-6-[(vinylcarbonyl)amino]-chinazolin,
- 4-[(3-Chlor-4-fluor-phenyl)amino]-7-[4-((R)-6-methyl-2-oxo-morpholin-4-yl)-butyloxy]-6-[(vinylcarbonyl)amino]-chinazolin,
- 4-[(3-Chlor-4-fluor-phenyl)amino]-7-[4-((S)-6-methyl-2-oxo-morpholin-4-yl)-butyloxy]-6-[(vinylcarbonyl)amino]-chinazolin, und
- 4-[(3-Chlor-4-fluorphenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-chinazolin,
gegebenenfalls in Form ihrer Racemate, Enantiomere oder Diastereomere, gegebenenfalls in Form ihrer pharmakologisch verträglichen Säureadditionssalze, ihrer Solvate und/oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als EGFR-Hemmer gelangen hierbei vorzugsweise Verbindungen zur Anwendung, die ausgewählt sind aus der Gruppe bestehend aus
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Ethinyl-phenyl)amino]-6,7-bis-(2-methoxy-ethoxy)-chinazolin,
- 4-[(3-Chlor-4-fluorphenyl)amino]-7-[3-(morpholin-4-yl)-propyloxy]-6-[(vinylcarbonyl)amino]-chinazolin,
- 3-Cyano-4-[(3-chlor-4-fluorphenyl)amino]-6-{[4-(N,N-dimethylamino)-1-oxo-2-buten-1-yl]amino}-7-ethoxy-chinolin,
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin,
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin,
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin,
- 4-[(3-Ethinyl-phenyl)amino]-6-{[4-(5,5-dimethyl-2-oxo-morpholin-4-yl)-1-oxo-2-buten-1-yl]amino}-chinazolin,
- 4-[(3-Chlor-4-fluor-phenyl)amino]-6-[2-(2,2-dimethyl-6-oxo-morpholin-4-yl)-ethoxy]-7-methoxy-chinazolin,
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin,
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin,
- 4-[(3-Chlor-4-fluor-phenyl)amino]-6-{2-[4-(2-oxo-morpholin-4-yl)-piperidin-1-yl]-ethoxy}-7-methoxy-chinazolin,
- 4-[(3-Chlor-4-fluor-phenyl)amino]-6-[1-(tert.-butyloxycarbonyl)-piperidin-4-yloxy]-7-methoxy-chinazolin,
- 4-[(3-Chlor-4-fluor-phenyl)amino]-6-(trans-4-amino-cyclohexan-1-yloxy)-7-methoxy-chinazolin,
- 4-[(3-Chlor-4-fluor-phenyl)amino]-6-(trans-4-methansulfonylamino-cyclohexan-1-yloxy)-7-methoxy-chinazolin,
- 4-[(3-Chlor-4-fluor-phenyl)amino]-6-(tetrahydropyran-3-yloxy)-7-methoxy-chinazolin,
- 4-[(3-Chlor-4-fluor-phenyl)amino]-6-(1-methyl-piperidin-4-yloxy)-7-methoxy-chinazolin,
- 4-[(3-Chlor-4-fluor-phenyl)amino]-6-{1-[(morpholin-4-yl)carbonyl]-piperidin-4-yloxy}-7-methoxy-chinazolin,
- 4-[(3-Chlor-4-fluor-phenyl)amino]-6-{1-[(methoxymethyl)carbonyl]-piperidin-4-yloxy}-7-methoxy-chinazolin,
- 4-[(3-Chlor-4-fluor-phenyl)amino]-6-(piperidin-3-yloxy)-7-methoxy-chinazolin,
- 4-[(3-Chlor-4-fluor-phenyl)amino]-6-[1-(2-acetylamino-ethyl)-piperidin-4-yloxy]-7-methoxy-chinazolin,
- 4-[(3-Chlor-4-fluor-phenyl)amino]-6-(tetrahydropyran-4-yloxy)-7-ethoxy-chinazolin,
- 4-[(3-Chlor-4-fluor-phenyl)amino]-6-((S)-tetrahydrofuran-3-yloxy)-7-hydroxy-chinazolin,
- 4-[(3-Chlor-4-fluor-phenyl)amino]-6-(tetrahydropyran-4-yloxy)-7-(2-methoxy-ethoxy)-chinazolin,
- 4-[(3-Chlor-4-fluor-phenyl)amino]-6-{trans-4-[(dimethylamino)sulfonylamino]-cyclohexan-1-yloxy}-7-methoxy-chinazolin,
- 4-[(3-Chlor-4-fluor-phenyl)amino]-6-{trans-4-[(morpholin-4-yl)carbonylamino]-cyclohexan-1-yloxy}-7-methoxy-chinazolin,
- 4-[(3-Chlor-4-fluor-phenyl)amino]-6-{trans-4-[(morpholin-4-yl)sulfonylamino]-cyclohexan-1-yloxy}-7-methoxy-chinazolin,
- 4-[(3-Chlor-4-fluor-phenyl)amino]-6-(tetrahydropyran-4-yloxy)-7-(2-acetylamino-ethoxy)-chinazolin,
- 4-[(3-Chlor-4-fluor-phenyl)amino]-6-(tetrahydropyran-4-yloxy)-7-(2-methansulfonylamino-ethoxy)-chinazolin,
- 4-[(3-Chlor-4-fluor-phenyl)amino]-6-{1-[(piperidin-1-yl)carbonyl]-piperidin-4-yloxy}-7-methoxy-chinazolin,
- 4-[(3-Chlor-4-fluor-phenyl)amino]-6-(1-aminocarbonylmethyl-piperidin-4-yloxy)-7-methoxy-chinazolin,
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin,
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin,
- 4-[(3-Chlor-4-fluor-phenyl)amino]-6-(trans-4-ethansulfonylamino-cyclohexan-1-yloxy)-7-methoxy-chinazolin,
- 4-[(3-Chlor-4-fluor-phenyl)amino]-6-(1-methansulfonyl-piperidin-4-yloxy)-7-ethoxy-chinazolin,
- 4-[(3-Chlor-4-fluor-phenyl)amino]-6-(1-methansulfonyl-piperidin-4-yloxy)-7-(2-methoxy-ethoxy)-chinazolin,
- 4-[(3-Chlor-4-fluor-phenyl)amino]-6-[1-(2-methoxy-acetyl)-piperidin-4-yloxy]-7-(2-methoxy-ethoxy)-chinazolin,
- 4-[(3-Chlor-4-fluor-phenyl)amino]-6-(cis-4-acetylamino-cyclohexan-1-yloxy)-7-methoxy-chinazolin,
- 4-[(3-Ethinyl-phenyl)amino]-6-[1-(tert.-butyloxycarbonyl)-piperidin-4-yloxy]-7-methoxy-chinazolin,
- 4-[(3-Ethinyl-phenyl)amino]-6-(tetrahydropyran-4-yloxy]-7-methoxy-chinazolin,
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin,
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin,
- 4-[(3-Chlor-4-fluor-phenyl)amino]-6-{cis-4-[(morpholin-4-yl)carbonylamino]-cyclohexan-1-yloxy}-7-methoxy-chinazolin,
- 4-[(3-Chlor-4-fluor-phenyl)amino]-6-{1-[2-(2-oxopyrrolidin-1-yl)ethyl]-piperidin-4-yloxy}-7-methoxy-chinazolin,
- 4-[(3-Chlor-4-fluor-phenyl)amino]-6-{1-[(morpholin-4-yl)carbonyl]-piperidin-4-yloxy}-7-(2-methoxy-ethoxy)-chinazolin,
- 4-[(3-Ethinyl-phenyl)amino]-6-(1-acetyl-piperidin-4-yloxy)-7-methoxy-chinazolin,
- 4-[(3-Ethinyl-phenyl)amino]-6-(1-methyl-piperidin-4-yloxy)-7-methoxy-chinazolin,
- 4-[(3-Ethinyl-phenyl)amino]-6-(1-methansulfonyl-piperidin-4-yloxy)-7-methoxy-chinazolin,
- 4-[(3-Chlor-4-fluor-phenyl)amino]-6-(1-methyl-piperidin-4-yloxy)-7(2-methoxy-ethoxy)-chinazolin,
- 4-[(3-Chlor-4-fluor-phenyl)amino]-6-(1-isopropyloxycarbonyl-piperidin-4-yloxy)-7-methoxy-chinazolin,
- 4-[(3-Chlor-4-fluor-phenyl)amino]-6-(cis-4-methylamino-cyclohexan-1-yloxy)-7-methoxy-chinazolin,
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin,
- 4-[(3-Ethinyl-phenyl)amino]-6-(piperidin-4-yloxy)-7-methoxy-chinazolin,
- 4-[(3-Ethinyl-phenyl)amino]-6-[1-(2-methoxy-acetyl)-piperidin-4-yloxy]-7-methoxy-chinazolin,
- 4-[(3-Ethinyl-phenyl)amino]-6-{1-[(morpholin-4-yl)carbonyl]-piperidin-4-yloxy}-7-methoxy-chinazolin,
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin,
- 4-[(3-Chlor-4-fluor-phenyl)amino]-6-{1-[(2-methyl-morpholin-4-yl)carbonyl]-piperidin-4-yloxy}-7-methoxy-chinazolin,
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin,
- 4-[(3-Chlor-4-fluor-phenyl)amino]-6-{1-[(N-methyl-N-2-methoxyethyl-amino)carbonyl]-piperidin-4-yloxy}-7-methoxy-chinazolin,
- 4-[(3-Chlor-4-fluor-phenyl)amino]-6-(1-ethyl-piperidin-4-yloxy)-7-methoxy-chinazolin,
- 4-[(3-Chlor-4-fluor-phenyl)amino]-6-{1-[(2-methoxyethyl)carbonyl]-piperidin-4-yloxy}-7-methoxy-chinazolin,
- 4-[(3-Chlor-4-fluor-phenyl)amino]-6-{1-[(3-methoxypropyl-amino)-carbonyl]-piperidin-4-yloxy}-7-methoxy-chinazolin,
- 4-[(3-Chlor-4-fluor-phenyl)amino]-6-[cis-4-(N-methansulfonyl-N-methyl-amino)-cyclohexan-1-yloxy]-7-methoxy-chinazolin,
- 4-[(3-Chlor-4-fluor-phenyl)amino]-6-[cis-4-(N-acetyl-N-methyl-amino)-cyclohexan-1-yloxy]-7-methoxy-chinazolin,
- 4-[(3-Chlor-4-fluor-phenyl)amino]-6-(trans-4-methylamino-cyclohexan-1-yloxy)-7-methoxy-chinazolin,
- 4-[(3-Chlor-4-fluor-phenyl)amino]-6-[trans-4-(N-methansulfonyl-N-methyl-amino)-cyclohexan-1-yloxy]-7-methoxy-chinazolin,
- 4-[(3-Chlor-4-fluor-phenyl)amino]-6-(trans-4-dimethylamino-cyclohexan-1-yloxy)-7-methoxy-chinazolin,
- 4-[(3-Chlor-4-fluor-phenyl)amino]-6-(trans-4-{N-[(morpholin-4-yl)carbonyl]-N-methyl-amino}-cyclohexan-1-yloxy)-7-methoxy-chinazolin,
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin,
- 4-[(3-Chlor-4-fluor-phenyl)amino]-6-(1-methansulfonyl-piperidin-4-yloxy)-7-methoxy-chinazolin,
- 4-[(3-Chlor-4-fluor-phenyl)amino]-6-(1-cyano-piperidin-4-yloxy)-7-methoxy-chinazolin,
- 3-Cyano-4-[(3-chlor-4-fluorphenyl)amino]-6-{[4-(N,N-dimethylamino)-1-oxo-2-buten-1-yl]amino}-7-ethoxy-chinolin
- [4-[(3-chloro-4-fluoro-phenyl)amino]-6-{[4-(homomorpholin-4-yl)-1-oxo-2-buten-1-yl]amino}-7-[(S)-(tetrahydrofuran-3-yl)oxy]-chinazoline
- 4-[(3-Chlor-4-fluor-phenyl)amino]-7-(2-{4-[(S)-(2-oxo-tetrahydrofuran-5-yl)carbonyl]-piperazin-1-yl}-ethoxy)-6-[(vinylcarbonyl)amino]-chinazolin
- 4-[(3-Chlor-4-fluor-phenyl)amino]-7-[2-((S)-6-methyl-2-oxo-morpholin-4-yl)-ethoxy]-6-[(vinylcarbonyl)amino]-chinazolin
- 4-[(3-Chlor-4-fluor-phenyl)amino]-7-[4-((R)-6-methyl-2-oxo-morpholin-4-yl)-butyloxy]-6-[(vinylcarbonyl)amino]-chinazolin
- 4-[(3-Chlor-4-fluor-phenyl)amino]-7-[4-((S)-6-methyl-2-oxo-morpholin-4-yl)-butyloxy]-6-[(vinylcarbonyl)amino]-chinazolin
- 4-[(3-Chlor-4-fluorphenyl)amino]-6-[(4-{N-[2-(ethoxycarbonyl)-ethyl]-N-[(ethoxycarbonyl)methyl]amino}-1-oxo-2-buten-1-yl)amino]-7-cyclopropylmethoxy-chinazolin
Cetuximab, Trastuzumab, ABX-EGF, Mab ICR-62, Gefitinib, Canertinib und Erlotinib, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Rezeptor Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als PAF-Antagonisten gelangen hierbei vorzugsweise Verbindungen zur Anwendung, die ausgewählt sind aus der Gruppe bestehend aus Lexipafant und 4-(2-Chlorphenyl)-9-methyl-2-[3(4-morpholinyl)-3-propanon-1-yl]-6H-thieno-[3,2-f]-[1,2,4]triazolo[4,3-a][1,4]diazepin
- 6-(2-Chlorphenyl)-8,9-dihydro-1-methyl-8-[(4-morpholinyl)carbonyl]-4H,7H-cyclo-penta-[4,5]thieno-[3,2-f][1,2,4]triazolo[4,3-a][1,4]diazepin,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTB4-Rezeptor Antagonisten gelangen hierbei vorzugsweise Verbindungen zur Anwendung wie z.B. Amebulant (=[[4-[[3-[[4-[1-(4-hydroxyphenyl)-1-methylethyl]phenoxy]methyl]phenyl]methoxy]phenyl]iminomethyl]-Carbaminsäure-ethyl ester), gegebenenfalls in Form ihrer Racemate, Enantiomere, Diastereomere und gegebenenfalls in Form ihrer pharmakologisch verträglichen Säureadditionssalze, Solvate, Prodrugs oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Rezeptor Antagonisten gelangen hierbei vorzugsweise Verbindungen zur Anwendung, die ausgewählt sind aus der Gruppe bestehend aus Montelukast, Pranlukast, Zafirlukast, und (E)-8-[2-[4-[4-(4-Fluorophenyl)butoxy]phenyl]ethenyl]-2-(1H-tetrazol-5-yl)-4H-1-benzopyran-4-one (MEN-91507)
- 4-[6-Acetyl-3-[3-(4-acetyl-3-hydroxy-2-propylphenylthio)propoxy]-2-propylphenoxy]-buttersäure (MN-001)
- 1-(((R)-(3-(2-(6,7-Difluor-2-chinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yl)-(E)-ethenyl)phenyl)-3-(2-(1-hydroxy-1-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Rezeptor Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als Histamin H1 Rezeptor Antagonisten gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Olopatadine, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Histamin H4 Rezeptor Antagonisten gelangen hierbei vorzugsweise Verbindungen zur Anwendung wie z.B. (5-chloro-1H-indol-2-yl)(4-methyl-1-piperazinyl)-Methanone (JNJ-7777120), gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat

Als Inhibitoren von Nicht-Rezeptor Tyrosine Kinasen wie zum Beispiel LYN, LCK, SYK, ZAP-70, FYN, BTK oder ITK gelangen hierbei vorzugsweise Verbindungen zur Anwendung, die ausgewählt sind aus der Gruppe bestehend aus
- 2-[(2-aminoethyl)amino]-4-[(3-bromophenyl)amino]-5-Pyrimidinecarboxamid;
- 2-[[7-(3,4-dimethoxyphenyl)imidazo[1,2-c]pyrimidin-5-yl]amino]-3-Pyridinecarboxamid;
- 6-[[5-fluoro-2-[3,4,5-trimethoxyphenyl)amino]-4-pyrimidinyl]amino]-2,2-dimethyl-2H-Pyrido[3,2-b]-1,4-oxazin-3(4H)-on;
- N-[3-bromo-7-(4-methoxyphenyl)-1,6-naphthyridin-5-yl]-1,3-Propanediamin
- 7-(4-methoxyphenyl)-N-methyl-1,6-Naphthyridin-5-amin;
- N-[7-(4-methoxyphenyl)-1,6-naphthyridin-5-yl]-1,3-Propanediamin;
- N-[7-(2-thienyl)-1,6-naphthyridin-5-yl-1,3-Propanediamin;
- N-[7-[4-(dimethylamino)phenyl]-1,6-naphthyridin-5-yl]-1,2-Ethanediamin;
- N-[7-(4-methoxyphenyl)-2-(trifluoromethyl)-1,6-naphthyridin-5-yl]-1,3-Propanediamin;
- N-[7-(4-methoxyphenyl)-3-phenyl-1,6-naphthyridin-5-yl]-1,3-Propanediamin;
- N-(7-phenyl-1,6-naphthyridin-5-yl)-1,3-Propanediamin;
- N-[7-(3-fluorophenyl)-1,6-naphthyridin-5-yl]-1,3-Propanediamin;
- N-[7-(3-chlorophenyl)-1,6-naphthyridin-5-yl]-1,3-Propanediamin;
- N-[7-[3-(trifluoromethoxy)phenyl]-1,6-naphthyridin-5yl]-1,3-Propanediamin;
- N-[7-(4-fluorophenyl)-1,6-naphthyridin-5-yl]-1,3-Propanediamin;
- N-[7-(4-fluorophenyl)-1,6-naphthyridin-5-yl]-1,3-Propanediamin;
- N-[7-(4-chlorophenyl)-1,6-naphthyridin-5-yl]-1,3-Propanediamin;
- N-[7-(4'-methyl[1,1'-biphenyl]-4-yl)-1,6-naphthyridin-1,3-Propanediamin;
- N-[7-[4-(dimethylamino)phenyl]-1,6-naphthyridin-5-yl]-1,3-Propanediamin;
- N-[7-[4-(diethylamino)phenyl]-1,6-naphthyridin-5-yl]-1,3-Propanediamin;
- N-[7-[4-(4-morpholinyl)phenyl]-1,6-naphthyridin-5-yl]-1,3-Propanediamin;
- N-[7-[4-[[2-(dimethylamino)ethyl]methylamino]phenyl]-1,6-naphthyridin-5-yl]-1,3-Propanediamin;
- N-[7-(4-bromophenyl)-1,6-naphthyridin-5-yl]-1,3-Propanediamin;
- N-[7-(4-methylphenyl)-1,6-naphthyridin-5-yl]-1,3-Propanediamin;
- N-[7-[4-(methylthio)phenyl]-1,6-naphthyridin-5-yl]-1,3-Propanediamin;
- N-[7-[4-(1-methylethyl)phenyl]-1,6-naphthyridin-5-yl]-1,3-Propanediamin;
- 7-[4-(dimethylamino)phenyl]-N-methyl-1,6-Naphthyridin-5-amin;
- 7-[4-(dimethylamino)phenyl]-N,N-dimethyl-1,6-Naphthyridin-5-amin;
- N-[7-[4-(dimethylamino)phenyl]-1,6-naphthyridin-5-yl]-1,4-Butanediamin;
- N-[7-[4-(dimethylamino)phenyl]-1,6-naphthyridin-5-yl]-1,5-Pentanediamin;
- 3-[[7-[4-(dimethylamino)phenyl]-1,6-naphthyridin-5-yl]oxy]-1-Propanol;
- 4-[5-(4-aminobutoxy)-1,6-naphthyridin-7-yl]-N,N-dimethyl-Benzenamin;
- 4-[[7-[4-(dimethylamino)phenyl]-1,6-naphthyridin-5-yl]amino]-1-Butanol;
- N-[7-[4-(dimethylamino)phenyl]-1,6-naphthyridin-5-yl]-N-methyl-1,3-Propanediamin;
- N-[7-[4-(dimethylamino)phenyl]-1,6-naphthyridin-5-yl]-N'-methyl-1,3-Propanediamin;
- N-[7-[4-(dimethylamino)phenyl]-1,6-naphthyridin-5-yl]-N,N'-dimethyl-1,3-Propanediamin;
- 1-amino-3-[[7-[4-(dimethylamino)phenyl]-1,6-naphthyridin-5-yl]amino]-2-Propanol;
- N-[7-[4-(dimethylamino)phenyl]-1,6-naphthyridin-5-yl]-2,2-dimethyl-1,3-Propanediamin;
- 7-[4-(dimethylamino)phenyl]-N-(3-pyridinylmethyl)-1,6-Naphthyridin-5-amin;
- N-[(2-aminophenyl)methyl]-7-[4-(dimethylamino)phenyl]-1,6-Naphthyridin-5-amin;
- N-[7-[6-(dimethylamino)[1,1'-biphenyl]-3-yl]-1,6-naphthyridin-5-yl]-1,3-Propanediamin;
- N-[7-[3-chloro-4-(diethylamino)phenyl]-1,6-naphthyridin-5-yl]-1,3-Propanediamin;
- N-[7-[4-(dimethylamino)-3-methoxyphenyl]-1,6-naphthyridin-5-yl]-1,3-Propanediamin;
- N-[7-[4-(diethylamino)phenyl]-3-methyl-1,6-naphthyridin-5-yl]-1,3-Propanediamin;
- N-[7-(3'-fluoro[1,1'-biphenyl]-3-yl)-1,6-naphthyridin-5-yl]-1,2-Ethanediamin,
- N-[7-(4-methoxyphenyl)-1,6-naphthyridin-5-yl]-1,6-Naphthyridine-1,3-Propanediamin;
- N,N'-bis(3-aminopropyl)-7-(4-methoxyphenyl)-2,5-diamin;
- N-[7-(4-methoxyphenyl)-2-(phenylmethoxy)-1,6-naphthyridin-5-yl]-1,6-Naphthyridine-1,3-Propanediamin;
- N5-(3-aminopropyl)-7-(4-methoxyphenyl)-N2-(phenylmethyl)-2,5-diamin;
- N-[7-(2-naphthalenyl)-1,6-naphthyridin-5-yl]-1,3-Propanediamin;
- N-[7-(2'-fluoro[1,1'-biphenyl]-4-yl)-1,6-naphthyridin-5-yl]-1,3-Propanediamin;
- N-[7-(3,4,5-trimethoxyphenyl)-1,6-naphthyridin-5-yl]-1,3-Propanediamin;
- N-[7-(3,4-dimethylphenyl)-1,6-naphthyridin-5-yl]-1,3-Propanediamin;
- 1-amino-3-[[7-(2-naphthalenyl)-1,6-naphthyridin-5-yl]amino]-2-Propanol;
- 1-amino-3-[[7-(2'-fluoro[1,1'-biphenyl]-4-yl)-1,6-naphthyridin-5-yl]amino]-2-Propanol;
- 1-amino-3-[[7-(4'-methoxy[1,1'-biphenyl]-4-yl)-1,6-naphthyridin-5-yl]amino]-2-Propanol;
- 1-amino-3-[[7-(3,4,5-trimethoxyphenyl)-1,6-naphthyridin-5-yl]amino]-2-Propanol;
- 1-amino-3-[[7-(4-bromophenyl)-1,6-naphthyridin-5-yl]amino]-2-Propanol;
- N-[7-(4'-methoxy[1,1'-biphenyl]-4-yl)-1,6-naphthyridin-5-yl]-2,2-dimethyl-1,3-Propanediamin;
- 1-[[7-[4-(dimethylamino)phenyl]-1,6-naphthyridin-5-yl]amino]-2-Propanol;
- 2-[[2-[[7-[4-(dimethylamino)phenyl]-1,6-naphthyridin-5-yl]amino]ethyl]thio]-Ethanol;
- 7-[4-(dimethylamino)phenyl]-N-(3-methyl-5-isoxazolyl)-1,6-Naphthyridin-5-amin;
- 7-[4-(dimethylamino)phenyl]-N-4-pyrimidinyl-1,6-Naphthyridin-5-amin;
- N-[7-[4-(dimethylamino)phenyl]-1,6-naphthyridin-5-yl]-1,3-Cyclohexanediamin;
- N,N-dimethyl-4-[5-(1-piperazinyl)-1,6-naphthyridin-7-yl]-Benzenamin;
- 4-[5-(2-methoxyethoxy)-1,6-naphthyridin-7-yl]-N,N-dimethyl-Benzenamin;
- 1-[7-[4-(dimethylamino)phenyl]-1,6-naphthyridin-5-yl]-4-Piperidinol;
- 1-[7-[4-(dimethylamino)phenyl]-1,6-naphthyridin-5-yl]-3-Pyrrolidinol;
- 7-[4-(dimethylamino)phenyl]-N-(2-furanylmethyl)-1,6-Naphthyridin-5-amin;
- 7-[4-(dimethylamino)phenyl]-N-[3-(1H-imidazol-1-yl)propyl]-1,6-Naphthyridin-5-amin;
- 1-[7-[4-(dimethylamino)phenyl]-1,6-naphthyridin-5-yl]-4-Piperidinecarboxamid;
- 1-[3-[[7-[4-(dimethylamino)phenyl]-1,6-naphthyridin-5-yl]amino]propyl]-2-Pyrrolidinon;
- N-[3'-[5-[(3-aminopropyl)amino]-1,6-naphthyridin-7-yl][1,1'-biphenyl]-3-yl]-Acetamid;
- N-[7-(4'-fluoro[1,1'-biphenyl]-4-yl)-1,6-naphthyridin-5-yl]-1,3-Propanediamin;
- N-[4'-[5-[(3-aminopropyl)amino]-1,6-naphthyridin-7-yl][1,1'-biphenyl]-3-yl]-Acetamid;
- N-[7-[4-(1,3-benzodioxol-5-yl)phenyl]-1,6-naphthyridin-5-yl]-1,3-Propanediamin;
- N-[7-[4-(2-thienyl)phenyl]-1,6-naphthyridin-5-yl]-1,3-Propanediamin;
- N-[7-[4-fluoro-3-(trifluoromethyl)phenyl]-1,6-naphthyridin-5-yl]-1,3-Propanediamin;
- N-[7-[4-(3-pyridinyl)phenyl]-1,6-naphthyridin-5-yl]-1,3-Propanediamin;
- N-[7-(1,3-benzodioxol-5-yl)-1,6-naphthyridin-5-yl]-1,3-Propanediamin;
- N-[7-(6-methoxy-2-naphthalenyl)-1,6-naphthyridin-5-yl]-1,3-Propanediamin;
- 7-[4-(dimethylamino)phenyl]-N-(4-pyridinylmethyl)-1,6-Naphthyridin-5-amin;
- 3-[[7-[4-(dimethylamino)phenyl]-1,6-naphthyridin-5-yl]methylamino]-Propanenitril;
- 7-[4-(dimethylamino)phenyl]-N-[1-(phenylmethyl)-4-piperidinyl]-1,6-Naphthyridin-5-amin;
- N-[7-[4-(dimethylamino)phenyl]-1,6-naphthyridin-5-yl]-1,2-Cyclohexanediamin,
- N-[7-[4-(dimethylamino)phenyl]-1,6-naphthyridin-5-yl]-1,2-Cyclohexanediamine, (1R,2S)-rel-.
- N-[7-[4-(dimethylamino)phenyl]-1,6-naphthyridin-5-yl]-1,2-Benzenedimethanamine;
- N-[7-[4-(diethylamino)phenyl]-1,6-naphthyridin-5-yl]-1,4-Butanediamine;
- N-[7-[3',5'-bis(trifluoromethyl)[1,1'-biphenyl]-4-yl]-1,6-naphthyridin-5-yl]-,3-Propanediamine;
- N-[7-(3'-methoxy[1,1'-biphenyl]-4-yl)-1,6-naphthyridin-5-yl]-1,3-Propanediamine;
- N-[7-(3'-fluoro[1,1'-biphenyl]-4-yl)-1,6-naphthyridin-5-yl]-1,3-Propanediamine;
- 4-[[7-[4-(dimethylamino)phenyl]-1,6-naphthyridin-5-yl]oxy]-1-Butanol;
- N-[7-[4-(dimethylamino)phenyl]-1,6-naphthyridin-5-yl]-1,4-Cyclohexanediamine;
- 7-[4-(dimethylamino)phenyl]-N-(2,2,6,6-tetramethyl-4-piperidinyl)-1,6-Naphthyridin-5-amine;
- N-[7-[3-bromo-4-(dimethylamino)phenyl]-1,6-naphthyridin-5-yl]-1,3-Propanediamine;
- N-[7-(1-methyl-1H-indol-5-yl)-1,6-naphthyridin-5-yl]-1,3-Propanediamine;
- N-[7-[3-(trifluoromethyl)phenyl]-1,6-naphthyridin-5-yl]-1,3-Propanediamine;
- N-[7-[4-(trifluoromethyl)phenyl]-1,6-naphthyridin-5-yl]-1,3-Propanediamine;
- N-[7-(3-bromo-4-methoxyphenyl)-1,6-naphthyridin-5-yl]-1,3-Propanediamine;
- N-[7-[4-[[3-(dimethylamino)propyl]methylamino]phenyl]-1,6-naphthyridin-5-yl]-1,4-Cyclohexanediamine;
- N-[7-[4-[[2-(dimethylamino)ethyl]methylamino]phenyl]-1,6-naphthyridin-5-yl]-1,4-Cyclohexanediamine;
- N-[7-[4-(dimethylamino)-3-methoxyphenyl]-1,6-naphthyridin-5-yl]-1,4-Cyclohexanediamine;
- N-[7-[4-(4-morpholinyl)phenyl]-1,6-naphthyridin-5-yl]-1,4-Cyclohexanediamine;
- N-[7-[3-bromo-4-(4-morpholinyl)phenyl]-1,6-naphthyridin-5-yl]-1,4-Cyclohexanediamine;
- 4-[[7-[4-[[2-(dimethylamino)ethyl]methylamino]phenyl]-1,6-naphthyridin-5-yl]oxy]-Cyclohexanol;
- N-[7-[3-bromo-4-(4-morpholinyl)phenyl]-1,6-naphthyridin-5-yl]-1,3-Propanediamine;
- N,N-dimethyl-4-[5-(4-methyl-1-piperazinyl)-1,6-naphthyridin-7-yl]-Benzenamine;
- 4-[[7-[4-[[3-(dimethylamino)propyl]methylamino]phenyl]-1,6-naphthyridin-5-yl]oxy]-Cyclohexanol;
- N-[7-[4-[[2-(dimethylamino)ethyl]methylamino]phenyl]-1,6-naphthyridin-5-yl]-1,4-Butanediamin;
- [3-[[5-[(3-aminopropyl)amino]-7-(4-methoxyphenyl)-1,6-naphthyridin-2-yl]amino]propyl]-Carbaminsäure-1,1-dimethylethyl ester,
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat

Als MAP Kinase Inhibitoren gelangen hierbei vorzugsweise Verbindungen zur Anwendung, die ausgewählt sind aus der Gruppe bestehend aus:
- Bentamapimod (AS-602801)
- Doramapimod (BIRB-796),
- 5-Carbamoylindole (SD-169),
- 6-[(aminocarbonyl)(2,6-difluorophenyl)amino]-2-(2,4-difluorophenyl)-3-Pyridinecarboxamide (VX-702),
- alpha-[2-[[2-(3-pyridinyl)ethyl]amino]-4-pyrimidinyl]-2-Benzothiazoleacetonitrile (AS-601245),
- 9,12-Epoxy-1H-diindolo[1,2,3-fg:3',2',1'-kl]pyrrolo[3,4-i][1,6]benzodiazocine-10-Carboxylsäure (CEP-1347),
- 4-[3-(4-chlorophenyl)-5-(1-methyl-4-piperidinyl)-1H-pyrazol-4-yl]-Pyrimidine (SC-409),
gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als iNOS-Inhibitoren gelangen hierbei vorzugsweise Verbindungen zur Anwendung, die ausgewählt sind aus der Gruppe bestehend aus: S-(2-Aminoethyl)isothioharnstoff, Aminoguanidin, 2-Aminomethylpyridin, 5,6-dihydro-6-methyl-4H-1,3-Thiazin-2-amine (AMT), L-Canavanin, 2-Iminopiperidin, S-Isopropylisothioharnstoff, S-Methylisothioharnstoff, S-Ethylisothioharnstoff, S-Methyltiocitrullin, S-Ethylthiocitrullin, L-NA (N^{ω}-Nitro-L-arginin), L-NAME (N^{ω}-Nitro-L-argininmethylester), L-NMMA (N^{ω}-Monomethyl-L-arginin), L-NIO (N^{ω}-Iminoethyl-L-ornithin), L-NIL (N^{ω}-Iminoethyl-lysin), (S)-6-Acetimidoylamino-2-amino-Hexansäure (1*H*-tetrazol-5-yl)-amid (SC-51), N-[[3-(aminomethyl)phenyl]methyl]-Ethanimidamide (1400W), (S)-4-(2-Acetimidoylaminoethylsulfanyl)-2-amino-buttersäure (GW274150), 2-[2-(4-Methoxy-pyridin-2-yl)-ethyl]-3H-imidazo[4,5-b]pyridin (BYK191023), 2-((R)-3-Amino-1-phenyl-propoxy)-4-chlor-5-fluorbenzonitril, 2-((1R,3S)-3-Amino-4-hydroxy-1-thiazol-5-yl-butylsulfanyl)-6-trifluoromethyl-nicotinonitril, 2-((1R,3S)-3-Amino-4-hydroxy-1-thiazol-5-yl-butylsulfanyl)-4-chlor-benzonitril, 2-((1R,3S)-3-Amino-4-hydroxy-1 -thiazol-5-yl-butylsulfanyl)-5-chlorbenzonitril, (2S,4R)-2-Amino-4-(2-chlor-5-trifluoromethyl-phenylsulfanyl)-4-thiazol-5-ylbutan-1-ol, 2-((1R,3S)-3-Amino-4-hydroxy-1-thiazol-5-yl-butylsulfanyl)-5-chlornicotinonitril, 4-((S)-3-Amino-4-hydroxy-1-phenyl-butylsulfanyl)-6-methoxy-nicotinonitril, substituierte 3-Phenyl-3,4-dihydro-1-Isochinolinamin wie z.B. (1S,5S,6R)-7-Chlor-5-methyl-2-aza-bicyclo[4.1.0]hept-2-en-3-ylamin (ONO-1714), (4R,5R)-5-Ethyl-4-methylthiazolidin-2-ylideneamin, (4R,5R)-5-Ethyl-4-methyl-selenazolidin-2-ylideneamin, 4-Aminotetrahydrobiopterin, (E)-3-(4-Chlor-phenyl)-N-(1-{2-oxo-2-[4-(6-trifluormethylpyrimidin-4-yloxy)-piperidin-1-yl]-ethylcarbamoyl}-2-pyridin-2-yl-ethyl)-acrylamid (FR260330), 3-(2,4-Difluor-phenyl)-6-[2-(4-imidazol-1-ylmethyl-phenoxy)-ethoxy]-2-phenyl-pyridin (PPA250), 3-{[(Benzo[1,3]dioxol-5-ylmethyl)-carbamoyl]-methyl}-4-(2-imidazol-1-yl-pyrimidin-4-yl)-piperazin-1-carbonsäuremethylester (BBS-1), (R)-1-(2-Imidazol-1-yl-6-methyl-pyrimidin-4-yl)-pyrrolidin-2-carbonsäure (2-benzo[1,3]dioxol-5-ylethyl)-amid (BBS-2) und deren pharmazeutischen Salze, Prodrugs oder Solvate.

Als iNOS-Inhibitoren im Rahmen der vorliegenden Erfindung können weiterhin antisense-Oligonucleotide, insbesondere solche antisense-Oligonucleotide, die iNOS-kodierende Nukleinsäuren binden, eingesetzt werden. Z.B. werden in WO 01/52902 antisense-Oligonucleotide, insbesondere antisense-Oligonucleotide, die iNOS kodierende Nukleinsäuren binden, zur Modulierung der Expression von iNOS beschrieben.

Als MRP4-Inhibitoren gelangen hierbei vorzugsweise Verbindungen zur Anwendung, die ausgewählt sind aus der Gruppe bestehend aus *N*-Acetyl-dinitrophenyl-Cysteine, cGMP, Cholate, Diclofenac, Dehydroepiandrosterone 3-glucuronide, Dehydroepiandrosterone 3-sulphate, Dilazep, Dinitrophenyl-S-glutathione, Estradiol 17-beta-glucuronide, Estradiol 3,17-disulphate, Estradiol 3-glucuronide, Estradiol 3-sulphate, Estrone 3-sulphate, Flurbiprofen, Folate, N5-formyl-tetrahydrofolate, Glykocholat, Glykolithocholsäure sulfat,, Ibuprofen, Indomethacin, Indoprofen, Ketoprofen, Lithocholsäuresulfat sulphat, Methotrexat, ((*E*)-3-[[[3-[2-(7-Chloro-2-chinolinyl)ethenyl]phenyl]-[[3-dimethylamino)-3-oxopropyl]thio]methyl]thio]-propansäure), alpha-Naphthyl-beta-D-glucuronid, Nitrobenzyl mercaptopurine ribosid, Probenecid, Sildenafil, Sulfinpyrazone, Taurochenodeoxycholate, Taurocholat, Taurodeoxycholat, Taurolithocholat, Topotecan, Trequinsin, Zaprinast und Dipyridamol, gegebenenfalls in Form ihrer Racemate, Enantiomere, Diastereomere und ihrer pharmakologisch verträglichen Säure-Additionssalze und Hydrate.

Als Leukotriene Biosynthese Inhibitoren wie zum Beispiel aus der Gruppe der 5-Lipoxygenase (5-LO) Inhibitoren, cPLA2 Inhibitoren, Leukotriene A4 Hydrolase Inhibitoren oder FLAP Inhibitoren, gelangen hierbei vorzugsweise Verbindungen zur Anwendung, die ausgewählt sind aus der Gruppe bestehend aus Zileuton, Tipelukast, Licofelone, Darapladib,
gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Nicht-steroidale antientzündliche Agenzien (NSAIDs) gelangen hierbei vorzugsweise Verbindungen zur Anwendung, die ausgewählt sind aus der Gruppe bestehend aus Piroxicam, Diclofenac, Naproxen, Flurbiprofen, Fenoprofen, Ketoprofen, Ibuprofen, Nimesulide, Indomethacin, Sulindac, Azapropazone, Phenylbutazone, Aspirin; Meloxicam, Celecoxib, Rofecoxib, Valdecoxib, Lumarocoxib, Parecoxib, Tenoxicam und Etoricoxib, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als CRTH2 Antagonisten gelangen hierbei vorzugsweise Verbindungen zur Anwendung, die ausgewählt sind aus der Gruppe bestehend aus Ramatroban und Laropiprant, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als DP1-Rezeptor Modulatoren gelangen hierbei vorzugsweise Verbindungen zur Anwendung, die ausgewählt sind aus der Gruppe bestehend aus 7-[(1R,2R,3S,5S)-2-[[(5-hydroxybenzo[b]thien-3-yl)carbonyl]amino]-6,6-dimethylbicyclo[3.1.1]hept-3-yl]-, (5Z)-5-Heptensäure (S-5751), Laropiprant, und 2-[[4-[(1R,2S,3R,5R)-5-chloro-2-[(3S)-3-cyclohexyl-3-hydroxy-1-propyn-1-yl]-3-hydroxycyclopentyl]butyl]thio]-Essigsäure (TS-002), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Thromboxane Rezeptor Antagonist gelangen hierbei vorzugsweise Verbindungen zur Anwendung, die ausgewählt sind aus der Gruppe bestehend aus Seratrodast, N-[[(1,1-dimethylethyl)amino]carbonyl]-2-[(4-methylphenyl)amino]-5-nitro-Benzenesulfonamide (BM-573), (+/-)-sodium[2-(4-chlorophenylsulfonylaminomethyl)-indan-5-yl]acetate monohydrate (Z-335) und 2-[[[4-[[(4-chlorophenyl)sulfonyl]amino]butyl][[3-[[4-(1-methylethyl)-2-thiazolyl]methoxy]phenyl]methyl]amino]sulfonyl]-Benzoesäure (KP-496) gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Chemokine Rezeptor Antagonisten gelangen hierbei vorzugsweise Verbindungen zur Anwendung, die ausgewählt sind aus der Gruppe bestehend aus N-[5-chloro-2-[2-[(2R)-4-[(4-fluorophenyl)methyl]-2-methyl-1-piperazinyl]-2-oxoethoxy]phenyl]-Harnstoff hydrochloride (1:1) (BX-471), 2, N-[(1S,2S,4R)-4-(aminocarbonyl)-1-[(3-fluorophenyl)methyl]-2,7-dihydroxy-7-methyloctyl]- -Quinoxalinecarboxamide (CP-481715), (4,6-dimethyl-5-pyrimidinyl)[4-[(3S)-4-[(1R)-2-methoxy-1-[4-(trifluoromethyl)phenyl]ethyl]-3-methyl-1-piperazinyl]-4-methyl-1-piperidinyl]-Methanone (Sch-417690), 2-hydroxy-N,N-dimethyl-3-[[2-[[(1R)-1-(5-methyl-2-furanyl)propyl]amino]-3,4-dioxo-1-cyclobuten-1-yl]amino]-Benzamide (SCH-527123) und 1,4,8,11-Tetraazacyclotetradecane, 1,1'-[1,4-phenylenebis(methylene)]bis-, hydrochloride (1:8) (AMD-3100), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Neurokinin (NK1 oder NK2) Antagonisten gelangen hierbei vorzugsweise Verbindungen zur Anwendung, die ausgewählt sind aus der Gruppe bestehend aus: Saredutant, Nepadutant und Figopitant gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Sphingosine1-phosphat Rezeptor Modulatoren gelangen hierbei vorzugsweise Verbindungen zur Anwendung wie z.B. sonepcizumab, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Mucoregulatoren gelangen hierbei vorzugsweise Verbindungen zur Anwendung, die ausgewählt sind aus der Gruppe bestehend aus: 3-[2-oxo-2-[2-[[3-(trifluoromethyl)phenyl]amino]-3-pyridinyl]ethyl]-1(3H)-Isobenzofuranone (MSI-2216), Erdosteine, Fluorovent, Talniflumate, fudosteine, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als PPAR gamma Agonist gelangen hierbei vorzugsweise Verbindungen zur Anwendung, die ausgewählt sind aus der Gruppe bestehend aus: Rosiglitazone, Ciglitazone, Pioglitazone und N-[2-[2-[(3-fluorophenyl)imino]-4-[4-(4-morpholinyl)phenyl]-3(2H)-thiazolyl]ethyl]-N'-methyl-Harnstoff (SMP-028), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Rho Kinase Inhibitoren gelangen hierbei vorzugsweise Verbindungen zur Anwendung, wie z.B. Fasudil, gegebenenfalls in Form seiner Racemate, Enantiomere, Diastereomere und gegebenenfalls in Form seiner pharmakologisch verträglichen Säureadditionssalze, Prodrugs, Solvate oder Hydrate.

Als Adenosine Rezeptor Modulatoren gelangen hierbei vorzugsweise Verbindungen zur Anwendung, die ausgewählt sind aus der Gruppe bestehend aus 4-(3,4-dichlorophenyl)-5-(4-pyridinyl)-2-Thiazolamine (CGH-2466), 3-ethyl-3,9-dihydro-1-propyl-8-[1-[[3-(trifluoromethyl)phenyl]methyl]-1H-pyrazol-4-yl]-1H-Purine-2,6-dione (CVT-6883), N-(4-cyanophenyl)-2-[4-(2,3,6,9-tetrahydro-2,6-dioxo-1,3-dipropyl-1H-purin-8-yl)phenoxy]-Acetamide (MRS-1754), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Bradykinin Rezeptor Antagonisten gelangen hierbei vorzugsweise Verbindungen zur Anwendung, die ausgewählt sind aus der Gruppe bestehend aus Icatibant und 1-Piperazinepentanaminium, delta-amino-4-[[4-[[[2,4-dichloro-3-[[(2,4-dimethyl-8-chinolinyl)oxy]methyl]phenyl]sulfonyl]amino]tetrahydro-2H-pyran-4-yl]carbonyl]-N,N,N-trimethyl-ε-oxo-, chloride, hydrochloride (1:1:1), (deltaS)- (MEN-16132), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Endothelin Antagonisten gelangen hierbei vorzugsweise Verbindungen zur Anwendung, die ausgewählt sind aus der Gruppe bestehend aus Actelion-1, Ambrisentan, Sitaxsentan, N-(2-acetyl-4,6-dimethylphenyl)-3-[[(4-chloro-3-methyl-5-isoxazolyl)amino]sulfonyl]-2-Thiophenecarboxamid (TBC-3214) und Bosentan, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Interleukin 1-beta converting enzyme (ICE) Inhibitoren gelangen hierbei vorzugsweise Verbindungen zur Anwendung, die ausgewählt sind aus der Gruppe bestehend aus Pralnacasan und N-(4-amino-3-chlorobenzoyl)-3-methyl-L-valyl-N-[(2R,3S)-2-ethoxytetrahydro-5-oxo-3-furanyl]-L-Prolinamid (=VX-765), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Toll-like Rezeptor (TLR) Modulatoren gelangen hierbei vorzugsweise Verbindungen zur Anwendung, die ausgewählt sind aus der Gruppe bestehend aus Resiquimod, Heplisav, Resatorvid (TAK-242), gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als HMG-CoA Reductase Inhibitoren gelangen hierbei vorzugsweise Verbindungen zur Anwendung, die ausgewählt sind aus der Gruppe bestehend aus Cerivastatin, Pitavastatin, Rosuvastatin, Lovastatin, Simvastatin, Pravastatin, Fluvastatin und Avorvastatin, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als VLA-4 Antagonisten gelangen hierbei vorzugsweise Verbindungen zur Anwendung, die ausgewählt sind aus der Gruppe bestehend aus Natalizumab, valategrast, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als SHIP Agonisten gelangen hierbei vorzugsweise Verbindungen zur Anwendung, die ausgewählt sind aus der Gruppe bestehend aus 2,3,4,4a,5,6,6a,11,11a,11b-decahydro-4,4,6a,7,11b-pentamethyl-, (4aS,6aR,11aR,11bS)-1H-Benzo[a]fluoren-9-ol (AQX-MN100) und MN-106, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als anti-TNF-Antikörper gelangen hierbei vorzugsweise Verbindungen zur Anwendung, die ausgewählt sind aus der Gruppe bestehend aus Infliximab, Adalimumab, Golimumab. CytoFab und Etanercept.

Als Substanzen gegen Schwellungen der Atemwege gelangen hierbei vorzugsweise Verbindungen zur Anwendung, die ausgewählt sind aus der Gruppe bestehend aus Phenylephrine, Phenylpropanolamine, Pseudophedrine, Oxymetazoline, Epinephrine, Naphazoline, Xylometazoline, Propylhexedrine und Llevo-desoxyephedrine, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen gegen Husten gelangen hierbei vorzugsweise Verbindungen zur Anwendung, die ausgewählt sind aus der Gruppe bestehend aus Hydrocodone, Caramiphen, Carbetapentane und Dextramethorphan, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter Lipoxin A4 Derivative gelangen hierbei vorzugsweise Verbindungen zur Anwendung, die ausgewählt sind aus der Gruppe bestehend aus
- 7,9,11,13-Eicosatetraensäure, 5,6,15-trihydroxy-, (5S,6R,7E,9E,11Z,13E,15R)- (15-epi-lipoxin a4),
- 7,9,11,13-Eicosatetraensäure, 16-(4-fluorophenoxy)-5,6,15-trihydroxy-, (5S,6R,7E,9E,11Z,13E,15S)- (ATL-1),
- Aspirin-getriggeres Lipoxin A(4) and Analoge,
- protectin D1 (4,7,11,13,15,19-Docosahexaensäure, 10,17-dihydroxy-, (4Z,7Z,10R,11E,13E,15Z,17S,19Z)- ,
- Resolvin E1 (6,8,10,14,16-Eicosapentaensäure, 5,12,18-trihydroxy-, (5S,6Z,8E,10E,12R,14Z,16E,18R)-) and
- Benzo-Lipoxin A4 Analoge,
gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter FPRL1-Modulatoren gelangen hierbei vorzugsweise Verbindungen zur Anwendung, wie z.B. 5(S),6(R), 7-trihydroxyheptanonsäure-Methyl-ester, gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter PI3 Kinase Antagonisten gelangen hierbei vorzugsweise Verbindungen zur Anwendung, die ausgewählt sind aus der Gruppe bestehend aus
- 5-(Quinoxalin-6-ylmethylene)thiazolidine-2,4-dione (AS-605240),
- 2-[(6-amino-9H-purin-9-yl)methyl]-5-methyl-3-(2-methylphenyl)-4(3H)-Quinazolinone (C-87114),
- 2-Methyl-2-[4-[3-methyl-2-oxo-8-(chinolin-3-yl)-2,3-dihydroimidazo[4,5-c]chinolin-1-yl]phenyl]propionitrile (BEZ-235),
gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter CCR5 Antagonisten gelangen hierbei vorzugsweise Verbindungen zur Anwendung, die ausgewählt sind aus der Gruppe bestehend aus
- Maraviroc (4,4-difluoro-N-[(1S)-3-[(3-exo)-3-[3-methyl-5-(1-methylethyl)-4H-1,2,4-triazol-4-yl]-8-azabicyclo[3.2.1]oct-8-yl]-1-phenylpropyl]-Cyclohexancarboxamid), CCR5mAb004
- Vicriviroc ((4,6-dimethyl-5-pyrimidinyl)[4-[(3S)-4-[(1R)-2-methoxy-1-[4-(trifluoromethyl)phenyl]ethyl]-3-methyl-1-piperazinyl]-4-methyl-1-piperidinyl]-Methanon) und
- Nifeviroc (N-[1-[[(3S, 4R)-1-(cyclopentylcarbonyl)-4-hydroxy-4-phenyl-3-pyrrolidinyl]methyl]-4-piperidinyl]-N-2-propen-1-yl-(4-nitrophenyl)methyl ester-Carbaminsäure),
gegebenenfalls in Form ihrer Racemate, Enantiomere, Diastereomere und gegebenenfalls in Form ihrer pharmakologisch verträglichen Säureadditionssalze, Prodrugs, Solvate oder Hydrate.

Als Substanzen bevorzugter CXCR1 or CXCR2 Antagonisten gelangen hierbei vorzugsweise Verbindungen zur Anwendung, wie z.B. 3-[[3-[(Dimethylamino)carbonyl]-2-hydroxyphenyl]amino]-4-[[(R)-1-(5-methylfuran-2-yl)propyl]amino]cyclobut-3-ene-1,2-dione (SCH-527123),
gegebenenfalls in Form seiner Racemate, Enantiomere, Diastereomere und gegebenenfalls in Form seiner pharmakologisch verträglichen Säureadditionssalze, Prodrugs, Solvate oder Hydrate.

Erfindungsgemäß bevorzugt sind die Säureadditionssalze der oben genannten MAP Kinase Inhibitoren, iNOS-lnhibitoren, MRP4-Inhibitoren, Leukotriene Biosynthese Inhibitoren, Nicht-steroidalen antientzündlichen Agenzien (NSAIDs), CRTH2 Antagonisten, DP1-Rezeptor Modulatoren, Thromboxane Rezeptor Antagonisten, Chemokine Rezeptor Antagonisten, Neurokinin (NK1 oder NK2) Antagonisten, Sphingosine1-phosphat Rezeptor Modulatoren, Mucoregulatoren, PPAR gamma Agonisten, Rho Kinase Inhibitoren, Adenosine Rezeptor Modulatoren, Bradykinin Rezeptor Antagonisten, Endothelin Antagonisten, Interleukin 1-beta converting enzyme (ICE) Inhibitoren, Toll-like Rezeptoren (TLR) Modulatoren, HMG-CoA Reductase Inhibitoren, VLA-4 Antagonisten, SHIP Agonisten, anti-TNF-Antikörper, Substanzen gegen Schwellungen der Atemwege, Substanzen gegen Husten, Lipoxin A4 Derivative, PI3 Kinase Antagonisten, FPRL1-Modulatoren, CCR5 Antagonisten, CXCR1- oder CXCR2-Antagonisten ebenfalls ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

### Bezugszeichenliste

- 1: Kapselkappe
- 2: Kapselkörper
- 3: Sicke (an Kapselkappe)
- 4: Sicke (an Kapselkappe)
- 5: Wulst (an Kapselkörper)
- 6, 6a, 6b: Loch (an Kapselkappe)
- 7, 7a, 7b: Loch (an Kapselkörper)
- 8: Steg (an Kapselkörper)
- 9: Nut (an Kapselkappe)
- 11: Kapsel in erster Einsteckstellung (Löcher verdeckt)
- 12: Kapsel in zweiter Einsteckstellung (Löcher geöffnet)
- 13: Kapselkammer
- 13a: Längswand (der Kapselkammer)
- 14: Schieber
- 14a: eingeschobene Längswand (am Schieber)
- 14b: Vorsprung (am Schieber)
- 15: Sieb
- 16: Schacht
- 17: Kanal
- 18: Führungsschiene
- 19: Feder
- 20: Lufteinlassöffnung
- 21: Anschlagkante
- 22: Ring
- 22a: Wulst (an Ring)
- 23: Aufnahme für Ring
- 23a: Rastelement (an Aufnahme für Ring)

- 31: Kapsel (oben offen)
- 32: Kammer
- 32a: Oberseite (der Kammer)
- 32b: Füllkammer
- 33: Einlassloch
- 34: Pfeil (gibt Befüllrichtung an)
- 35: Sieb
- 36: Pfeil (Luftströmung in Richtung Patient)
- 37: Pfeil (Vibrationsrichtung)
- 38: Pfeil (Luftströmung)
- 39: Backe
- 40: Pulver
- 41: Loch (axial unten, in oben offener Kapsel)
- 42: Loch (seitlich unten in halbkugelförmigem Bereich, in oben offener Kapsel)
- 42a: Loch (seitlich unten an geschlossener Kapsel)
- 42b: Loch (seitlich oben an geschlossener Kapsel)
- 43: offenes Ende (bei oben offener Kapsel)
- 44: unteres Ende (bei oben offener Kapsel)
- 45: Loch (seitlich unten, in oben offener Kapsel)
- 46: Loch (in oben offener Kapsel)

- 50: Magazin
- 51: Träger
- 52: Aufnahme (in Magazin)
- 52a: Absatz (in Aufnahme)
- 53: Einsatz
- 53a: Absatz (an Einsatz)
- 53b: Spitze (an Einsatz)
- 55: Siegel
- 56: Stempel
- 57: Stempelring

- 60: Gerät
- 62: Röhre
- 63: flaches Bauteil
- 64: Schieber mit Einlass
- 65: Vibrationskammer
- 66: Stab
- 67: Mundstück
- 68: Pfeil (Luftströmung am Einlass)
- 69: Pfeil (Ansaugrichtung)
- 70: Gerät
- 71: Kapsel (geschlossen)
- 71a,b: halbkugelförmige Enden (der geschlossenen Kapsel)
- 72, 72a, 72b: vorgefertigte Löcher
- 73: Röhre
- 73a: Kragen (an Röhre)
- 73b: Sicke (außen am Kragen der Röhre)
- 73d: Schlitz (an Röhre)
- 73e: Griffhilfe (an Röhre)
- 73f: Federarm (an Röhre)
- 73g: Abstand (zwischen Röhren)
- 73k: Abschlussbereich
- 73s: Ausnehmung (in Wand der Röhre)
- 74: Kapselkammer
- 75: Stab
- 76: Einlass
- 77: Schieber
- 77a: Verjüngung (am Schieber)
- 77b: Federarm (am Schieber)
- 78: Mundstück
- 78b: Wulst (am unteren Innenrand des Mundstücks)
- 78c: Grifffläche (am Mundstück)
- 78d: Mundrohr (im Mundstück)
- 78e: Mittelwand (im Mundstück)
- 91: Pfeil (in Strömungsrichtung der Luft am Einlass)
- 92: Pfeil (in Einatmungsrichtung)

## Patentansprüche

1. System aus einem Inhalator und einer Kapsel (11, 71), die eine pharmazeutische, vorzugsweise pulverförmige Zubereitung enthält,
wobei die Kapsel zwei einseitig offene Kapselelemente, nämlich einen Kapselkörper (2) und eine Kapselkappe (1), umfasst, die teleskopartig über ihre Öffnungen ineinander gesteckt sind, so dass ein Hohlraum gebildet wird, und
wobei der Inhalator eine Kapselkammer (13, 74) zur Aufnahme einer Kapsel (11, 71) aufweist, wobei die Kapselkammer (13, 74) einen Lufteinlass und einen in Richtung eines Mundstücks (78) führenden Luftauslass aufweist, und die Kapsel (11, 71) aus einer Kapselaufnahme in die Kapselkammer (13, 74) eingeführt werden kann, **dadurch gekennzeichnet, dass**
mindestens eines der beiden Kapselelemente zusätzlich zur einseitigen Öffnung mindestens ein vorgefertigtes Loch (7, 6) aufweist und
der Inhalator einen Schieber (14, 77) aufweist, bei dessen Betätigung die Kapsel (11,71) und die Kapselaufnahme, aus der die Kapsel (11,71) in die Kapselkammer (13, 74) eingeführt wird, relativ so zueinander verschoben werden, dass an der Kapsel (11, 71) mindestens ein vorgefertigtes Loch (6, 7, 72a, 72b) freigegeben wird.

2. System aus einem Inhalator und mindestens einer Kapsel nach Anspruch 1 **dadurch gekennzeichnet, dass**
- die Kapsel (11, 71) vor Anwendung des Inhalators in einer zum Inhalator gehörenden Kapselaufnahme bevorratet ist, wobei die Kapselaufnahme mindestens ein Loch (72a, 72b) abdichtet, das nach dem Ineinanderstecken der Kapselelemente in den Hohlraum der Kapsel (11, 71) führt, und/oder alle Löcher (72a, 72b) abdichtet, die nach dem Ineinanderstecken der Kapselelemente in den Hohlraum der Kapsel (11, 71) führen, und
- mit dem Schieber (14, 77) die Kapsel (11, 71) aus der Kapselaufnahme in die Kapselkammer (13, 74) verschoben werden kann.

3. System aus Inhalator und Kapsel (11, 71) nach Anspruch 2, **dadurch gekennzeichnet, dass** der Schieber (14, 77) nach Verschieben der Kapsel (11, 71) in die Kapselkammer (13, 74) einen Wandbereich der Kapselkammer (13, 74) bildet und/oder dass im Schieber (14, 77) ein Lufteinlass für die Kapselkammer (13, 74) ausgebildet ist.

4. System aus Inhalator und Kapsel nach einem der Ansprüche 2 oder 3 **dadurch gekennzeichnet, dass** zwei Kapseln (11, 71) zum System gehören und der Inhalator zwei Kapselkammern (13, 74) und zwei Kapselaufnahmen aufweist.

5. System aus Inhalator und zwei Kapseln nach Anspruch 4 **dadurch gekennzeichnet, dass** die beiden Kapseln mit verschiedenen Formulierungen und/oder verschiedenen Formulierungsmengen befüllt sind.

6. System aus einem Inhalator und einer Kapsel (11, 71), die eine pharmazeutische, vorzugsweise pulverförmige Zubereitung enthält,
wobei die Kapsel zwei einseitig offene Kapselelemente, nämlich einen Kapselkörper (2) und eine Kapselkappe (1), umfasst, die teleskopartig über ihre Öffnungen ineinander gesteckt sind, so dass ein Hohlraum gebildet wird, und
wobei der Inhalator eine Kapselkammer (13, 74) zur Aufnahme einer Kapsel (11, 71) aufweist, wobei die Kapselkammer (13, 74) einen Lufteinlass und einen in Richtung eines Mundstücks (78) führenden Luftauslass aufweist, und die Kapsel (11, 71) in die Kapselkammer (13, 74) eingesetzt ist, **dadurch gekennzeichnet, dass** mindestens eines der beiden Kapselelemente zusätzlich zur einseitigen Öffnung mindestens ein vorgefertigtes Loch (7, 6) aufweist und
der Inhalator einen Schieber (14, 77) aufweist, bei dessen Betätigung die Kapsel (11,71) und ein ringförmiges Bauteil, relativ so zueinander verschoben werden, dass an der Kapsel (11, 71) mindestens ein vorgefertigtes Loch (6, 7, 72a, 72b) freigegeben wird.

7. System nach Anspruch 6, **dadurch gekennzeichnet, dass** die zusammengesetzte Kapsel neben dem Kapselkörper (2) und der Kapselkappe (1) das ringförmiges Bauteil aufweist, mittels dem mindestens ein Loch (6, 7, 72a, 72b) abgedichtet wird, das nach dem Ineinanderstecken von Kapselkörper (2) und Kapselkappe (1) in den Hohlraum der Kapsel (12) führt, und/oder mittels dem alle Löcher abgedichtet werden, die nach dem Ineinanderstecken von Kapselkörper (2) und Kapselkappe (1) in den Hohlraum der Kapsel führen, und dass mit dem Schieber (14, 77) das ringförmige Bauteil von der Kapsel (11, 71) getrennt werden kann, so dass das mindestens eine vorgefertigtes Loch (6, 7, 72a, 72b) an der Kapsel (11, 71) freigelegt wird.

8. System aus einem Inhalator und mindestens einer Kapsel (11, 71) nach Anspruch 7 **dadurch gekennzeichnet, dass** nach Betätigung des Schiebers (14, 77) das ringförmige Bauteil eine Abdichtung zwischen Schieber (14, 77) und Kapselaufnahme und/oder zwischen Schieber (14,77) und Kapselkammer (13, 74) bildet.

9. System nach einem der vorherigen Ansprüche, **dadurch gekennzeichnet, dass** die Kapselelemente aus einem Kunststoffmaterial, vorzugsweise beide aus dem gleichen Material und insbesondere aus Polyethylen, Polycarbonat, Polyester, Polypropylen oder Polyethylenterrephthalat, in einem Spritzgussverfahren gefertigt sind und die vorgefertigten Löcher (6, 7) bereits im gleichen Spritzgussverfahren ausgebildet worden sind.

10. System aus Inhalator und Kapsel (11, 71) nach einem der vorherigen Ansprüche **dadurch gekennzeichnet, dass** der Schieber (14, 77) mindestens ein Rastelement, insbesondere mindestens einen Federarm (77b), aufweist, das bei insbesondere vollständiger Betätigung des Schiebers (14, 77) derart mit einem anderen Bauteil des Inhalators verrastet, dass der Schieber (14, 77) nicht zerstörungsfrei in seine Ausgangslage zurückgeschoben werden kann.

11. Verfahren zur Bereitstellung eines Systems aus Inhalator und Kapsel nach einem der Ansprüche 2 oder 3 **dadurch gekennzeichnet, dass** zunächst ein einseitig offenes Kapselelement - der Kapselkörper (2) - in eine Kapselaufnahme eingesetzt wird, der Kapselkörper (2) mit einer abgemessenen Menge vorzugsweise pulverförmiger pharmazeutischer Zubereitung befüllt wird, der Kapselkörper anschließend mit einer Kapselkappe (1) verschlossen wird und die Kapselaufnahme mit den anderen Bauteilen des Inhalators zusammengesetzt wird.

12. Verfahren zur Bereitstellung eines Systems aus Inhalator und Kapsel nach einem der Ansprüche 7 oder 8 **dadurch gekennzeichnet, dass** zunächst ein einseitig offenes Kapselelement - der Kapselkörper (2) - in ein ringförmiges Bauteil eingesetzt wird, der Kapselkörper (2) mit einer abgemessenen Menge vorzugsweise pulverförmiger pharmazeutischer Zubereitung befüllt wird, der Kapselkörper anschließend mit einer Kapselkappe (1) verschlossen wird und das ringförmige Bauteil mit den anderen Bauteilen des Inhalators zusammengesetzt wird.

13. Verfahren zur Bereitstellung eines Systems aus Inhalator und Kapsel nach Anspruch 11 oder 12 **dadurch gekennzeichnet, dass** nach Zusammensetzen der Bauteile des Inhalators das System in einem vorzugsweise für Transport oder Lagerung geeigneten Zustand vorliegt, in dem die Löcher der von Kapselkörper (2) und Kapselkappe (1) gebildeten Kapsel (11, 71) verschlossen sind, und dass das System durch Schieben oder Ziehen an einem Bauteil des Inhalators oder des Systems in einen anwendungsbereiten Zustand überführt wird, wobei durch dieses Schieben oder Ziehen die Löcher der Kapsel freigegeben werden und/oder die Kapsel (11, 71) in eine Kapselkammer (13, 74) verschoben wird.

## Claims

1. System comprising an inhaler and a capsule (11, 71) which comprises a preferably powdered pharmaceutical preparation,
wherein the capsule comprises two capsule elements open at one end, namely a capsule body (2) and a capsule cap (1), which can be fitted into one another telescopically through their openings, so as to form a cavity, and
wherein the inhaler comprises a capsule chamber (13, 74) for receiving a capsule (11, 71), wherein the capsule chamber (13, 74) comprises an air inlet and an air outlet leading towards a mouthpiece (78) and the capsule (11, 71) can be inserted in the capsule chamber (13, 74) from a capsule receptacle,
**characterised in that**
at least one of the two capsule elements comprises at least one prefabricated hole (7, 6) in addition to the opening at one end and
the inhaler comprises a pusher (14, 77), which when actuated causes the capsule (11, 71) and the capsule receptacle, from which the capsule is inserted into the capsule chamber (13,74), to be moved relative to each other, such that at least one prefabricated hole (6, 7, 72a, 72b) is exposed on the capsule (11,71).

2. System comprising an inhaler and at least one capsule according to claim 1, **characterised in that**
- before the inhaler is used, the capsule (11, 71) is stored in a capsule receptacle belonging to the inhaler, the capsule receptacle sealing off at least one hole (72a, 72b) which leads into the cavity of the capsule (11, 71) after the capsule elements have been fitted into one another, and/or sealing off all the holes (72a, 72b) that lead into the cavity of the capsule (11, 71) after the capsule elements have been fitted into one another, and
- the capsule (11, 71) can be moved with the pusher (14, 77) out of the capsule receptacle into the capsule chamber (13, 74).

3. System comprising an inhaler and capsule (11,71) according to claim 2, **characterised in that** after the capsule (11, 71) has been moved into the capsule chamber (13, 74) the pusher (14, 77) forms a wall region of the capsule chamber (13, 74) and/or that an air inlet for the capsule chamber (13, 74) is formed in the pusher (14, 77).

4. System comprising an inhaler and a capsule according to one of claims 2 or 3, **characterised in that** two capsules (11, 71) belong to the system and the inhaler comprises two capsule chambers (13, 74) and two capsule receptacles.

5. System comprising an inhaler and two capsules according to claim 4, **characterised in that** the two capsules are filled with different formulations and/or different amounts of formulation.

6. System from an inhaler and a capsule (11, 71) which comprises a preferably powdered pharmaceutical preparation,
wherein the capsule comprises two capsule elements open at one end, namely a capsule body (2) and a capsule cap (1), which can be fitted into one another telescopically through their openings, so as to form a cavity, and
wherein the inhaler comprises a capsule chamber (13, 74) for receiving a capsule (11, 71), wherein the capsule chamber (13, 74) comprises an air inlet and an air outlet leading towards a mouthpiece (78) and the capsule (11, 71) is inserted in the capsule chamber (13, 74),
**characterised in that**
at least one of the two capsule elements comprises at least one prefabricated hole (7, 6) in addition to the opening at one end and
the inhaler comprises a pusher (14, 77), which when actuated causes the capsule (11, 71) and an annular component to be moved relative to each other, such that at least one prefabricated hole (6, 7, 72a, 72b) is exposed on the capsule (11, 71).

7. System according to claim 6, **characterised in that**
the assembled capsule comprises in addition to the capsule body (2) and the capsule cap (1) the annular component, by means of which at least one hole (6, 7, 72a, 72b) is sealed off which leads into the cavity of the capsule (12) after the fitting together of the capsule body (2) and capsule cap (1), and/or by means of which all the holes are sealed off which lead into the cavity of the capsule after the fitting together of the capsule body (2) and capsule cap (1), and
that with the pusher (14, 77) the annular component can be separated from the capsule (11, 71), so that the at least one prefabricated hole (6, 7, 72a, 72b) on the capsule (11, 71) is exposed.

8. System comprising an inhaler and at least one capsule (11, 71) according to claim 7, **characterised in that** after actuation of the pusher (14, 77) the annular component forms a seal between the pusher (14, 77) and the capsule receptacle and/or between the pusher (14,77) and the capsule chamber (13, 74).

9. System according to one of the preceding claims, **characterised in that** the capsule elements are made from a plastics material, preferably both from the same material and, in particular, from polyethylene, polycarbonate, polyester, polypropylene or polyethylene terephthalate, in an injection moulding process and the prefabricated holes (6, 7) have already been formed in the same injection moulding process.

10. System comprising an inhaler and capsule (11, 71) according to one of the preceding claims, **characterised in that** the pusher (14, 77) comprises at least one latching element - particularly at least one spring arm (77b) - which, particularly when the pusher (14, 77) is fully actuated, latches with another component of the inhaler such that the pusher (14, 77) cannot be non-destructively pushed back into its original position.

11. Method for producing a system comprising an inhaler and capsule according to one of claims 2 or 3, **characterised in that** first of all a capsule element - the capsule body (2) - open at one end is inserted in a capsule receptacle, the capsule body (2) is filled with a measured amount of preferably powdered pharmaceutical preparation, the capsule body is then closed off with a capsule cap (1) and the capsule receptacle is assembled with the other components of the inhaler.

12. Method for producing a system comprising an inhaler and capsule according to one of claims 7 or 8, **characterised in that** first of all a capsule element - the capsule body (2) - open at one end is inserted in an annular component, the capsule body (2) is filled with a measured amount of preferably powdered pharmaceutical preparation, the capsule body is then closed off with a capsule cap (1) and the annular component is assembled with the other components of the inhaler.

13. Method for producing a system comprising an inhaler and capsule according to claim 11 or 12, **characterised in that** after the assembly of the components of the inhaler the system is in a state which is preferably suitable for transporting or storage and in which the holes of the capsule (11, 71) formed by the capsule body (2) and capsule cap (1) are closed off, and that by pushing or pulling a component of the inhaler or system, the system is converted into a state ready for use, the holes of the capsule being exposed by this pushing or pulling and/or the capsule (11, 71) being moved into a capsule chamber (13, 74).

## Revendications

1. Système composé d'un inhalateur et d'une capsule (11, 71) contenant une préparation pharmaceutique, de préférence sous forme de poudre,
dans lequel la capsule comprend deux éléments de capsule ouverts d'un côté, à savoir un corps de capsule (2) et un capuchon de capsule (1), qui sont emboîtés de manière télescopique l'un dans l'autre par leurs ouvertures de sorte qu'une cavité est formée, et
dans lequel l'inhalateur comprend une chambre de capsule (13, 74) pour la réception d'une capsule (11, 71), dans lequel la chambre de capsule (13, 74) comprend une entrée d'air et une sortie d'air qui mène en direction d'un embout (78), et la capsule (11, 71) peut être extraite d'un logement de capsule et introduite dans la chambre de capsule (13, 74), **caractérisé en ce que**
au moins un des deux éléments de capsule comprend, en plus de l'ouverture unilatérale, au moins un trou préfabriqué (7, 6) et
l'inhalateur comprend un coulisseau (14, 77) lors de l'actionnement duquel la capsule (11, 71) et le logement de capsule dont est extraite la capsule (11, 71) et introduite dans la chambre de capsule (13, 74), sont déplacés l'un par rapport à l'autre de telle sorte qu'au moins un trou préfabriqué (6, 7, 72a, 72b) est dégagé au niveau de la capsule (11, 71)

2. Système composé d'un inhalateur et d'au moins une capsule selon la revendication 1, **caractérisé en ce que**
- avant l'utilisation de l'inhalateur, la capsule (11, 71) est stockée dans un logement de capsule faisant partie de l'inhalateur, dans lequel le logement de capsule obture au moins un trou (72a, 72b) qui, après emboîtement des éléments de capsule, mène dans la cavité de la capsule (11, 71) et/ou obture tous les trous (72a, 72b) qui, après emboîtement des éléments de capsule, mènent dans la cavité de la capsule (11, 71), et
- la capsule (11, 71) peut être extraite du logement de capsule et déplacée dans la chambre de capsule (13, 74) avec le coulisseau (14, 77)

3. Système d'inhalateur et de capsule (11, 71) selon la revendication 2, **caractérisé en ce que** le coulisseau (14, 77) forme une région de paroi de la chambre de capsule (13, 74) après déplacement de la capsule (11, 71) dans la chambre de capsule (13, 74) et/ou **en ce qu'**une entrée d'air est réalisée dans le coulisseau (14, 77) pour la chambre de capsule (13, 74)

4. Système d'inhalateur et de capsule selon l'une des revendications 2 ou 3, **caractérisé en ce que** deux capsules (11, 71) font partie du système et l'inhalateur comporte deux chambres de capsule (13, 74) et deux logements de capsule.

5. Système comprenant un inhalateur et deux capsules selon la revendication 4, **caractérisé en ce que** les deux capsules sont remplies de formulations et/ou de quantités de formulation différentes.

6. Système composé d'un inhalateur et d'une capsule (11, 71) contenant une préparation pharmaceutique, de préférence sous forme de poudre,
dans lequel la capsule comprend deux éléments de capsule ouverts d'un côté, à savoir un corps de capsule (2) et un capuchon de capsule (1), qui sont emboîtés de manière télescopique l'un dans l'autre par leurs ouvertures de sorte qu'une cavité est formée, et
dans lequel l'inhalateur comprend une chambre de capsule (13, 74) pour la réception d'une capsule (11, 71), dans lequel la chambre de capsule (13, 74) comprend une entrée d'air et une sortie d'air qui mène en direction d'un embout (78), et la capsule (11, 71) est insérée dans la chambre de capsule (13, 74), **caractérisé en ce que**
au moins un des deux éléments de capsule comprend, en plus de l'ouverture unilatérale, au moins un trou préfabriqué (7, 6) et
l'inhalateur comprend un coulisseau (14, 77) lors de l'actionnement duquel la capsule (11, 71) et un composant annulaire sont déplacés l'un par rapport à l'autre de telle sorte qu'au moins un trou préfabriqué (6, 7, 72a, 72b) est dégagé au niveau de la capsule (11, 71).

7. Système selon la revendication 6, **caractérisé en ce que** la capsule assemblée, outre le corps de capsule (2) et le capuchon de capsule (1), présente le composant annulaire au moyen duquel est obturé au moins un trou (6, 7, 72a, 72b) qui, après emboîtement du corps de capsule (2) et du capuchon de capsule (1), mène dans la cavité de la capsule (12), et/ou au moyen duquel sont obturés tous les trous qui, après emboîtement du corps de capsule (2) et du capuchon de capsule (1), mènent dans la cavité de la capsule, et **en ce que** le composant annulaire peut être séparé de la capsule (11, 71) avec le coulisseau (14, 77) de telle sorte qu'au moins un trou préfabriqué (6, 7, 72a, 72b) est découvert au niveau de la capsule (11, 71).

8. Système composé d'un inhalateur et d'au moins une capsule (11, 71) selon la revendication 7, **caractérisé en ce que**, après actionnement du coulisseau (14, 77), le composant annulaire forme une étanchéité entre le coulisseau (14, 77) et le logement de capsule et/ou entre le coulisseau (14, 77) et la chambre de capsule (13, 74).

9. Système selon l'une des revendications précédentes, **caractérisé en ce que** les éléments de capsule sont réalisés en une matière plastique, de préférence les deux dans le même matériau et en particulier en polyéthylène, polycarbonate, polyester, polypropylène ou polyéthylène téréphtalate, avec un procédé de moulage par injection et les trous préfabriqués (6, 7) ont déjà été formés avec ce même procédé de moulage par injection.

10. Système d'inhalateur et de capsule (11, 71) selon l'une des revendications précédentes, **caractérisé en ce que** le coulisseau (14, 77) comprend au moins un élément d'encliquetage, en particulier au moins un bras à ressort (77b), qui, lors de l'actionnement de préférence complet du coulisseau (14, 77), entre en prise avec un autre composant de l'inhalateur de telle sorte que le coulisseau (14, 77) peut être ramené dans sa position de départ de manière non destructive.

11. Procédé de fourniture d'un système d'inhalateur et de capsule selon l'une des revendications 2 ou 3, **caractérisé en ce qu'**un élément de capsule ouvert d'un côté - le corps de capsule (2) - est d'abord inséré dans un logement de capsule, le corps de capsule (2) est rempli avec une quantité mesurée de préparation pharmaceutique, de préférence sous forme de poudre, puis le corps de capsule est fermé par un capuchon de capsule (1) et le logement de capsule est assemblé avec les autres composants de l'inhalateur.

12. Procédé de fourniture d'un système d'inhalateur et de capsule selon l'une des revendications 7 ou 8, **caractérisé en ce qu'**un élément de capsule ouvert d'un côté - le corps de capsule (2) - est d'abord inséré dans un composant annulaire, le corps de capsule (2) est rempli avec une quantité mesurée de préparation pharmaceutique, de préférence sous forme de poudre, puis le corps de capsule est fermé par un capuchon de capsule (1) et le composant annulaire est assemblé avec les autres composants de l'inhalateur.

13. Procédé de fourniture d'un système d'inhalateur et de capsule selon l'une des revendications 11 ou 12, **caractérisé en ce que**, après assemblage des composants de l'inhalateur, le système se trouve dans un état qui convient de préférence au transport ou au stockage, dans lequel les trous de la capsule (11, 71) formée par le corps de capsule (2) et le capuchon de capsule (1) sont fermés, et **en ce que** le système est transféré dans un état prêt à être utilisé par coulissement ou traction au niveau d'un composant de l'inhalateur ou du système, dans lequel les trous de la capsule sont dégagés par ce coulissement ou cette traction et/ou la capsule (11, 71) est déplacée dans une chambre de capsule (13, 74).
